# EUROPEAN PATENT SPECIFICATION

(11) **EP 3 984 476 B1**
(45) Date of publication and mention of the grant of the patent: **22.05.2024**
(21) Application number: 21190598.9
(22) Date of filing: 23.12.2017
(51) Int. Cl.: A61B 17/22, A61B 17/32, A61B 10/02, A61B 17/02, A61B 17/00

(54) **TISSUE EXTRACTION DEVICES**
GEWEBEEXTRAKTIONSVORRICHTUNGEN
DISPOSITIFS D'EXTRACTION DE TISSUS

(30) Priority: 23.12.2016 US 201662438916 P; 13.03.2017 US 201762470625 P; 06.10.2017 US 201762569293 P
(43) Date of publication of application: 20.04.2022
(62) Divisional of application: 17884362.9
(73) Proprietor: Brigham and Women's Hospital, Inc., Boston, MA 02115 (US); Freyja Healthcare, LLC, North Andover, MA 01845 (US)
(72) Inventor: BONADIO, Frank, Bray (IE); EINARSSON, Jon, I., Boston, 02215 (US); SMITH, Jordan, Cambridge, 02138 (US); ISAKOV, Alexander, Sudbury, 01776 (US); PRADHAN, Debasish, 768003 Sambalpur (IN); SOLKAR, Athar, Anwar, 415612 Ratnagiri (IN); PATANKAR, Mangesh, Ratnakar, 400408 Navi Mumbai (IN); KATRE, Nikhil, 400705 Navi Mumbai (IN); CHAVAN, Tejas, 400705 Navi Mumbai (IN)
(74) Representative: Hancox, Jonathan Christopher

(56) References cited:
- EP-A1- 0 578 997
- WO-A1-2014/158880
- US-A- 5 735 289
- US-A1- 2016 346 121

## Description

### TECHNICAL FIELD

Various aspects of the present disclosure relate generally to surgical devices and methods. More specifically, the present disclosure relates to tissue extraction devices and related methods for minimally invasive surgery.

### BACKGROUND

Conventional tissue extraction devices, such as laparoscopic morcellators, may include a sharp spinning blade for cutting tissue. Such devices may be inefficient and may require prolonged operating times, resulting in increased cost. Such devices also may cause unintended injuries. In some instances, conventional laparoscopic morcellators have the potential to spread occult malignancy, and this may worsen patient prognosis. Improving the design and operation of tissue extraction devices may address one or more of the aforementioned issues.

Devices in the general field of the present invention are disclosed in US 2016/0346121 (lanchulev et al.), which shows a device according to the preamble of claim 1, and US 5,735,289 (Pfeffer et al.).

### SUMMARY

The invention relates to a tissue extraction device as defined in claim 1.

Aspects of the present disclosure relate to, among other things, tissue extraction devices and related methods. Each of the aspects disclosed herein may include one or more of the features described in connection with any of the other disclosed aspects.

It may be understood that both the foregoing general description and the following detailed description are exemplary and explanatory only and are not restrictive of the features claimed.

As used herein, the terms "comprises," "comprising," or any other variation thereof, are intended to cover a non-exclusive inclusion such that a process, method, article, or apparatus that comprises a list of elements does not necessarily include only those elements, but may include other elements not expressly listed or inherent to such process, method, article, or apparatus. The term "exemplary" is used in the sense of "example," rather than "ideal."

The disclosure provides embodiments of a kit that in turn includes a bag having a proximal portion and a distal portion, and a retractor ring separate from the bag. The retractor ring can be configured to be coupled to the proximal portion of the bag, and when coupled to the proximal portion of the bag, the retractor ring can be configured to be manipulated from (i) a first orientation, where the retractor ring engages a first region of the proximal portion of the bag, to (ii) a second orientation, where the retractor ring engages a second region of the proximal portion of the bag, the second region being distal to the first region, to pull the distal portion of the bag toward the retractor ring.

In some implementations, the retractor ring can include an inner ring and an outer ring, the inner ring being received in the outer ring. The outer ring can include a plurality of discrete segments. The outer ring can be more flexible than the inner ring. The outer ring can be rotatable relative to the inner ring in a poloidal direction of the retractor ring. The retractor ring can be configured to be coupled to the proximal portion of the bag by clamping the proximal portion of the bag between the inner ring and the outer ring. One of the inner ring and the outer ring can include a protrusion complementary to a recess in the other of the inner ring and the outer ring. The retractor ring can be configured to be coupled to the proximal portion of the bag by at least one of a hook and an adhesive. The proximal portion of the bag can include a channel configured to receive the retractor ring to couple the retractor ring to the proximal portion of the bag.

The disclosure provides embodiments of a method of applying tension to a distal portion of a bag using a proximal portion of the bag. An illustrative method includes coupling a retractor ring to the proximal portion of the bag, the retractor ring being separate from the bag prior to being coupled to the bag and manipulating the retractor ring from (i) a first orientation, where the retractor ring engages a first region of the proximal portion of the bag, to (ii) a second orientation, where the retractor ring engages a second region of the proximal portion of the bag, the second region being distal to the first region, to pull the distal portion of the bag toward the retractor ring.

In some implementations, manipulating the retractor ring can include rotating at least a portion of the retractor ring in a poloidal direction. Manipulating the retractor ring can include rolling the proximal portion of the bag around the retractor ring. Manipulating the retractor ring can include pulling the proximal portion of the bag proximally relative to the retractor ring.

The disclosure provides embodiments of a tissue extraction device including a bag having an interior and a cutting element extending through the interior of the bag. The cutting element includes a strand, and a cutting member on the strand. The cutting member includes an edge for cutting tissue as the cutting member is drawn across tissue.

In some implementations, the cutting member can include a blade on the strand. The edge can be linear, and/or curved. If desired, the edge can form a partial helix. The cutting member can be one of a plurality of cutting members on the strand, and the edge is one of a plurality of edges of the plurality of cutting members. Each edge of the plurality of edges can be discrete, separated from all other edges of the plurality of edges by a gap.

The tissue extraction device can further include a member coupled to an interior surface of the bag, such that a channel is defined between the member and the interior surface. The cutting element extends through the channel.

The cutting element can include eyelets. The tissue extraction device can further include handles. The handles can be removably coupled to the eyelets.

The tissue extraction device can further include a wound retractor surrounding the cutting element. The wound retractor can include a clip that removably receives a portion of the cutting element. The wound retractor can have a spiral shape.

The bag can include an inner layer and an outer layer. The inner layer can include a mesh. The tissue extraction device can further include a wound retractor surrounding the cutting element. The wound retractor can include a hook for engaging the mesh. A rim of the inner layer can be removably coupled to a rim of the outer layer.

Embodiments are also provided of a method of extracting a tissue specimen from a subject with a tissue extraction device having a bag and a cutting element. The method can include inserting the bag and the cutting element together through an opening in the subject, inserting the tissue specimen into the bag, extracting a proximal portion of the bag, and ends of the cutting element, from the subject, via the opening, holding the ends of the cutting element, and cutting the tissue specimen with the cutting element by moving the cutting element back-and-forth in a sawing motion across the tissue specimen.

The method can further include inserting a wound retractor into the bag and positioning the wound retractor in the opening. The method can further include removably securing the cutting element to the wound retractor. The method can further include retracting the opening by expansion of the wound retractor. The method can further include compressing the tissue specimen between the cutting element and an end of the wound retractor. The method can further include securing the bag to the wound retractor. Securing the bag to the wound retractor can include hooking the bag onto the wound retractor.

The bag can include an inner layer and an outer layer. The method can further include separating the inner layer from the outer layer to tighten the inner layer around the tissue specimen. Separating the inner layer from the outer layer can include breaking a separable connection that removably couples the inner layer to the outer layer.

Cutting the tissue specimen can include moving a cutting member on the cutting element back-and-forth across the tissue specimen. Cutting the tissue specimen can include moving an edge of the cutting member back-and-forth across the tissue specimen.

Inserting the bag and the cutting element through the opening can include penetrating the opening with an introducer, and deploying the bag and the cutting element from the introducer. The cutting element can move from a collapsed configuration to an expanded configuration when the cutting element is deployed from the introducer. The cutting element can move the bag from a collapsed configuration to an expanded configuration when the bag is deployed from the introducer. The introducer can include a tubular elongate member for receiving the bag and the cutting element. The introducer can further include an inflatable securing member for securing the tubular elongate member in the opening.

The tubular elongate member can include a proximal section and a distal section. The proximal section and the distal section can be removably coupled. The proximal section can be uncoupled from the distal section and removed from the subject after the bag and the cutting element are deployed from the introducer. The distal section can remain in the opening as the tissue specimen is inserted into the bag.

The disclosure further provides a method of extracting a tissue specimen from a subject with a tissue extraction device having a bag and a cutting element. The method includes inserting the bag and the cutting element together through an opening in the subject, inserting the tissue specimen into the bag, extracting a proximal portion of the bag, and ends of the cutting element, from the subject, via the opening, separating an inner layer of the bag from an outer layer of the bag, moving the inner layer of the bag relative to the outer layer of the bag to tighten the inner layer of the bag around the tissue specimen, and cutting the tissue specimen with the cutting element.

If desired, the method can further including inserting a wound retractor into the opening. The method can further include securing the inner layer of the bag to the wound retractor to maintain a tightness of the inner layer of the bag around the tissue specimen. The method can still further include compressing the tissue specimen between the inner layer of the bag and an end of the wound retractor. The method can still further include compressing the tissue specimen between the cutting element and an end of the wound retractor. If desired securing the inner layer of the bag to the wound retractor can include bringing the inner layer into engagement with one or more hooks on the wound retractor. Bringing the inner layer of the bag into engagement with the one or more hooks can include sliding the one or more hooks in a radial direction relative to a proximal-facing surface of the wound retractor. The method can still further include inflating a portion of the wound retractor to secure the wound retractor in the opening. The method can still further include adjusting a width of a wound- engaging portion of the wound retractor to retract the opening. If desired, adjusting a width of the wound engaging portion can include moving discrete sections of the wound engaging portion apart from each other.

The disclosure further provides embodiments of an illustrative tissue extraction device that includes a bag having an interior, a strand extending through the interior of the bag, wherein the strand includes a plurality of braided filaments, the braided filaments defining a series of protrusions and recesses for abrading tissue when pulled across the tissue.

The disclosure further provides embodiments of an illustrative tissue extraction device, including only one bag having an interior, a basket in the interior of the bag, a cutting element releasably coupled to the basket, wherein the cutting element includes a cutting edge for cutting tissue as the cutting edge is drawn across tissue. The cutting element can include a plurality of rotatably coupled links that form a chain. The cutting element can include a strand and a plurality of circular saw blades rotatably coupled to the strand. If desired, the circular saw blades can be operatively coupled to a drive mechanism for rotating the circular saw blades relative to the strand.

The disclosure further provides embodiments of a kit for extracting tissue. The kit includes a bag assembly that in turn includes at least one bag and at least one cutting element, an introducer assembly for introducing the bag assembly into a body cavity, a retractor ring configured for coupling to the bag assembly, wherein eversion of the retractor ring rolls a portion of the bag assembly around the retractor ring, a protector disc configured for coupling to the bag assembly and the retractor ring, the protector disc having an opening for receiving the at least one cutting element, and a clip for restraining the at least one cutting element.

The disclosure further provides embodiments of a tissue extraction device, including a bag having and defined by an interior surface, and a cutting element extending through the interior of the bag. The cutting element includes a cutting member having an edge for cutting tissue as the cutting member is drawn across tissue, and a cover movable between a first configuration covering the edge of the cutting member and a second configuration exposing the edge of the cutting member.

The disclosure yet further provides embodiments of a tissue extraction device, including a bag having an interior, and a plurality of cutting elements extending through or along a surface of the bag, wherein the cutting elements have a common end. The cutting elements can be substantially parallel. The cutting elements can have a woven pattern. The tissue extraction device can further include a handle coupled to the common end. If desired, at least one of the cutting elements can transition from a first shape to a second shape upon being released from the interior of the bag. The at least one cutting element can include a shape memory alloy. If desired, the bag can include an inflatable rib. If desired, the bag can include an inner layer and an outer layer separated by an inflatable chamber, and an inflating connection for inflating the inflatable chamber. In some implementations, the inflatable chamber can include an inflatable toroid. In some implementations, the inflatable chamber can include an inflatable corrugation. In some implementations, the inflatable chamber can include a plurality of inflatable cells, wherein at least two of the inflatable cells have a common wall. In some implementations, at least one of the cutting elements can include a barbed suture. In some implementations, at least one of the cutting elements can include a bladed suture. In some implementations, the plurality of cutting elements can be a chain of cutting elements. In some implementations, the chain of cutting elements can include curved cutting elements. In some implementations, each of the curved cutting elements can include a curved cutting edge located on a convex end. In some implementations, the curved edge can face an interior space of the bag.

The disclosure further provides a tissue extraction device including a bag having an interior, and a plurality of strands extending through the interior of the bag, wherein the strands have a common end and each of the strands include a plurality of cutting elements.

If desired, at least one of the cutting elements can transition from a first shape to a second shape upon being released from the interior of the bag. At least one of the cutting elements can include a shape memory alloy. In some implementations, the cutting elements can be configured and arranged to expand such that the cutting elements flare outwardly.

The disclosure further provides embodiments of a tissue extraction device including a first ring including a first component of a ratchet and a channel configured and arranged to receive a second component of the ratchet, wherein the second component is on an arm of a retractor, a second ring removably coupled to the first ring, the second ring including a plurality of retainers positioned at even intervals along the second ring, and a third ring removably coupled to the first ring, the third ring including hooks configured and arranged to receive a bag to be rolled around the third ring.

If desired, the first component of the ratchet can be a pawl positioned in the channel, and the second component of the ratchet is a rack on the arm of the retractor. The retractor can include a blade coupled to the arm. In some implementations, at least a portion of the blade can overlap at least a portion of an adjacent blade.

The disclosure further provides embodiments of a method of extracting tissue. Such methods can include one or more of inserting a bag into the patient via an incision, inserting a tissue specimen into the bag, coupling the edge of the bag to a ring via hooks on the ring, rolling a portion of the bag around the ring, coupling an outer ring to the ring, the outer ring coupled to an inner ring, cutting the tissue specimen with the cutting element, decoupling the inner ring from the outer ring, inserting retractors into the incision, and extracting the cut tissue specimen from the patient.

The method can further include inflating inflatable ribs of the bag prior to cutting the tissue specimen. Inserting the retractors can include coupling the retractors to ratchets of the outer ring, and incrementally retracting the retractors to open the incision such that the cut tissue specimen can pass through a collar formed by the retractors.

### BRIEF DESCRIPTION OF THE DRAWINGS

The accompanying drawings, which are incorporated in and constitute a part of this specification, illustrate exemplary aspects of the present disclosure and together with the description, serve to explain the principles of the disclosure.
Fig. 1 is a perspective view of a kit, in accordance with aspects of the present disclosure.
Fig. 2 is a perspective view of an introducer assembly of the kit of Fig. 1, in accordance with aspects of the present disclosure.
Figs. 3A and 3B are perspective views of components of the introducer assembly of Fig. 2, in accordance with aspects of the present disclosure.
Figs. 4A and 4B are perspective views of components of the introducer assembly of Fig. 2, in accordance with aspects of the present disclosure.
Figs. 5A-5C are perspective views of components of the introducer assembly of Fig. 2, in accordance with aspects of the present disclosure.
Figs. 6A and 6B are perspective and cross-sectional views of a wound retractor ring of the kit of Fig. 1, in accordance with aspects of the present disclosure.
Figs. 7A-7C are perspective and cross-sectional views of a protector snap disc of the kit of Fig. 1, in accordance with aspects of the present disclosure.
Fig. 8 is a perspective view of a bag assembly of the kit of Fig. 1, in accordance with aspects of the present disclosure.
Figs. 9A-9D are perspective views of the bag assembly, wound retractor ring, and protector snap disc in use, in accordance with aspects of the present disclosure.
Figs. 10A-10C are side views of an exemplary tissue extraction device, in accordance with aspects of the presentdisclosure.
Figs. 11A-11C are side views of an exemplary cutting element, in accordance with aspects of the present disclosure.
Fig. 12 is a side view of an exemplary tissue extractiondevice, in accordance with aspects of the present disclosure.

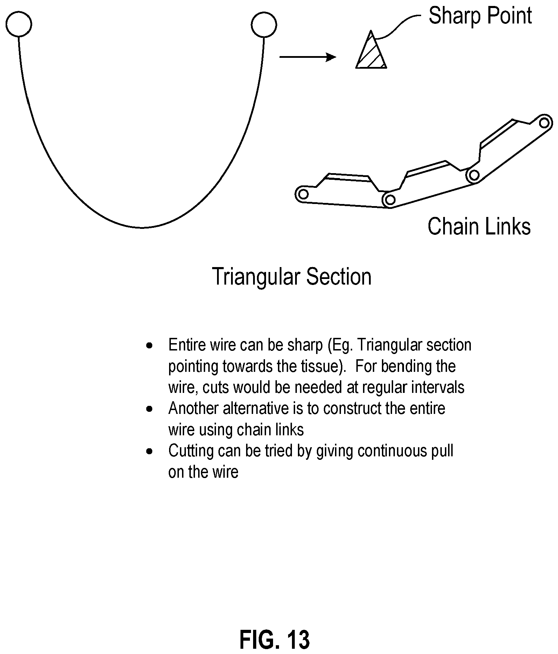

Fig. 13 is a side view of an exemplary cutting element, in accordance with aspects of the present disclosure.
Fig. 14 is a side view of an exemplary cutting element, in accordance with aspects of the present disclosure.
Fig. 15 is a perspective view of an exemplary bag assembly, in accordance with aspects of the present disclosure.
Fig. 16 is a perspective view of a retractor ring of the bag assembly of Fig. 15, in accordance with aspects of the present disclosure.
Figs. 17 and 18 are top and side views, respectively, of the retractor ring of Fig. 16, in accordance with aspects of the present disclosure.
Fig. 19 is a perspective view of the retractor ring of Fig. 16, in an expanded state, in accordance with aspects of the present disclosure.
Figs. 20A and 20B are side and front views, respectively, of the retractor ring of Fig. 16, in its expanded state, in accordance with aspects of the present disclosure.
Fig. 21A and 21B are top and rear views, respectively, of the retractor ring of Fig. 16, in its expanded state, in accordance with aspects of the present disclosure.
Fig. 22 is a perspective view of another exemplary bag assembly, in accordance with aspects of the present disclosure.
Fig. 23 is a cross-sectional perspective view of the bag assembly of Fig. 22, in accordance with aspects of the present disclosure.
Fig. 24 is another cross-sectional perspective view of the bag assembly of Fig. 22, in accordance with aspects of the present disclosure.
Figs. 25, 26A, and 27B are perspective, top, and side views, respectively, of a retractor ring of the bag assembly of Fig. 22, in accordance with aspects of the present disclosure.
Figs. 27A and 27B are side and front views, respectively, of the retractor ring of Fig. 22, in an expanded state, in accordance with aspects of the present disclosure.
Fig. 28 is a perspective view of another exemplary retractor ring, in accordance with aspects of the present disclosure.
Fig. 29 is a cross-sectional perspective view of the retractor ring of Fig. 28, in accordance with aspects of the present disclosure.
Figs. 30A, 30B, and 31A are top, side, and bottom views, respectively, of the retractor ring of Fig. 28, in accordance with aspects of the present disclosure.
Fig. 31B is a cross-sectional side view of the retractor ring of Fig. 28, the cross-section being taken along the line B-B in Fig. 31A, in accordance with aspects of the present disclosure.
Fig. 32 is a top view of an exemplary inner reinforcement ring, in accordance with aspects of the present disclosure.
Fig. 33 is a perspective view of another exemplary retractor ring, in accordance with aspects of the present disclosure.
Fig. 34 is a cross-sectional perspective view of the retractor ring of Fig. 33, in accordance with aspects of the present disclosure.
Figs. 35A and 35B are top and side views, respectively, of the retractor ring of Fig. 33, in accordance with aspects of the present disclosure.
Fig. 36 is a perspective view of an inner ring of the retractor ring of Fig. 33, in accordance with aspects of the present disclosure.
Fig. 37 is a cross-sectional perspective view of the inner ring of Fig. 36, in accordance with aspects of the present disclosure.
Figs. 38A, 38B, 39A, and 39B are top, side, bottom, and cross-sectional side views of the inner ring of Fig. 36, in accordance with aspects of the present disclosure. The cross-sectional side view of Fig. 39B is taken along the line A-A in Fig. 39A.
Fig. 40 is a side view of a cross-sectional face of the inner ring of Fig. 36, in accordance with aspects of the present disclosure.
Fig. 41 is a cross-sectional perspective view of an outer ring of the retractor ring of Fig. 33, in accordance with aspects of the present disclosure.
Figs. 42A and 42B are top and cross-sectional side views, respectively, of the outer ring of Fig. 41, in accordance with aspects of the present disclosure. The cross-sectional side view of Fig. 41B is taken along the line C-C in Fig. 42A.
Fig. 43 is a side view of a cross-sectional face of the outer ring of Fig. 41, in accordance with aspects of the present disclosure.
Fig. 44 is a perspective view of another exemplary retractor ring, in accordance with aspects of the present disclosure.
Fig. 45 is a cross-sectional perspective view of the retractor ring of Fig. 44, in accordance with aspects of the present disclosure.
Figs. 46 and 46B are top and side views, respectively, of the retractor ring of Fig. 44, in accordance with aspects of the present disclosure.
Figs. 47A and 47B are bottom and cross-sectional side views, respectively, of the retractor ring of Fig. 44, in accordance with aspects of the present disclosure. The cross-sectional side view of Fig. 47B is taken along the line D-D in Fig. 47A.
Fig. 48 is a side view of a cross-sectional face of the retractor ring of Fig. 44, in accordance with aspects of the present disclosure.
Figs. 49 and 50 are perspective and cross-sectional perspective views, respectively, of an inner ring of the retractor ring of Fig. 44, in accordance with aspects of the present disclosure.
Figs. 51A, 51B, 52A, and 52B are top, side, bottom, and cross-sectional side views, respectively, of the inner ring of Fig. 49, in accordance with aspects of the present disclosure. The cross-sectional side view of Fig. 52B is taken along the line E-E in Fig. 52A.
Fig. 53 is a side view of a cross-sectional face of the inner ring of Fig. 49, in accordance with aspects of the present disclosure.
Fig. 54 is a cross-sectional perspective view of an outer ring of the retractor ring of Fig. 54, in accordance with aspects of the present disclosure.
Figs. 55A, 55B, 56A and 56B are top, side, bottom, and cross-sectional side views, respectively, of the outer ring of Fig. 54, in accordance with aspects of the present disclosure. The cross-sectional side view of Fig. 56B is taken along the line F-F in Fig. 56A.
Fig. 57 is a side view of a cross-sectional face of the outer ring of Fig. 54, in accordance with aspects of the present disclosure.
Fig. 58A is a perspective view of another exemplary retractor ring, in accordance with aspects of the present disclosure.
Fig. 58B is a cross-sectional perspective view of the retractor ring of Fig. 58A, in accordance with aspects of the present disclosure.
Figs. 59A, 59B, and 60 are top, side, and cross-sectional side views, respectively, of the retractor ring of Fig. 58A, in accordance with aspects of the present disclosure. The cross-sectional side view of Fig. 60 is taken along the line C-C in Fig. 59 A.
Figs. 61A and 61B are perspective and cross-sectional perspective views, respectively, of an inner ring of the retractor ring of Fig. 58A, in accordance with aspects of the present disclosure.
Figs. 62A, 62B, 63A, and 63B are top, side, bottom, and cross-sectional side views, respectively, of the inner ring of Fig. 61A, in accordance with aspects of the present disclosure. The cross-sectional side view of Fig. 61B is taken along the line G-G in Fig. 61A.
Fig. 64 is a side view of a cross-sectional face of the inner ring of Fig. 61A, in accordance with aspects of the present disclosure.
Figs. 65A and 65B are perspective and cross-sectional perspective views, respectively, of an outer ring of the retractor ring of Fig. 58A, in accordance with aspects of the present disclosure.
Fig. 66 is a side view of a cross-sectional face of the outer ring of Fig. 65A, in accordance with aspects of the present disclosure.
Fig. 67 is a perspective view of another exemplary retractor ring, in accordance with aspects of the present disclosure.
Fig. 68 is a cross-sectional perspective view of the retractor ring of Fig. 67, in accordance with aspects of the present disclosure.
Figs. 69A and 69B are top and cross-sectional side views, respectively, of the retractor ring of Fig. 67, in accordance with aspects of the present disclosure. The cross-sectional side view of Fig. 69B is taken along the line E-E in Fig. 69A.
Fig. 70 is a side view of a cross-sectional face of the retractor ring of Fig. 67, in accordance with aspects of the present disclosure.
Figs. 71A, 71B, 72A and 72B are top, side, bottom, and cross-sectional side views, respectively, of an inner ring of the retractor ring of Fig. 67, in accordance with aspects of the present disclosure. The cross-sectional side view of Fig. 72B is taken along the line G-G in Fig. 72A.
Fig. 73 is a side view of a cross-sectional face of the inner ring of Fig. 71A, in accordance with aspects of the present disclosure.
Fig. 74 is a cross-sectional perspective view of an outer ring of the retractor ring of Fig. 67, in accordance with aspects of the present disclosure.
Figs. 75A, 75B, 76A and 76B are top, side, bottom, and cross-sectional side views, respectively, of the outer ring of Fig. 74, in accordance with aspects of the present disclosure. The cross-sectional side view of Fig. 76B is taken along the line H-H in Fig. 76A.
Fig. 77 is a side view of a cross-sectional face of the outer ring of Fig. 74, in accordance with aspects of the present disclosure.
Fig. 78 is a perspective view of another exemplary retractor ring, in accordance with aspects of the present disclosure.
Fig. 79 is a cross-sectional perspective view of the retractor ring of Fig. 78, in accordance with aspects of the present disclosure.
Figs. 80A, 80B, and 81A are top, cross-sectional side, and side views, respectively, of the retractor ring of Fig. 78, in accordance with aspects of the present disclosure.
Fig. 81B is a side view of a cross-sectional face of the retractor ring of Fig. 78, in accordance with aspects of the present disclosure.
Fig. 82 is a cross-sectional perspective view of an inner ring of the retractor ring of Fig. 78, in accordance with aspects of the present disclosure.
Figs. 83 and 84 are perspective and cross-sectional perspective views, respectively, of an outer ring of the retractor ring of Fig. 78, in accordance with aspects of the present disclosure.
Figs. 85 and 86 are perspective and side views, respectively, of another exemplary bag assembly, in accordance with aspects of the present disclosure.
Figs. 87A and 87B are side and cross-sectional side views, respectively, of the bag assembly of Fig. 85, in accordance with aspects of the present disclosure. The cross-sectional side view of Fig. 87B is taken along the line B-B in Fig. 87A.
Fig. 88 is a partial cross-sectional side view of the bag assembly of Fig. 85, in accordance with aspects of the present disclosure.
Figs. 89 and 90 are perspective and cross-sectional perspective views, respectively, of a retractor ring of the bag assembly of Fig. 85, in accordance with aspects of the present disclosure.
Figs. 91A, 91B, 92A, and 92B are top, side, bottom, and cross-sectional side views, respectively, of the retractor ring of Fig. 89, in accordance with aspects of the present disclosure. The cross-sectional side view of Fig. 92B is taken along the line G-G in Fig. 92A.
Fig. 93 is a perspective view of an inner ring of the retractor ring of Fig. 89, in accordance with aspects of the present disclosure.
Figs. 94A, 94B, 95A, and 95B are top, side, bottom, and cross-sectional side views, respectively, of the inner ring of Fig. 93, in accordance with aspects of the present disclosure. The cross-sectional side view of Fig. 95B is taken along the line F-F in Fig. 95A.
Fig. 96 is a perspective view of an outer ring of the retractor ring of Fig. 88, in accordance with aspects of the present disclosure.
Figs. 97A and 97B are top and side views, respectively, of the outer ring of Fig. 97, in accordance with aspects of the present disclosure.
Fig. 98 is a perspective view of another exemplary bag assembly, in accordance with aspects of the present disclosure.
Fig. 99 is a cross-sectional perspective view of the bag assembly of Fig. 98, in accordance with aspects of the present disclosure.
Fig. 100 is a partially-transparent perspective view of the bag assembly of Fig. 98, in accordance with aspects of the present disclosure.
Fig. 101 is a partial cross-sectional side view of the bag assembly of Fig. 98, in accordance with aspects of the present disclosure.
Figs. 102 and 103 are perspective and cross-sectional perspective views, respectively, of a retractor ring of the bag assembly of Fig. 98, in accordance with aspects of the present disclosure.
Fig. 104 is a partial cross-sectional side view of the retractor ring of Fig. 102, in accordance with aspects of the present disclosure.
Fig. 105 is a side view of a cross-sectional face of the retractor ring of Fig. 102, in accordance with aspects of the present disclosure.
Figs. 106 and 107 are perspective and cross-sectional perspective views, respectively, of an inner ring of the retractor ring of Fig. 102, in accordance with aspects of the present disclosure.
Figs. 108A, 108B, and 108C are top, side, and cross-sectional side views, respectively, of the inner ring of Fig. 106, in accordance with aspects of the present disclosure. The cross-sectional side view of Fig. 108C is taken along the line J-J in Fig. 108A.
Fig. 109 is a side view of a cross-sectional face of the inner ring of Fig. 106, in accordance with aspects of the present disclosure.
Figs. 110 and 111 are perspective and cross-sectional perspective views, respectively, of an outer ring of the retractor ring of Fig. 102, in accordance with aspects of the present disclosure.
Figs. 112A, 112B, and 112C are top, side, and cross-sectional side views, respectively, of the outer ring of Fig. 110, in accordance with aspects of the present disclosure. The cross-sectional side view of Fig. 112C is taken along the line K-K in Fig. 112A.
Fig. 113 is a perspective view of another exemplary bag assembly, in accordance with aspects of the present disclosure.
Fig. 114 is a perspective view of a retractor ring of the bag assembly of Fig. 113, in a contracted state, in accordance with aspects of the present disclosure.
Fig. 115 is another perspective view of the retractor ring of Fig. 113, in an expanded state, in accordance with aspects of the present disclosure.
Fig. 116 is a perspective view of another exemplary bag assembly, in accordance with aspects of the present disclosure.
Fig. 117 is a cross-sectional perspective view of the bag assembly of Fig. 116, in accordance with aspects of the present disclosure.
Fig. 118 is a perspective view of a retractor ring of the bag assembly of Fig. 116, in accordance with aspects of the present disclosure.
Fig. 119 is another perspective view of the retractor ring of Fig. 116, in accordance with aspects of the present disclosure.
Figs. 120A and 120B are top and side views, respectively, of the retractor ring of Fig. 116, in accordance with aspects of the present disclosure.
Figs. 121A and 121B are other top and side views, respectively, of the retractor ring of Fig. 116, in accordance with aspects of the present disclosure.
Fig. 122 is a perspective view of another exemplary bag assembly, in accordance with aspects of the present disclosure.
Fig. 123 is a perspective view of a retractor ring of the bag assembly of Fig. 122, in accordance with aspects of the present disclosure.
Figs. 124A and 124B are top and side views, respectively, of the retractor ring of Fig. 123, in accordance with aspects of the present disclosure.
Figs. 125A and 125B are bottom and cross-sectional side views, respectively, of the retractor ring of Fig. 123, in accordance with aspects of the present disclosure. The cross-sectional side view of Fig. 125B is taken along the line A-A in Fig. 125A.
Figs. 126 and 127 are top and perspective views, respectively, of a section of an outer ring of the retractor ring of Fig. 123, in accordance with aspects of the present disclosure.
Fig. 128 is a perspective view of another exemplary bag assembly, in accordance with aspects of the present disclosure.
Fig. 129 is a partial top cross-sectional view of the bag assembly of Fig. 128, in accordance with aspects of the present disclosure.
Fig. 130A is a top view of an exemplary mesh bag, in accordance with aspects of the present disclosure.
Fig. 130B includes side views of a bag assembly including the mesh bag of Fig. 130A, in accordance with aspects of the present disclosure.
Figs. 131A and 131B show a plurality of cutting elements having common ends.
Fig. 132A shows a perspective view of a bag including three strands.
Fig. 132B shows a plurality of cutting elements of a strand in an expanded arrangement.
Fig. 132C illustrates the cutting elements of an expanded strand in contact with a tissue specimen.
Fig. 133 shows cutting elements having a weaved/woven (crisscrossed) pattern.
Fig. 134A shows a barbed suture, including a barb, that can serve as a cutting element.
Fig. 134B shows bladed suture, including a blade, that can serve as a cutting element.
Fig. 135A shows a side view of a bag including inflatable ribs.
Fig. 135B shows a bottom view of the bag of Fig. 135A including inflated inflatable ribs.
Fig. 135C shows a top view of the bag of Fig. 135A including inflated inflatable ribs.
Fig. 135D shows a side view of the bag of Fig. 135A in a compressed state.
Fig. 136 shows a cross-sectional view of a bag including inflatable toroids.
Fig. 137 shows a cross-sectional view of a bag including inflatable corrugation.
Fig. 138A shows a cross-sectional view of a bag including inflatable cells.
Fig. 138B shows a side view of the bag of Fig. 138B.
Fig. 139A shows a perspective view of a wound retractor.
Fig. 139B shows a sectional view of the wound retractor of Fig. 139A.
Fig. 139C shows a perspective view of the wound retractor of Fig. 139A with the inner ring removed from the outer ring.
Fig. 140A shows a perspective view of the outer ring of the wound retractor of Fig. 139A and a plurality of retractors.
Fig. 140B shows a sectional view of the outer ring of wound retractor of Fig. 139A with a retractor arm positioned within a channel of the outer ring.
Fig. 140C shows a perspective view of the outer ring of wound retractor of Fig. 139A with retractors positioned within channels of the outer ring.
Fig. 140D shows a perspective view of the outer ring of wound retractor of Fig. 139A with retractors positioned within channels of the outer ring.
Fig. 141 shows an exploded view of the wound retractor of Fig. 139A.
Fig. 142A shows a further illustrative cutter in accordance with the present disclosure.
Figs. 142B and 142C show a portion of the cutter of Fig. 142A.
Figs. 142D and 142E show a portion of a chain of cutting elements.
Fig. 142F shows a cutting element in accordance with the present disclosure.

### DETAILED DESCRIPTION

The present disclosure is drawn to laparoscopic tissuedevices and related methods. Reference now will be made in detail to aspects of the present disclosure, examples of which are illustrated in the accompanying drawings. Wherever possible, the same reference numbers will be used throughout the drawings to refer to the same or like parts. The term "distal" refers to a portion farthest away from a user when introducing a device into a subject. By contrast, the term "proximal" refers to a portion closest to the user when placing the device into the subject. The term "approximately," when used to describe a numerical value, may be anywhere in a range of ± 5% from the numerical value.

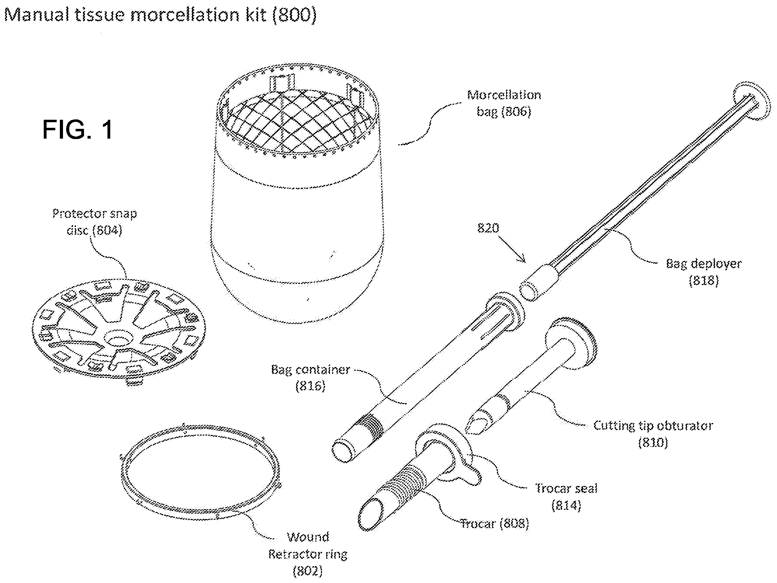

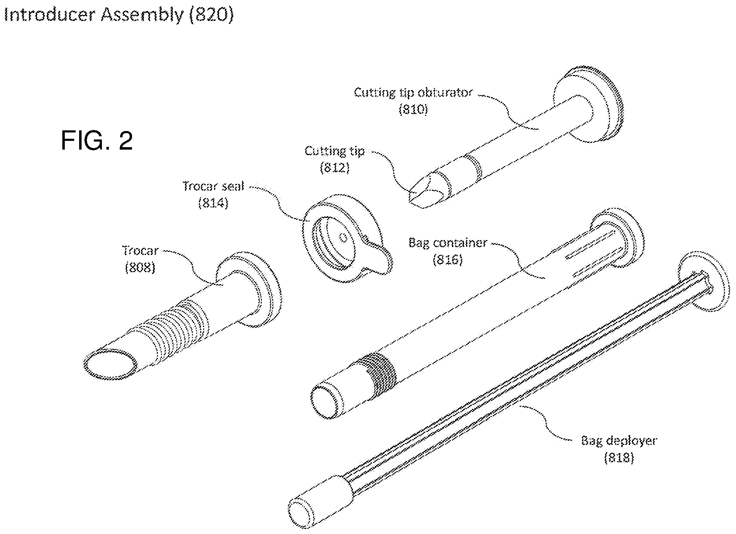

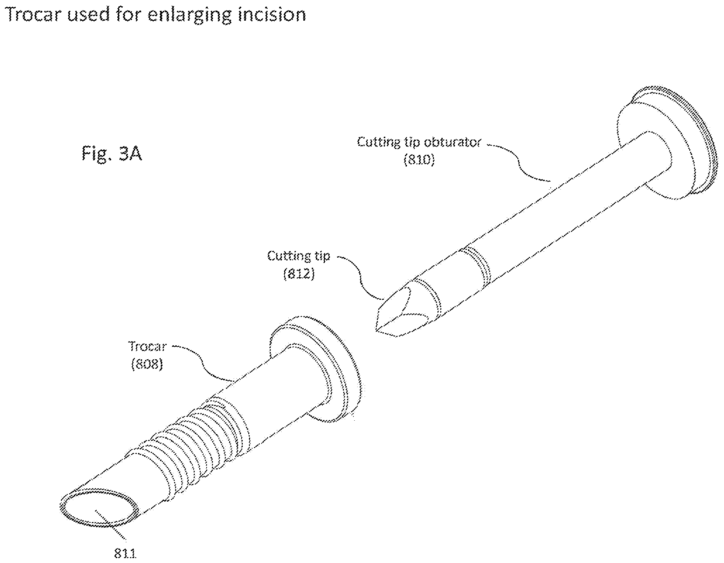

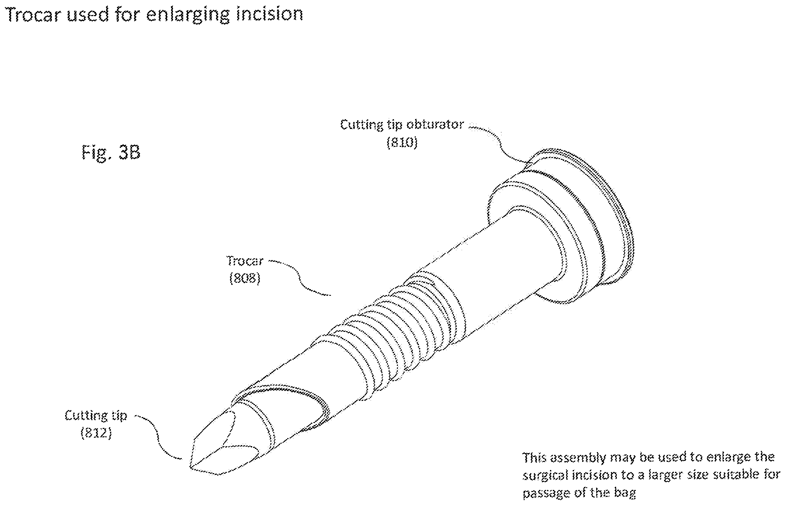

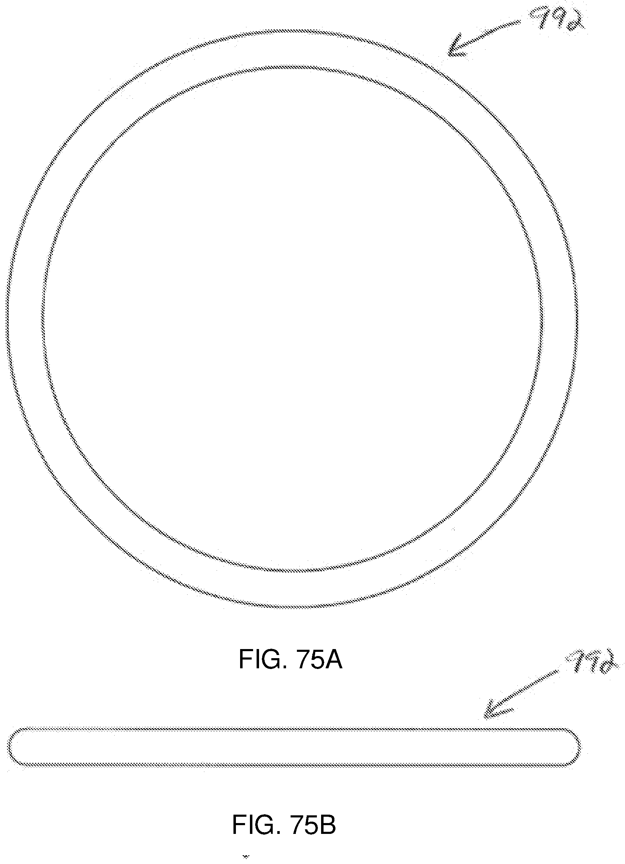

Fig. 1 shows components of a manual tissue morcellationkit 800. Kit 800 may include, for example, a wound retractor ring 802, a protector snap disc 804, a morcellation bag 806, and an introducer assembly 820. Each of these components will be described in greater detail in the paragraphs below, with reference being made to Figs. 2, 3A, 3B, 3A, 3B, 4A-4C, 5A, 5B, 6A-6C, 7, and 8A-8D. It is contemplated, however, that any of the components of kit 800 may be swapped out for similar components (e.g., bags, introducers, and/or wound retractors) shown in figures preceding Fig. 1. It also is contemplated that components shown in figures preceding Fig. 1 may be combined to form another version of a kit similar to kit 800.Fig. 75 shows a disassembled view of introducer assembly 820. Introducer assembly 820 may include, for example, a trocar 808, an obturator 810 having a cutting tip 812, a trocar seal 814, a bag container 816, and a bag deployer 818. Figs. 3A and 3B show trocar 808 and obturator 810 in assembled and disassembled states, respectively. Trocar 808 may have the same features as distal section 405c of introducer 399c (of International Patent Application No. PCT/US2016/061595, filed November 11, 2016), except external threading 813 on trocar 808 may not be on an enlarged, tapered region, but rather, may be on a straight, tubular region. Alternatively, an enlarged, tapered region may be included. Obturator 810 may be inserted into a passage 811 in trocar 808. When obturator 810 is fully inserted into trocar 808, cutting tip 812 may at least partially protrude from a distal end of trocar 808. In use, the user may grasp the assembled trocar 808 and obturator 810, and force cutting tip 812 through tissue, or force cutting tip 812 through an incision already made in tissue. This may create a sufficiently-sized wound opening in the tissue for trocar 808 to pass through. In one example, trocar 808 may have a diameter of approximately 2 cm to approximately 4 cm. For example, trocar 808 may have a diameter of approximately 2.5 cm in a region where it engages tissue.

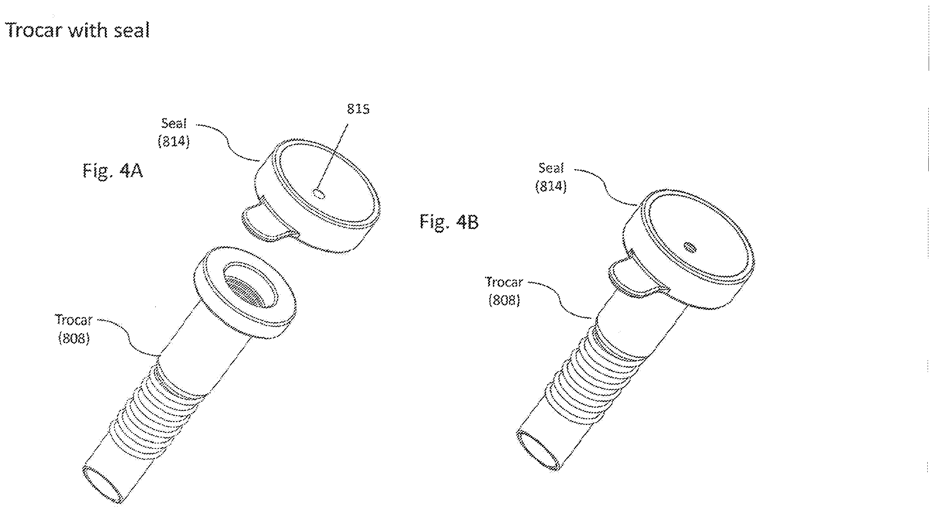

Figs. 4A and 4B show trocar 808 and a cap or seal 814 in assembled and disassembled states, respectively. Cap 814 may have the same features as cap 421c (of International Patent Application No. PCT/US2016/061595, filed November 11, 2016). Cap 814 may cover a proximal end of trocar 808. Cap 814 may include an opening 815 for receiving an instrument (not shown), and for forming a seal with an outer surface of the instrument.

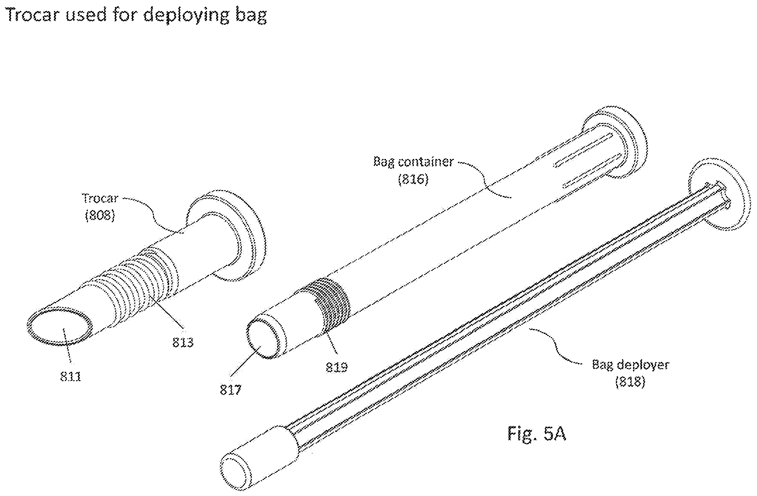

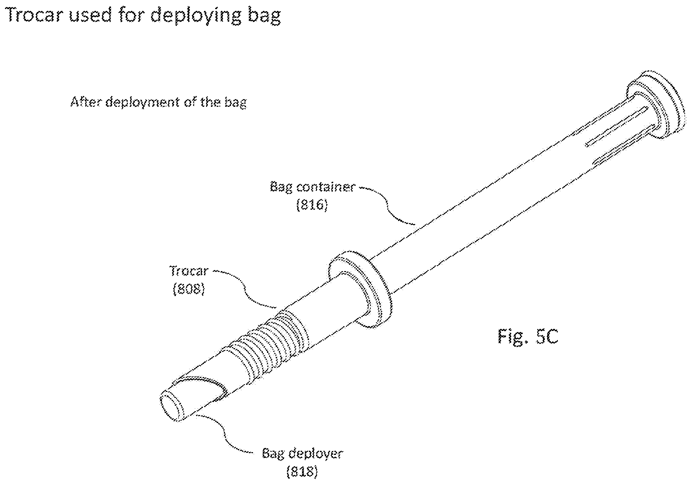

Figs. 5A-5C show trocar 808, bag container 816, and bag deployer 818 in disassembled, partially-assembled, and fully-assembled states, respectively. Bag container 816 may have the same features as proximal section 403c of introducer 399c (of International Patent Application No. PCT/US2016/061595, filed November 11, 2016), and may house bag 806 for delivery into the subject. Bag container 816 may be coupled to trocar 808 in the same manner that proximal section 403c may be coupled to distal section 405c (of International Patent Application No. PCT/US2016/061595, filed November 11, 2016) (e.g., may threaded engagement). Bag deployer 818 may have the same features as pusher 409c(of International Patent Application No. PCT/US2016/061595, filed November 11, 2016), and may be used to push bag 806 out of bag container 816.

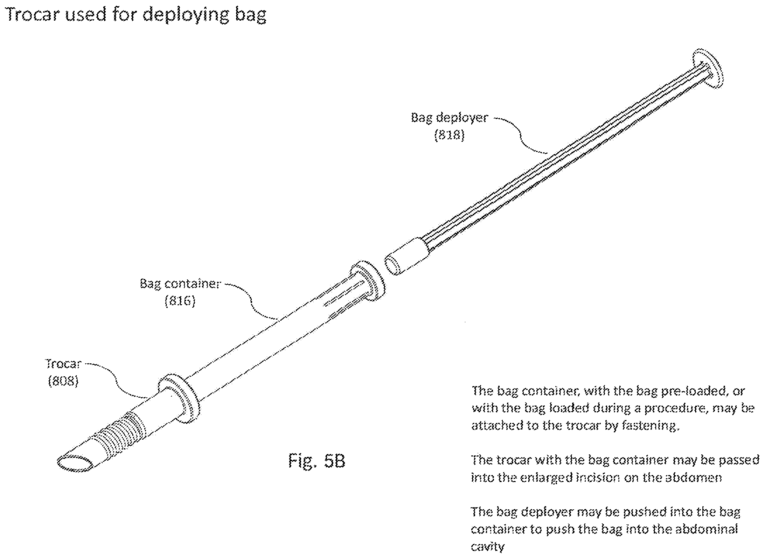

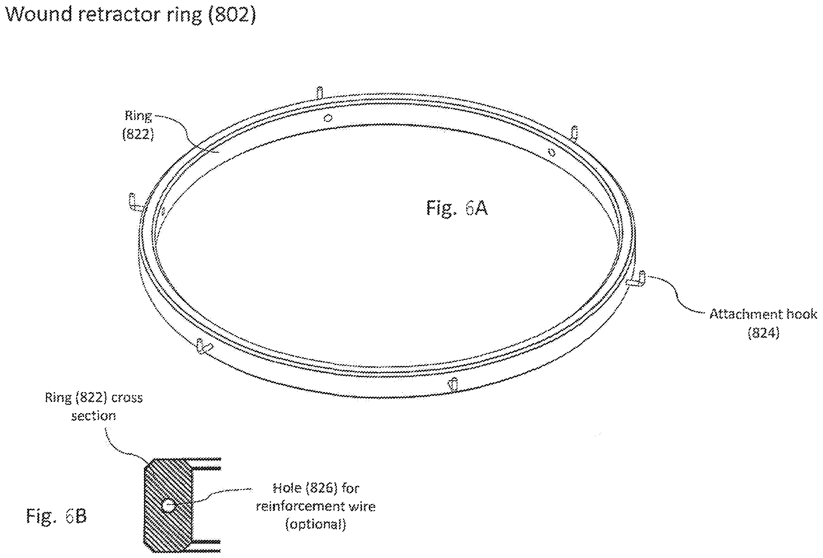

Figs. 5A and 5B show wound retractor ring 802. Wound retractor ring 802 may include a ring body 822 and one or more attachment hooks 824. Ring body 822 may include a hole or passage extending therethrough for receiving a reinforcement wire (not shown). Wound retractor ring 802 may be everted by the user. Fig. 6A shows a first stable configuration of wound retractor ring 802. A second stable configuration of wound retractor ring 802 may be achieved by everting wound retractor ring 802 until attachment hooks 824 face radially-inward. Wound retractor ring 802 may be everted/flipped between its first and second stable configurations. When wound retractor ring 802 is in between its stable configurations, internal forces in wound retractor ring 802 may bias wound retractor ring 802 toward one of its stable configurations. In another example of a tissue extraction device, wherein inner layer 838 is omitted, wound retractor ring 802 may be coupled to outer layer 836 to retract outer layer 836.

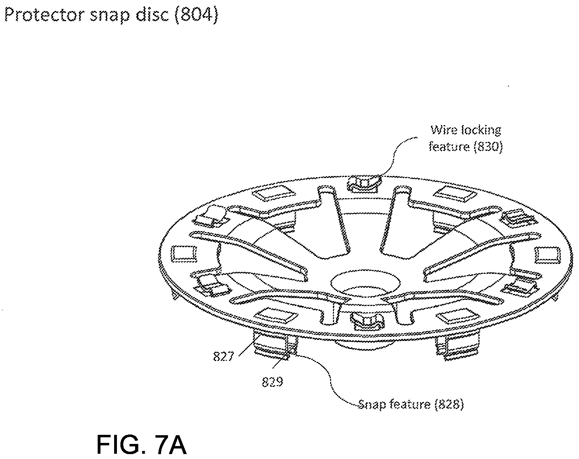

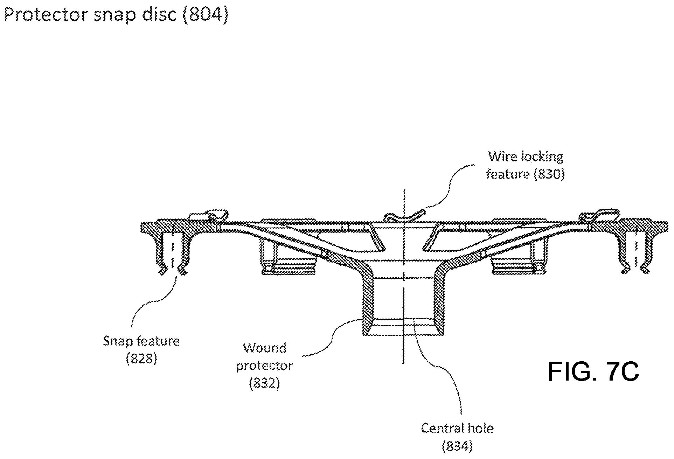

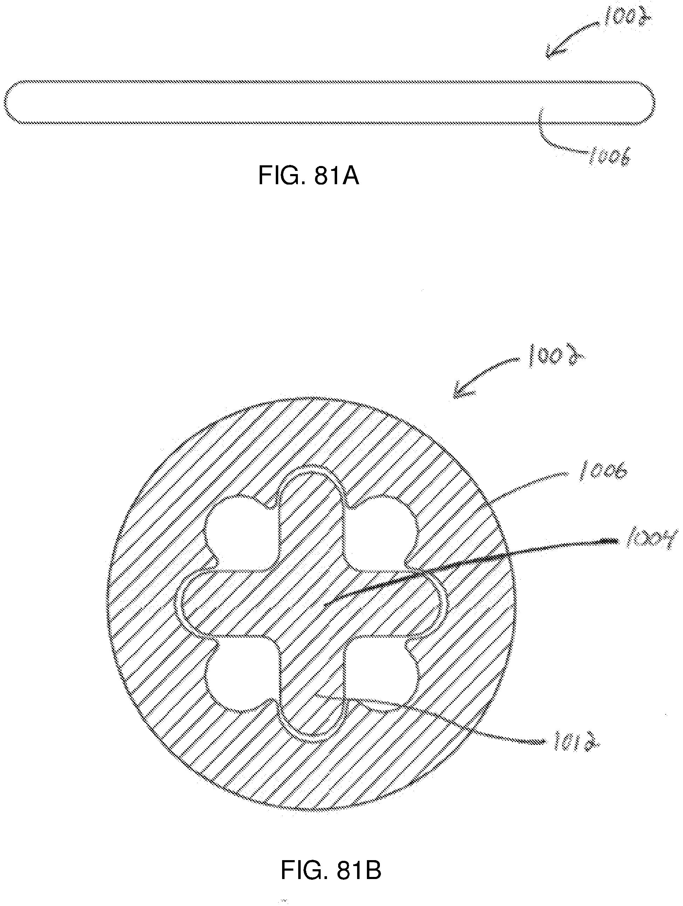

Figs. 7A-7C show protector snap disc 804. Protectorsnap disc 804 may include one or more snap features 828. Snap feature 828 may include, for example, opposing arms 827 and 829. Arms 827 and 829 may be forced against ring 822 of wound retractor ring 802, causing arms 827 and 829 to move from their rest configuration (Fig. 81A) to a spread-apart configuration (not shown). When arms 827 and 829 fully receive and move beyond ring 822, arms 827 and 829 may move back toward their rest configuration, thereby coupling protector snap disc 804 to wound retractor ring 802. The reverse movement may be used to uncouple protector snap disc 804 from wound retractor ring 802.

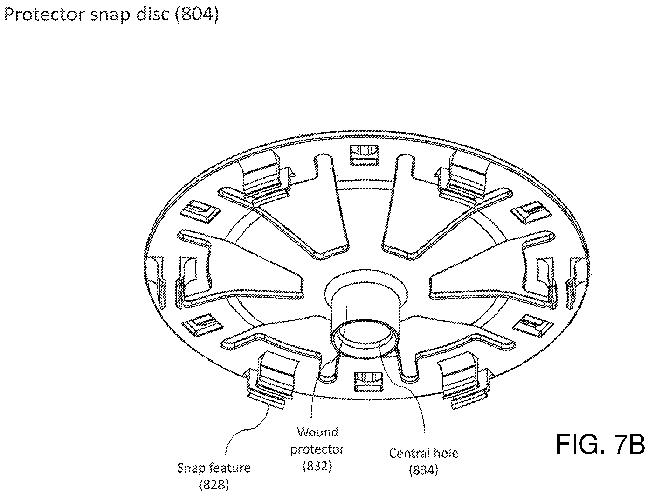

Protector snap disc 804 also may include one or more wire locking features 830 similar to clips 373 (of International Patent Application No. PCT/US2016/061595, filed November 11, 2016), for securing cutting elements (not shown), such as cutting elements 304, to protector snap disc 804. Protector snap disc 804 also may include a wound protector 832 having a central hole 834 running therethrough (Fig. 7B). The cutting elements may be directed through central hole 834. When the user cuts tissue in bag 806 with the strands, wound protector 832 may ensure a working space remains open during a procedure, and/or protect the margins of the bodily orifice or incision from potentially being injured by the cutting elements.

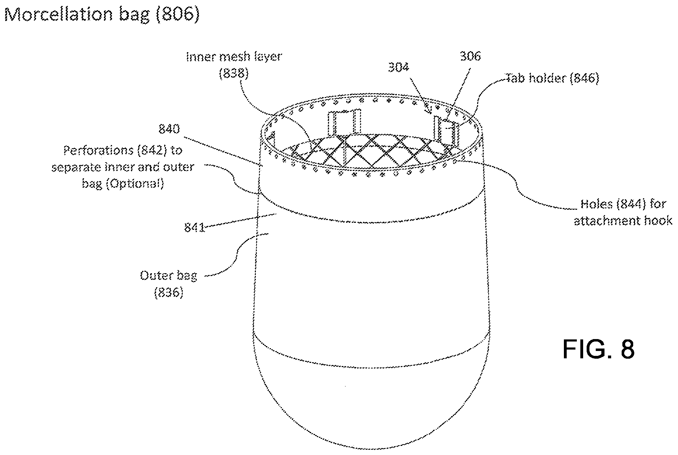

Fig. 8 shows bag 806. Bag 806 may include an outerlayer 836 and an inner layer 838. Outer layer 836 may be similar to outer layer 355 of bag 354 (of International Patent Application No. PCT/US2016/061595, filed November 11, 2016). Inner layer 838 may be similar to inner layer 358 of bag 354. A proximal portion 840 of inner layer 838 may be coupled to a proximal portion 841 of outer layer 336. Proximal portion 840 and proximal portion 841 may be joined by a perforated section 842, allowing the user to separate outer layer 836 from inner layer 838 by tearing apart proximal portion 840 and proximal portion 841 at perforated section 842. In one example, proximal portions 840 and 841 may be made of the same material.

A proximal end of proximal portion 840 may include one or more holes 844 for receiving one or more attachment hooks 844 (Fig. 6A), for securing wound retractor ring 802 to inner layer 838. Once secured, everting wound retractor ring 802 may roll proximal portion 840 around wound retractor ring 802, thereby shortening an axial extent of inner layer 838 by drawing a distal end of inner layer 838 toward a proximal end of inner layer 838. The amount of shortening may be determined by the number of times wound retractor ring 802 is everted.

A proximal end of proximal portion 840 may include one or more tab holders 846. Tab holders 846 may secure anchoring elements 306 of cutting elements 304 to an interior surface of proximal portion 840. The user may release anchoring elements 306 from tab holders 846 by sliding anchoring elements 306 distally out from between tab holders 846 and the interior surface of proximal portion 840. Alternatively, inner layer 838 may include splittable members 365, including distal portion 367, for securing cutting elements 304.

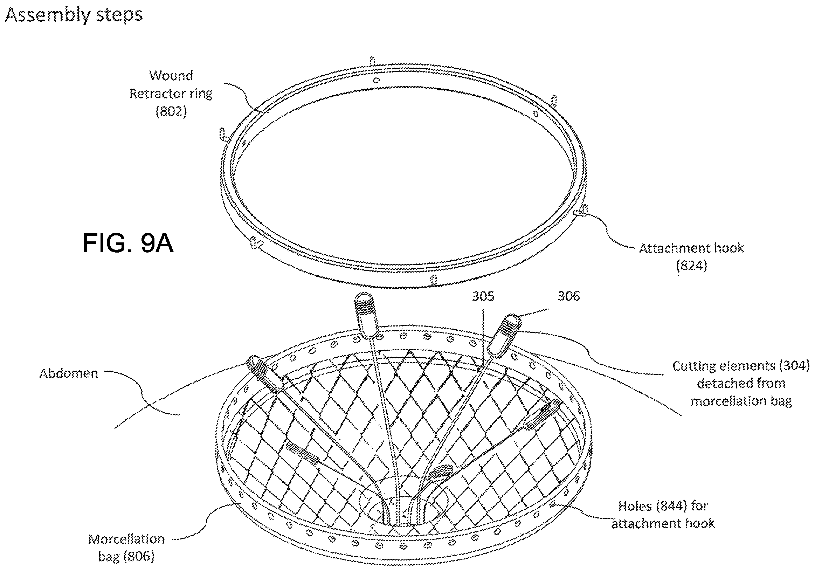

Fig. 9A shows bag 806 extending partially into the subject's abdomen and partially out of the subject's abdomen. At this stage, a tissue specimen may already be contained in bag 806 within the abdomen. Optionally, at this stage, inner layer 838 may be separated from outer layer 836. Anchoring elements 306 are freed from tab holders 846 (not shown here, but shown in Fig. 8). Wound retractor ring 802 is positioned for coupling to proximal portion 840.

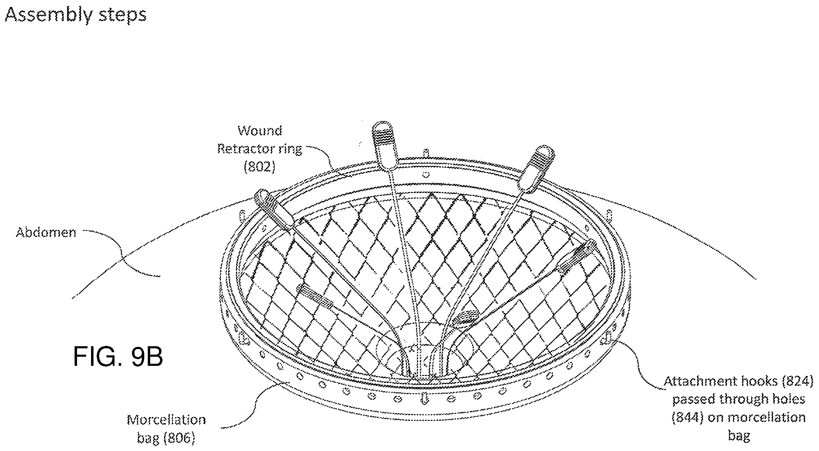

In Fig. 9B, attachment hooks 824 are placed in holes 844 to attach wound retractor ring 802 to proximal portion 840. Optionally, at this stage, inner layer 838 may be separated from outer layer 836. The user may evert/flip wound retractor ring 802 one or more times to induce tension on inner layer 838, so that inner layer 838 pulls the tissue specimen away from outer layer 836, and firmly holds onto the tissue specimen in preparation for cutting. Wound retractor ring 802 may stay in one of its stable configurations, thereby maintaining inner layer 838 in the tensioned state when the user releases wound retractor ring 802 after eversion/flipping.

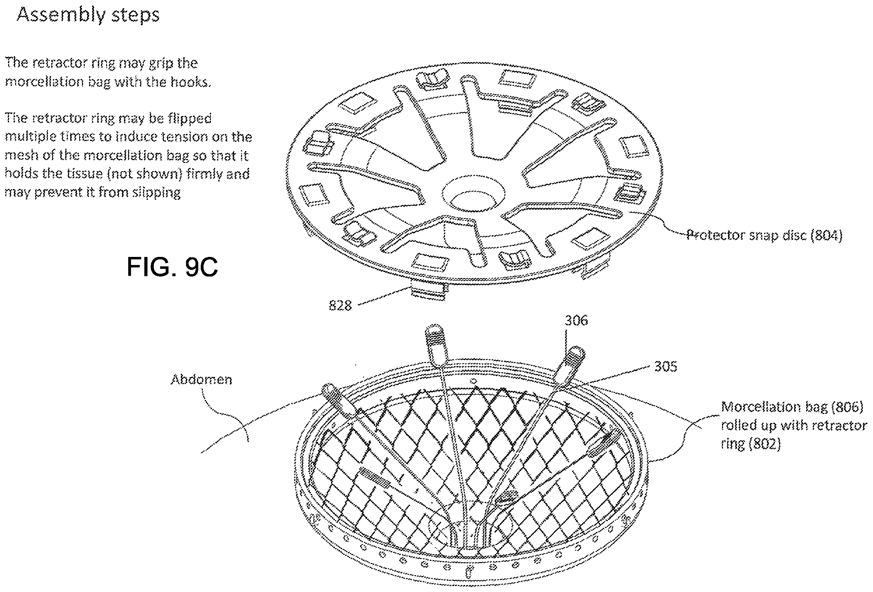

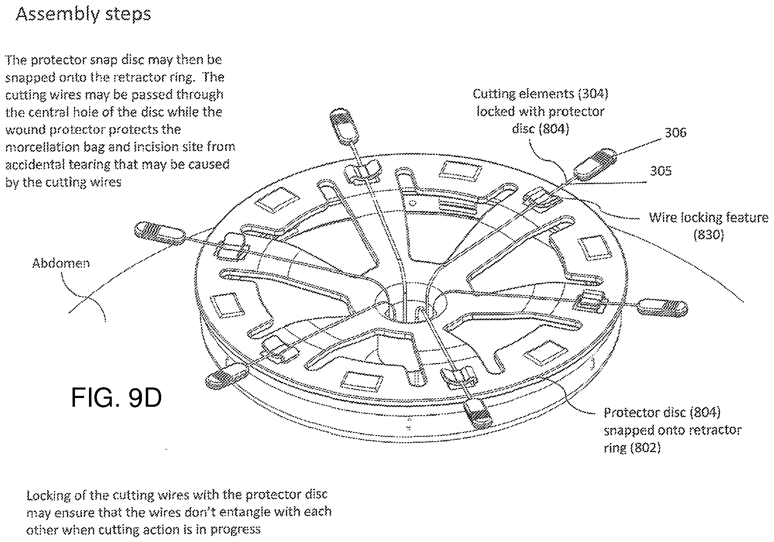

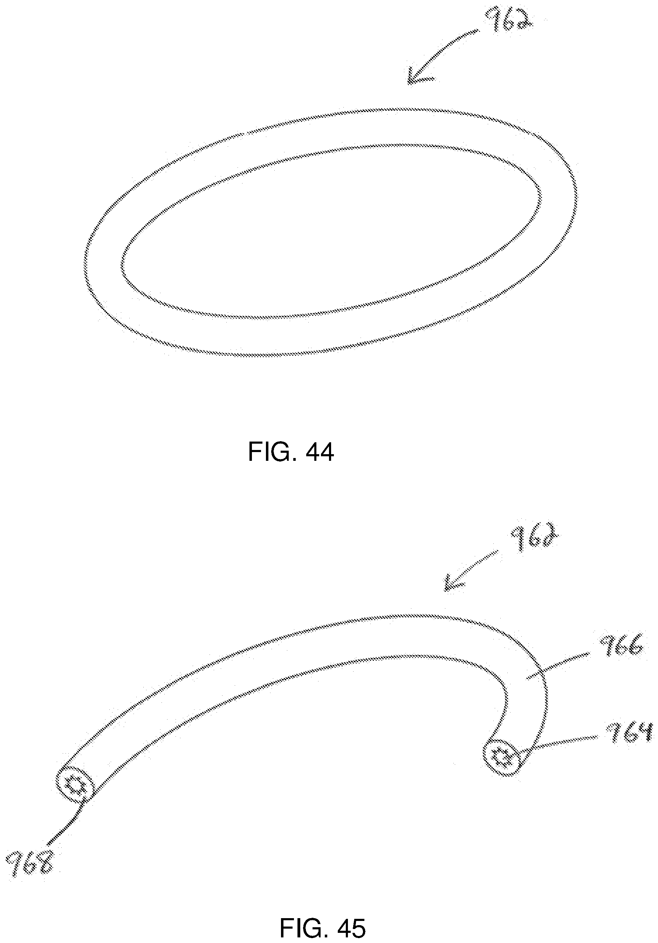

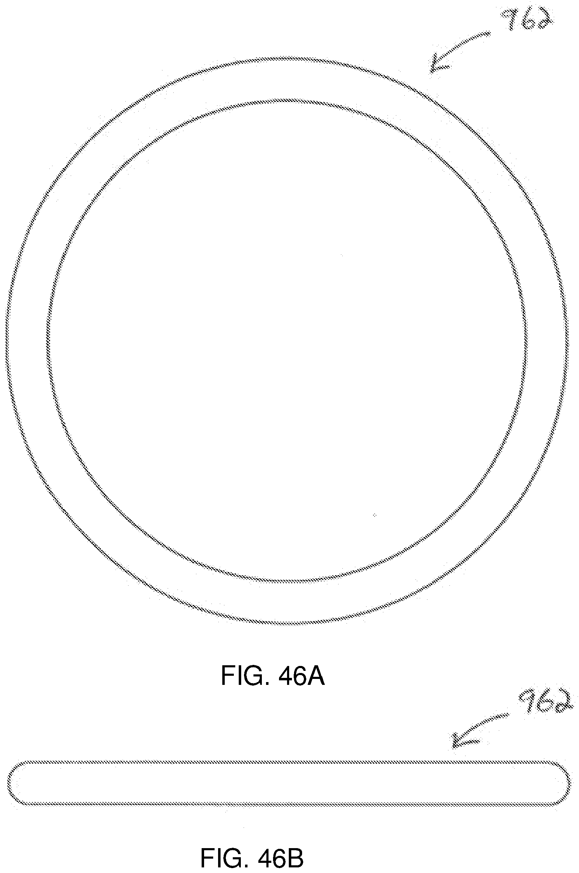

In Fig. 9C, protector snap disc 804 is positioned for coupling to wound retractor ring 802 and bag 806. The user guides strands 305 and anchoring elements 306 of cutting elements 304 through central hole 834, and brings snap features 828 into engagement with wound retractor ring 802 (and portions of inner layer 838 wrapped around wound retractor ring 802). Fig. 9D shows protector snap disc 804 secured to wound retractor ring 802 and bag 806. The user may then move strands 305 of cutting elements 304 into wire locking features 830, serving to secure and/or position cutting elements 304 in preparation for cutting the tissue specimen. The user may release one or more of cutting elements 304 at a time to cut the tissue specimen in a manner similar to that which is shown in Figs. 45A, 45B, 46A, and 46B.

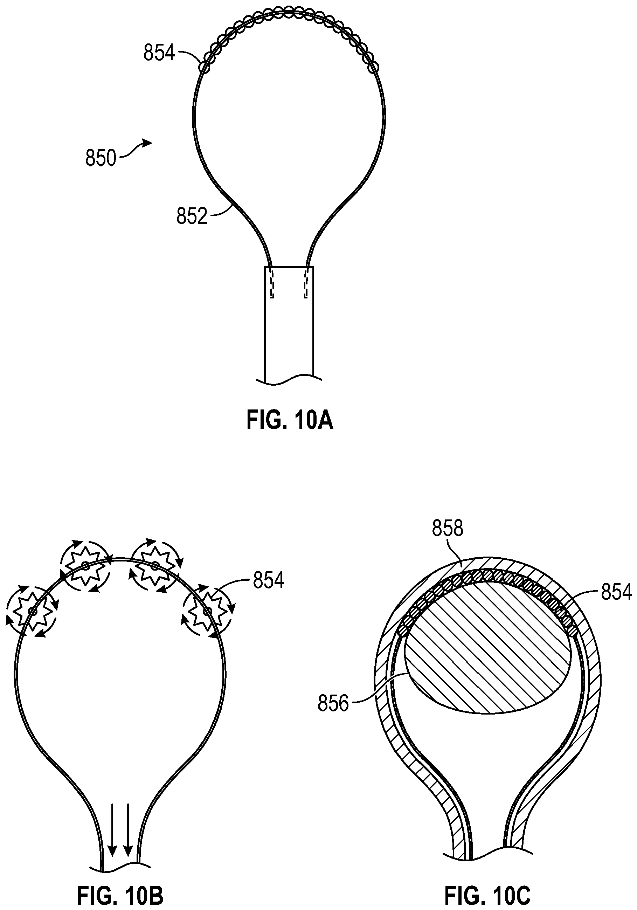

Figs. 10A-10C show aspects of a cutting element 850. Cutting element 850 may include a strand 852, which may be similar to strand 305. One or more circular saw blades 854 may be rotatably mounted on strand 305 at, for example, a central portion of strand 305 that may engage a tissue specimen. Saw blades 854 may be operatively coupled to a drive assembly (not shown) (e.g., an external motor) that may cause blades 854 to rotate to cut through tissue 856. The drive assembly may be operatively coupled to blades 854 may a gear assembly, drive chain, and/or any other suitable mechanical connection. Cutting element 850 also may be pulled against tissue 856 while blades 854 spin to further facilitate cutting through tissue 856. Fig. 10C shows that cutting elements 850 may be positioned within a basket 858. Basket 858 may be formed by circumferentially-spaced arms, such as splittable members 365 (of PCT/US2016/061595). Splittable members 365 may house cutting elements 850 prior to cutting elements 850 being released to engage tissue 856. Splittable members 365 may be pulled against tissue 856 to hold tissue 856 duringcutting.

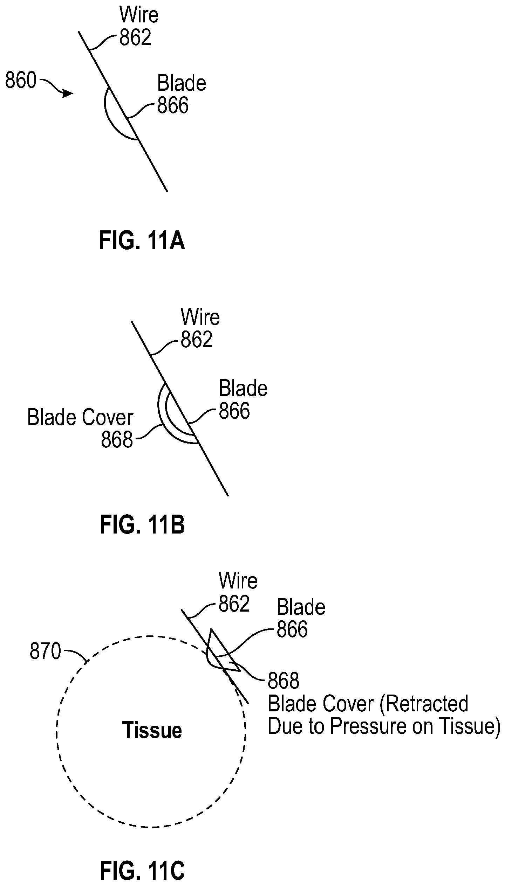

Figs. 11A-11C show aspects of a cutting element 860. Cutting element 860 may include a strand 862 and a cutting member or component 864 similar to any of the aforementioned cutting components. In one example, cutting member 864 may include a cutting blade 866. Cutting element 860 also may include a blade cover 868. Blade cover 868 may cover cutting blade 866 (Fig. 11B). When cutting element 860 is pulled against tissue 870 (Fig. 11C), blade cover 868 may bend, shift,or otherwise deflect to expose cutting blade 866. When blade cover 868 is not being forced against tissue 870, it may move back into its covering position. Thus, cutting blade 866 may be automatically exposed when cutting tissue 870, and may be automatically covered when not cutting tissue 870.

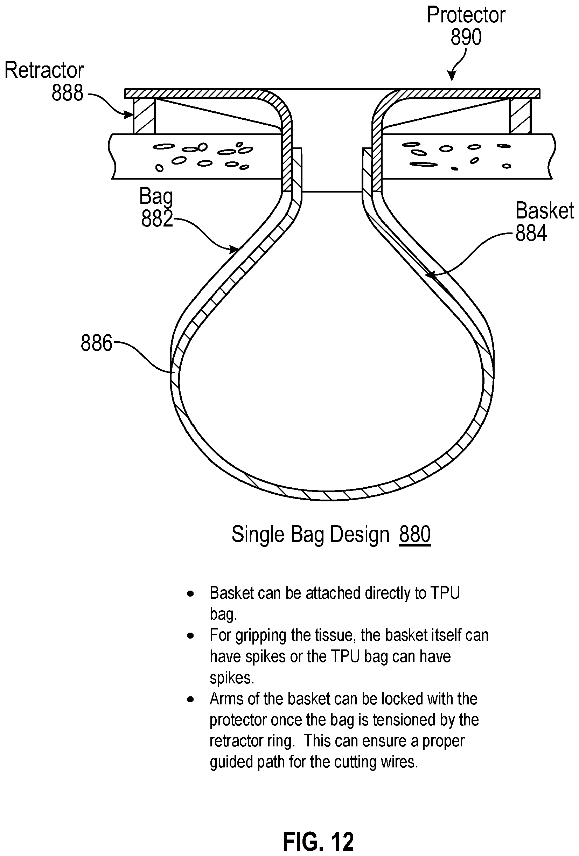

Fig. 12 shows a tissue extraction device 880 including an outer layer or bag 882, and no inner layer made of mesh. Basket 884 may cover one or more cutting elements, such as any of the aforementioned cutting elements. A tissue specimen may be placed into bag 882, and basket 884 may be pulled up. Additionally or alternatively, bag 882 may be rolled on a ring 888 (e.g., similar to retractor ring 802 (Fig. 6A)) to hold the tissue steady. The inner surfaces of arms 886 may be textured (e.g., roughened, covered by protrusions, and/or covered by indentations) to help hold the tissue in place. Optionally, arms 886 may be connected by slim sutures (not shown) to help compress the tissue and/or maintain positioning of arms 886. It is also contemplated that a protector 890 (e.g., similar to protector snap disc 804 (Fig. 81A)) may be coupled to ring 888 and/or bag 882.

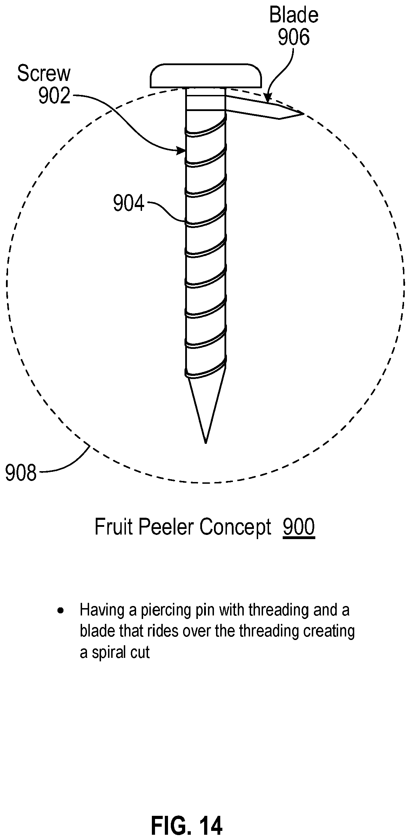

Fig. 14 shows a cutting element 900 including a screw 902 with external threading 904, and a blade 906 that is coupled to the screw 902. Blade 906 may ride along threading 904. Screw 902 may be screwed into a tissue specimen 908, which may be contained in any of the aforementioned bags, and blade ride along threading 904 to cut tissue specimen 908.

In another example, any of the aforementioned mesh layers may be used to cut tissue. This may be achieved by pulling the mesh layers against tissue contained therein until the struts of the mesh layers cut into the tissue. The struts may include one or more sharpened edges to facilitate their entry into the tissue.

In another example, any of the aforementioned cutting elements may be operatively coupled to a transducer (not shown) that may vibrate one or more portions of the cutting elements at a high frequency (e.g., at an ultrasonic frequency). This vibration may facilitate cutting of tissue by the cutting elements. Additionally or alternatively, any of the aforementioned cutting elements may be operatively coupled to an electrosurgical unit that may provide electrical energy to one or more electrode portions of the cutting elements. Once electrified, the electrode portions may be able to cut through tissue more easily.

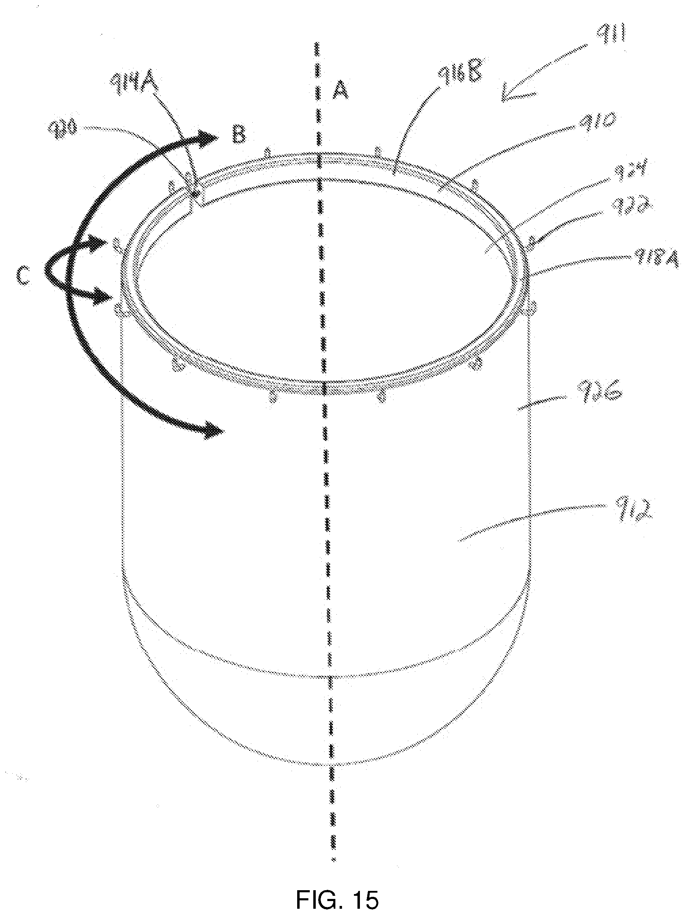

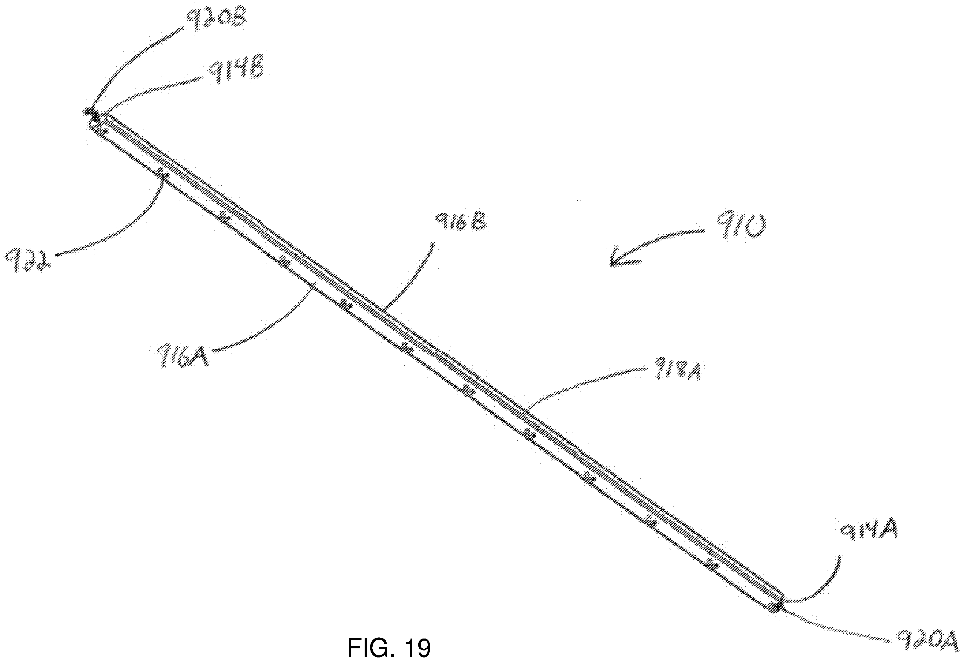

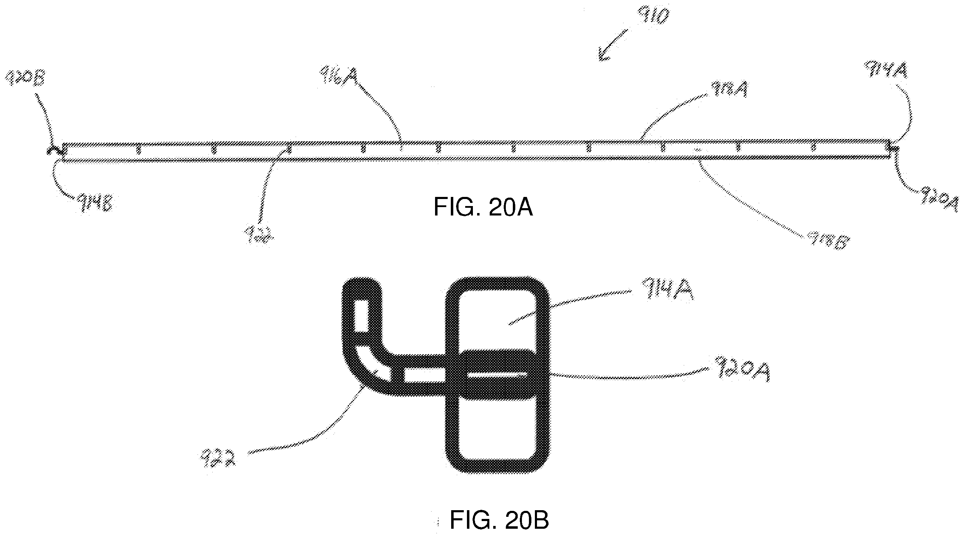

Figs. 15-21B show aspects of an exemplary bag assembly 911. Bag assembly 911 may include a bag 912 and a retractor ring 910. For reference purposes, a central longitudinal axis (A) of bag assembly 911, bag 912, and/or retractor ring 910; a toroidal direction (B) of retractor ring 910, and a poloidal direction of retractor ring 910, also are shown. A central toroidal axis (not shown) of retractor ring 910 may include an axis running through a center of retractor ring 910 in toroidal direction B. When retractor ring 910 rotates (e.g., everts and/or inverts) about its central toroidal axis, the retractor ring 910 is rotating in the poloidal direction. These axes and directions also may be applicable to the other retractor rings disclosed herein.

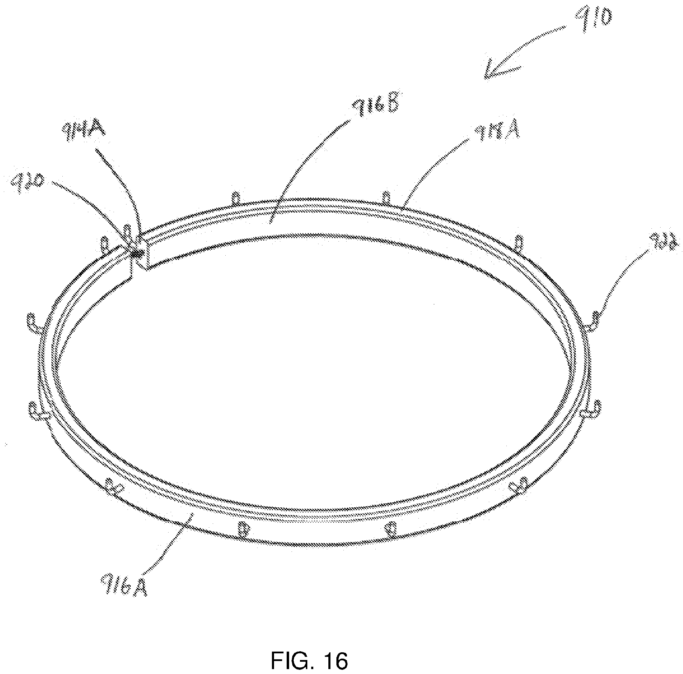

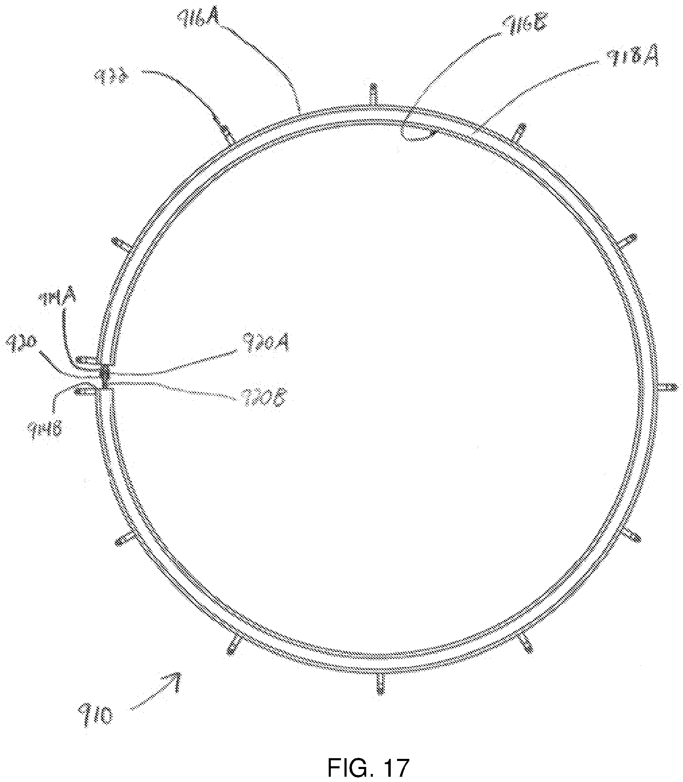

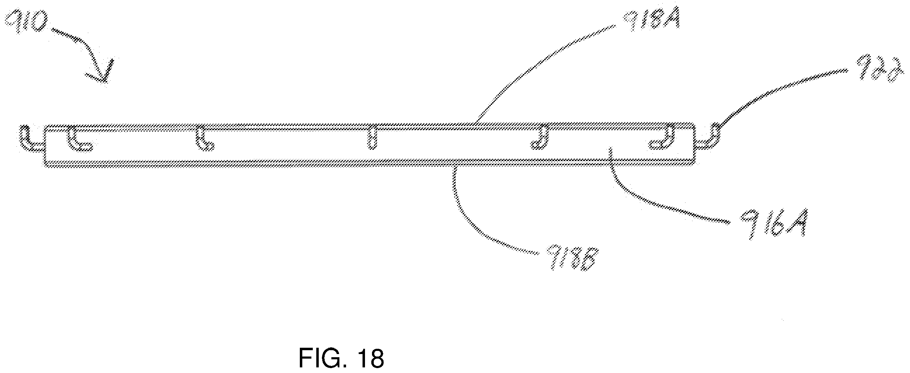

Retractor ring 910 is shown in greater detail in Figs. 16-19. Retractor ring 910 may include two end faces 914A and 914B (see, e.g., Fig. 17), two side faces 916A and 916B (see, e.g., Fig. 17), a top face 918A, a bottom face 918B (see, e.g., Fig. 18), a joining arrangement 920 (see, e.g., Fig. 17), and one or more hooks 922 (see, e.g., Fig. 16). One or more of the edges of retractor ring 910, formed where the various faces intersect, may be beveled, rounded, convex, or concave. Retractor ring 910 may be separate from bag 912, at least initially, and may be coupled to bag 912 when in use. It is contemplated, for example, that retractor ring 910 and bag 912 may be individual components of a kit.

When retractor ring 910 is coupled to bag 912 (see, e.g., Fig. 15), side face 916A may contact an interior surface 924 of bag 912. End faces 914A and 914B may extend transverse (e.g., substantially perpendicular) to side faces 916A and 916B. Joining arrangement 920 may link end face 914A to end face 914B to keep retractor ring 910 in its annular or toroidal form. Joining arrangement 920 may include, for example, one or more of an eye bolt, eye lag, u-bolt, j-bolt, and/or any other suitable fastener that may be coupled to at least one of end faces 914A and end 914B, to secure end face 914A to end face 914B. In one example, joining arrangement 920 may include a first joining member 920A (see, e.g., Fig. 17) on end face 914A that may be selectively coupled to a second joining member 920B on end face 914B. Joining arrangement 920 may be used to constrain retractor ring 910 in its annular or toroidal configuration before, while, or after retractor ring 910 is coupled to bag 912.

Hooks 922 (see, e.g., Fig. 16), or other suitable fastening devices or materials on side face 916A, may couple retractor ring 910 to bag 912. Hooks 922 may curve or bend upward (proximally). The curve/bend may help ensure that retractor ring 910 and bag 912 stay coupled during rolling by preventing bag 912 from sliding off of hooks 922. Hooks 922 may be inserted into one or more apertures in bag 912, may puncture bag 912 to form apertures therein, and/or may be inserted into openings in a mesh bag (not shown) within bag 912 or used in place of bag 912. One advantage of having retractor ring 910 separate from bag 912, at least initially, is that retractor ring 910 need not be inserted into the subject's body with bag 912, allowing insertion of bag 912 through a smaller incision or opening. Further, retractor ring 910 will not be in the way while the tissue specimen is being inserted into bag 912, and/or while the proximal portion of bag 912 is being extracted from the subject's body. Rather, retractor ring 910 may be introduced after the proximal portion of bag 912 already has been withdrawn from the subject's body. It should be understood that the same advantage exists for the other embodiments of retractor ring described herein.

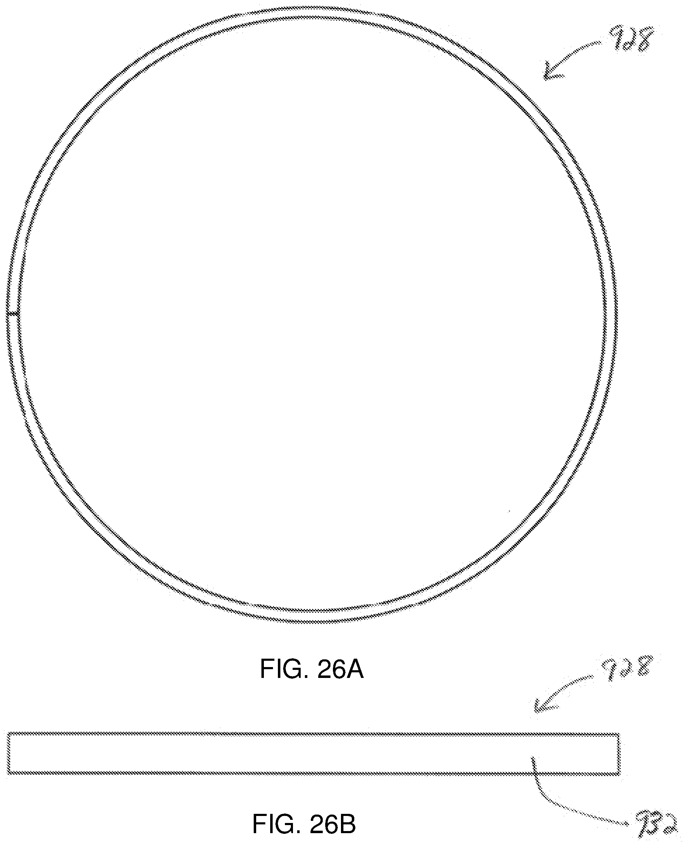

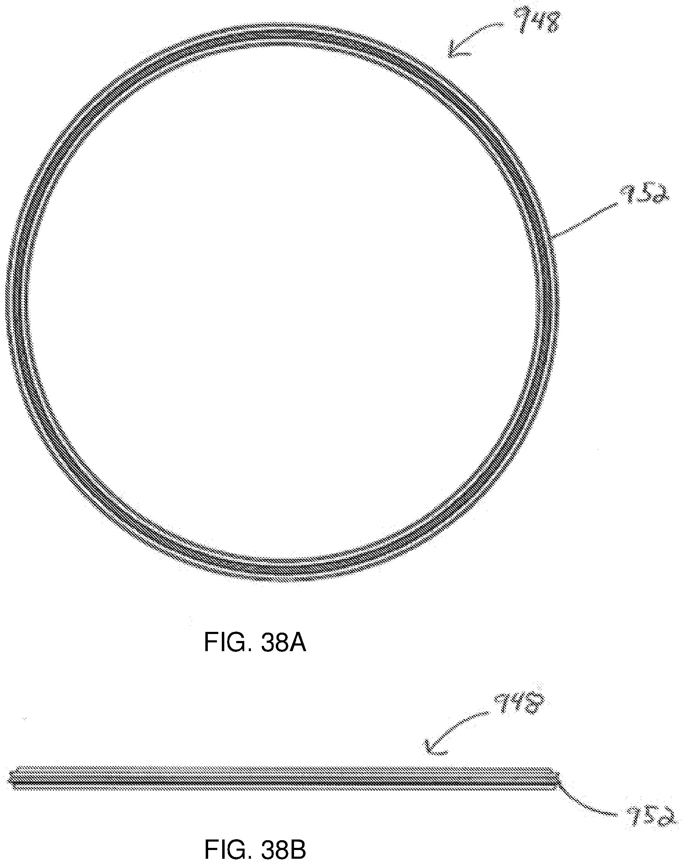

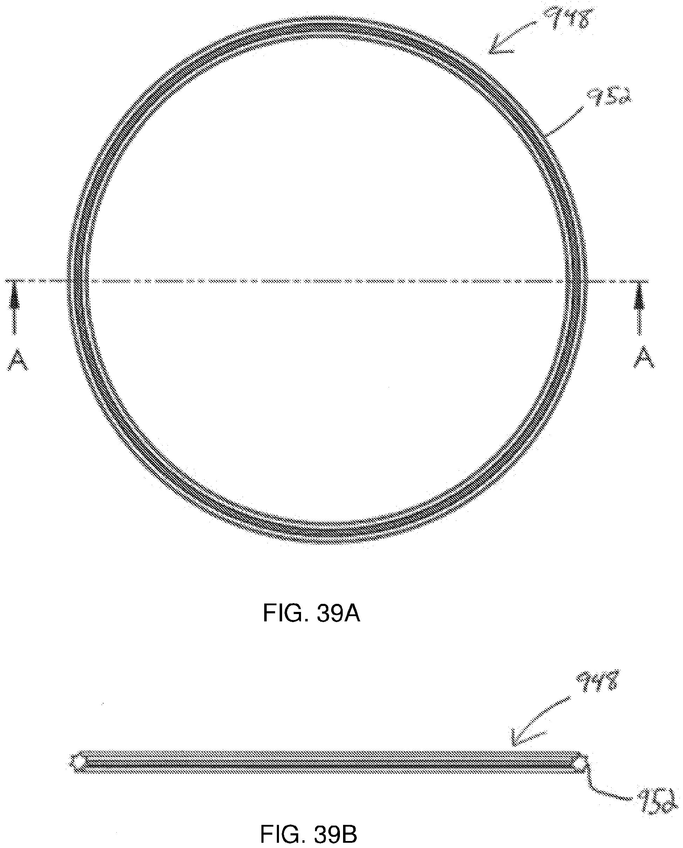

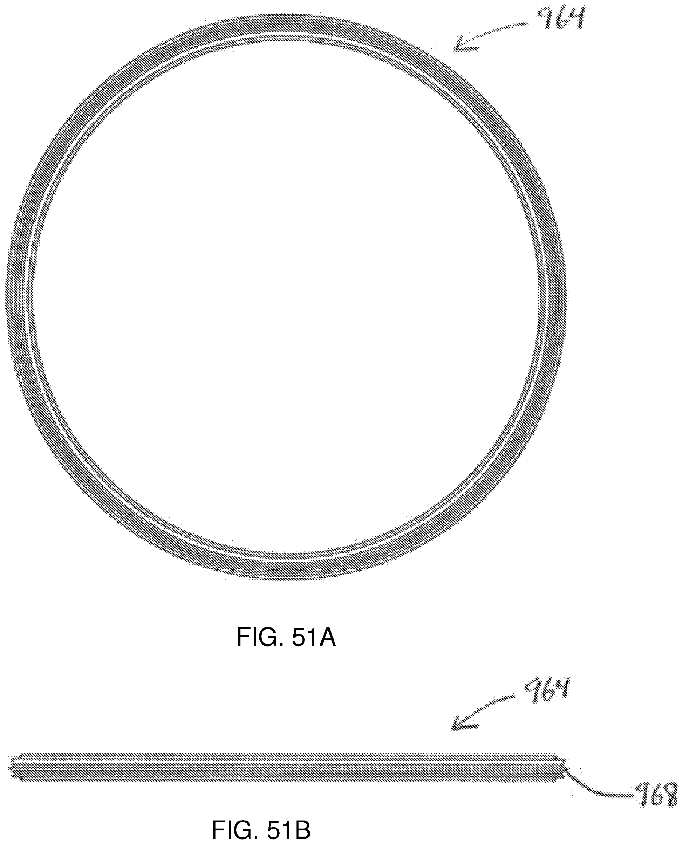

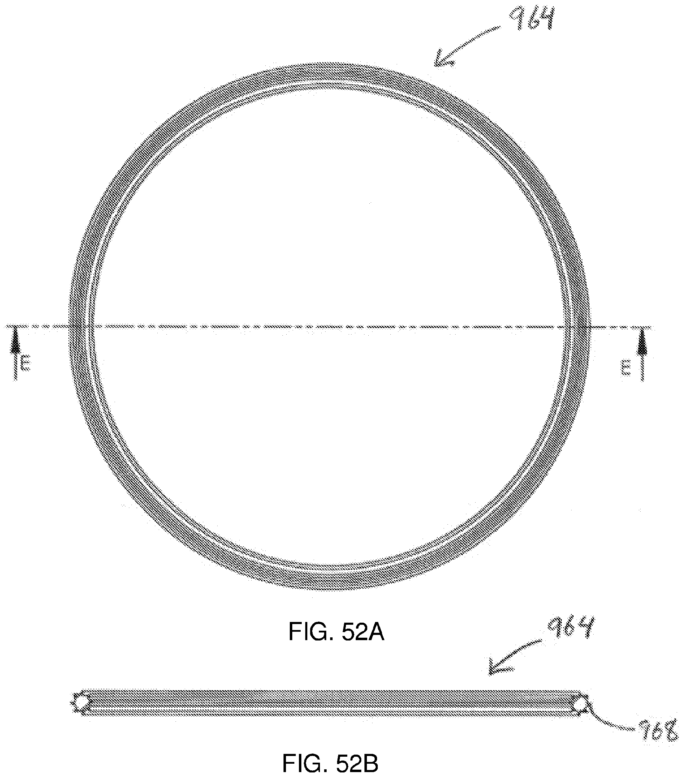

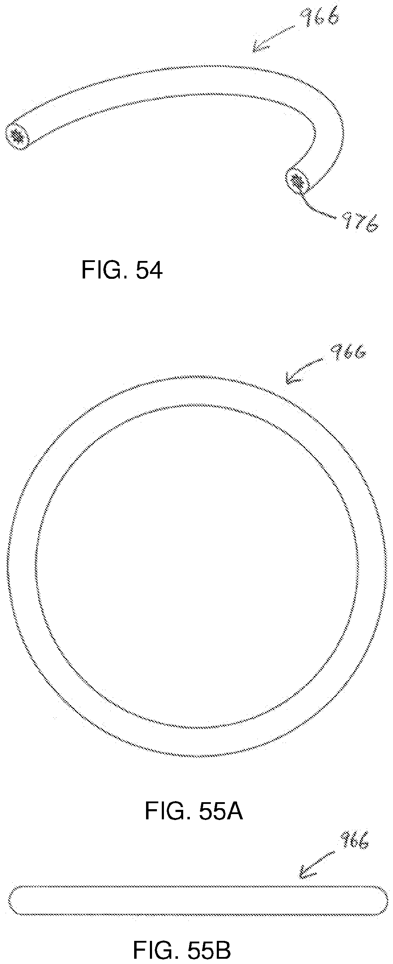

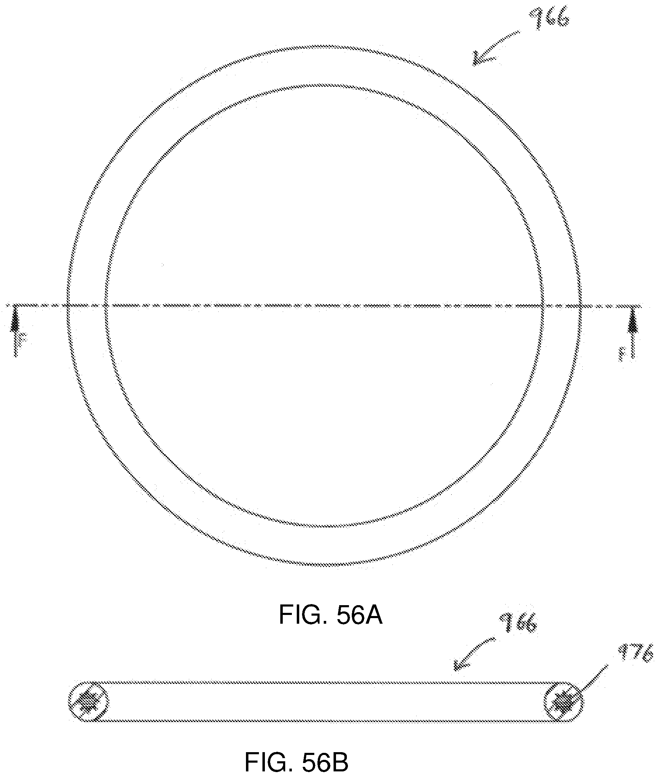

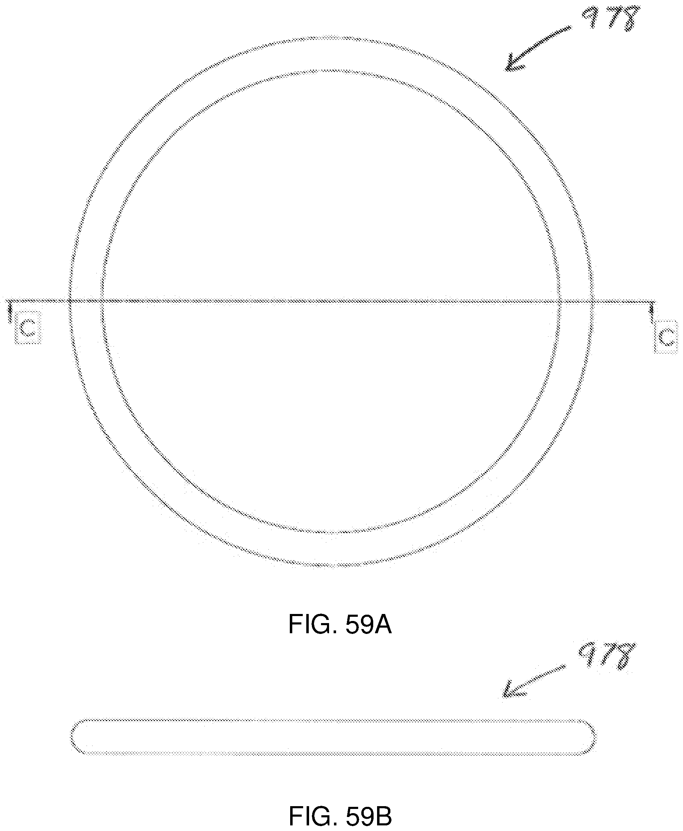

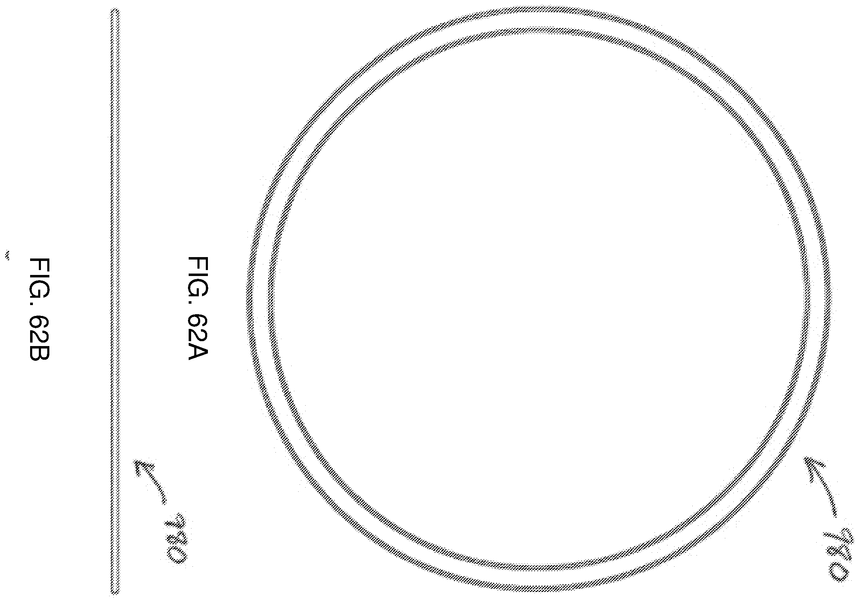

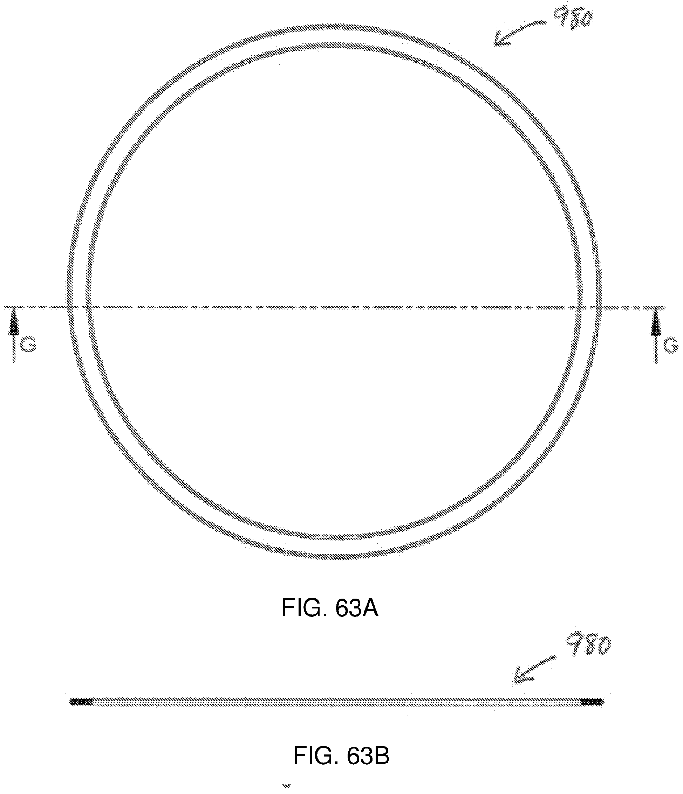

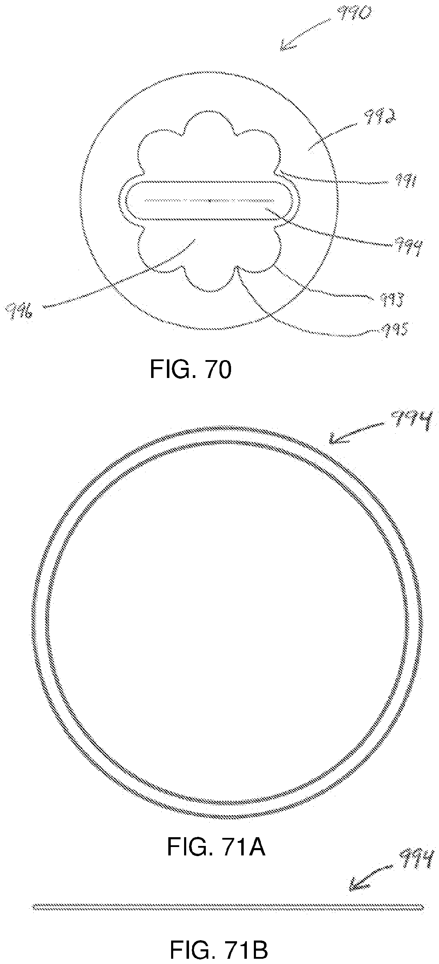

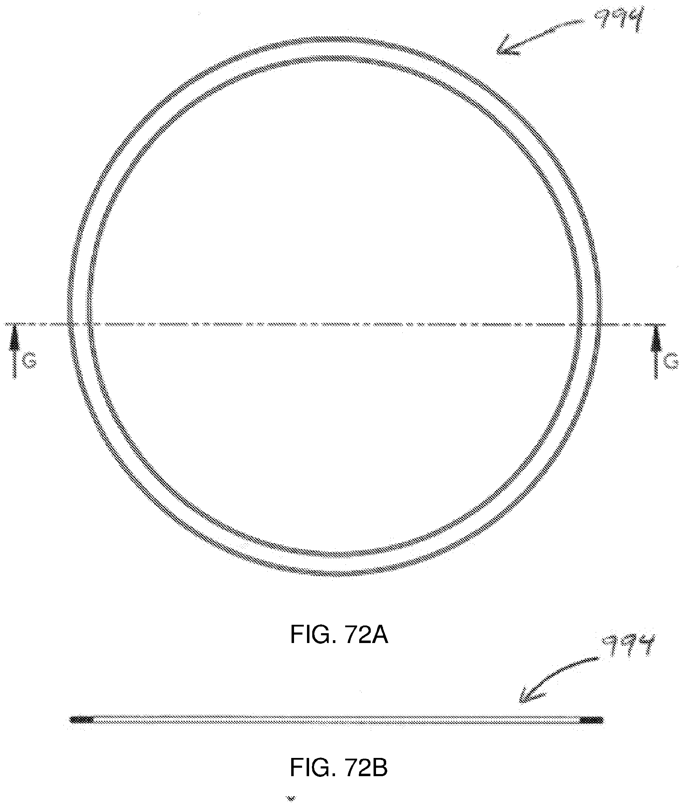

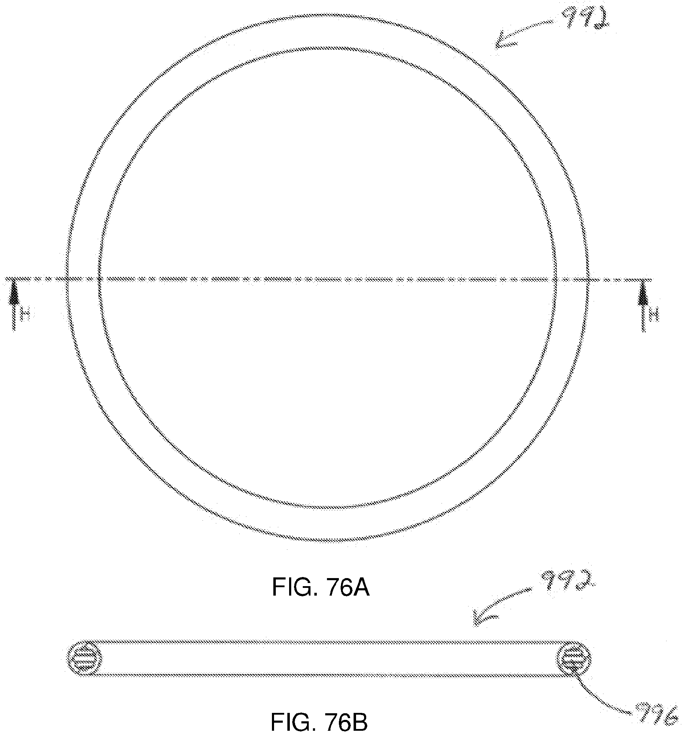

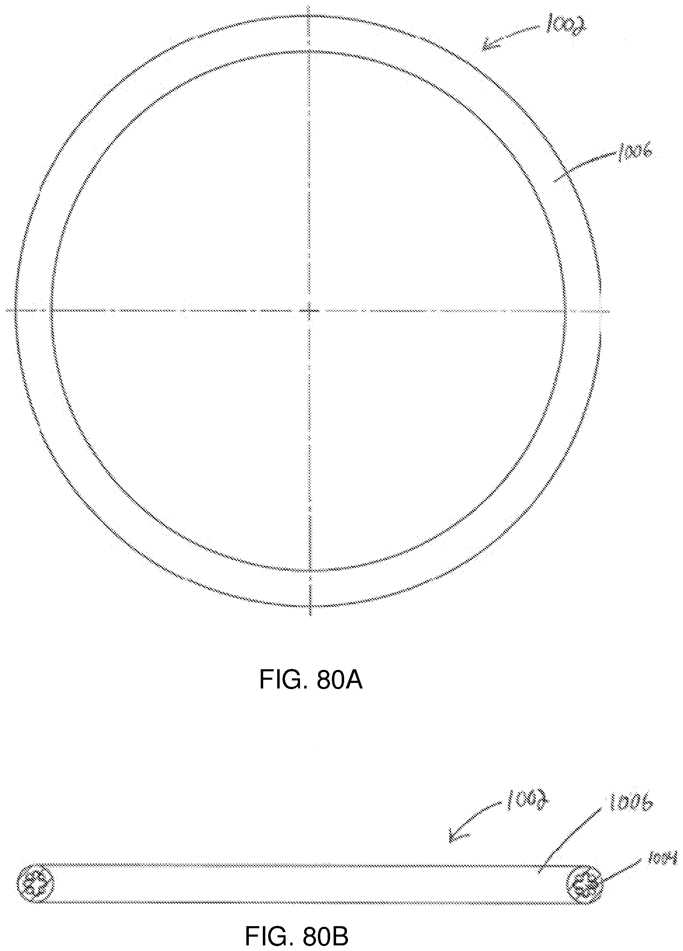

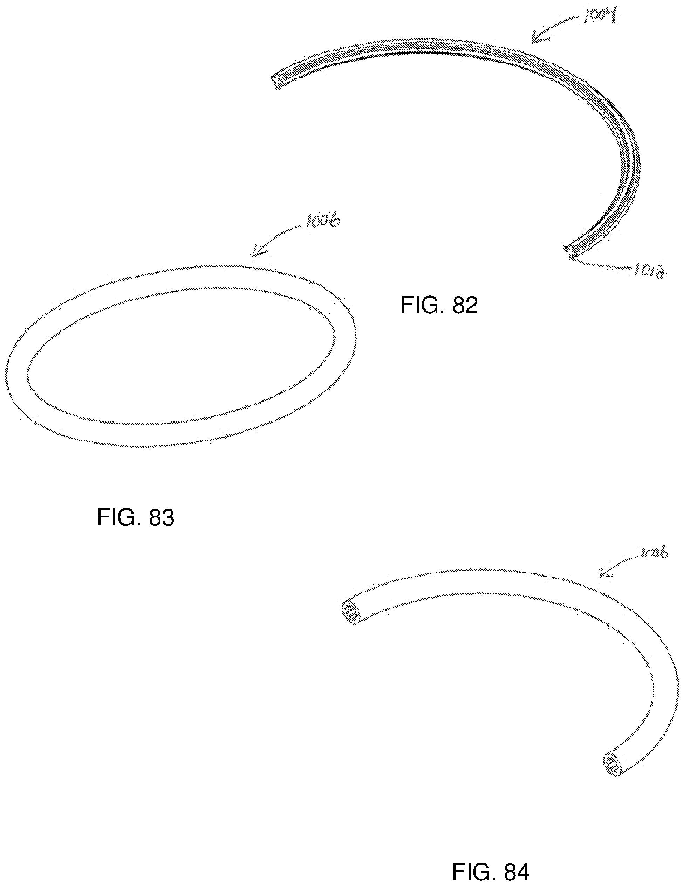

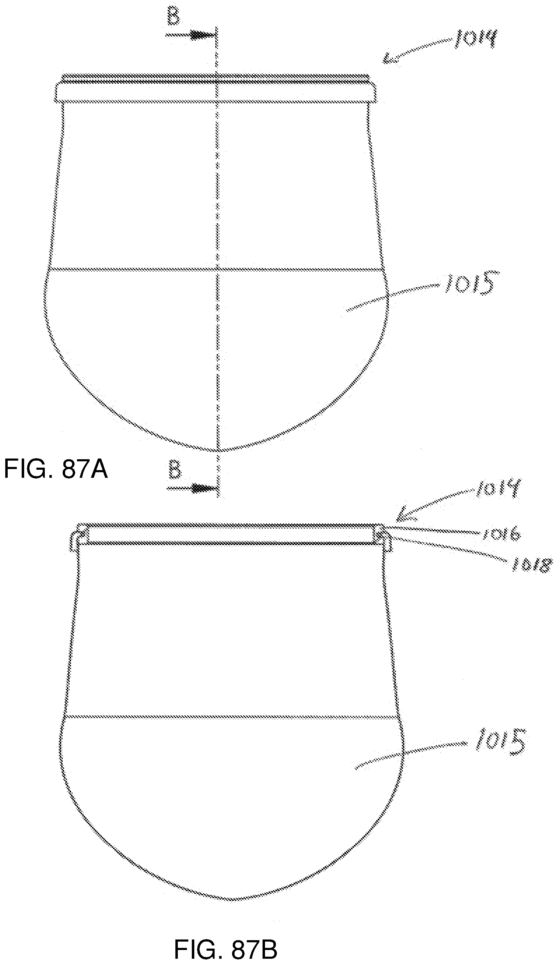

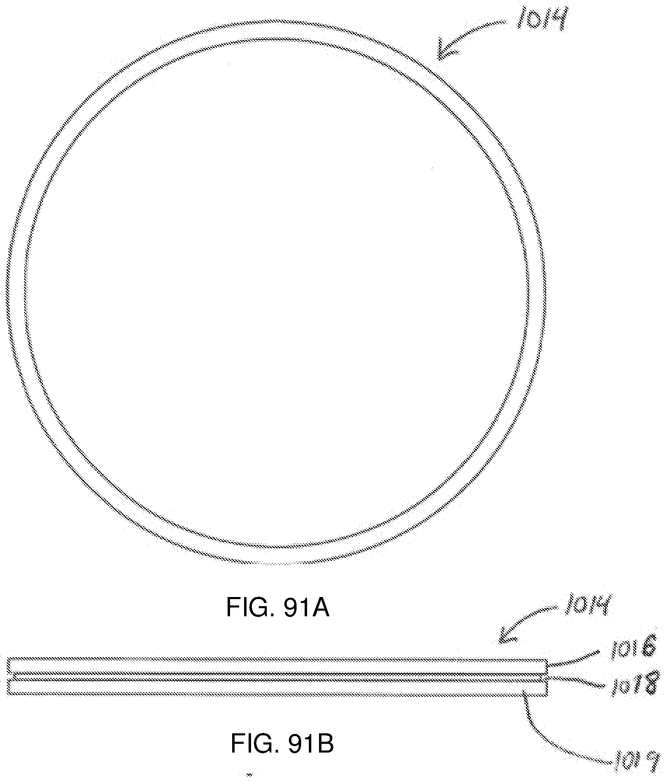

Retractor ring 910 may be rotated (e.g., inverted and/or everted) about its central toroidal axis to wrap bag 912 around retractor ring 910. Retractor ring 910 may move from one stable or equilibrium state to another one during rotation. An equilibrium state of retractor ring 910 is shown, for example, in Figs. 15-91, where side faces 916A and 916B may extend substantially parallel to a central longitudinal axis of retractor ring 910. Retractor ring 910 may have two equilibrium states-a first equilibrium state where side face 916A faces radially-outwardly and side face 916B faces radially-inwardly, and a second equilibrium state where side face 916B faces radially-outwardly and side face 916A faces radially-inwardly. A non-equilibrium state of retractor ring 910 is not depicted, but may correspond to a configuration in which side faces 916A and 916B extend transverse (e.g., substantially perpendicular to) the central longitudinal axis of retractor ring 910. When retractor ring 910 is in the non-equilibrium state, internal forces in retractor ring 910 may bias retractor ring 910 to move toward an equilibrium state. As such, less of an external force may be needed to move retractor ring 910 from a non-equilibrium state to an equilibrium state than from an equilibrium state to a non-equilibrium state.

When retractor ring 910 is in its constrained configuration, and in an equilibrium state, one of side face 916A and side face 916B may face radially-outwardly, and may be in tension, while the other of side face 916A and side face 916B may face radially-inwardly, and may be in compression. The type of force associated with each of side faces 916A and 916B may reverse each time retractor ring 910 is rotated from one of its equilibrium states to the other.

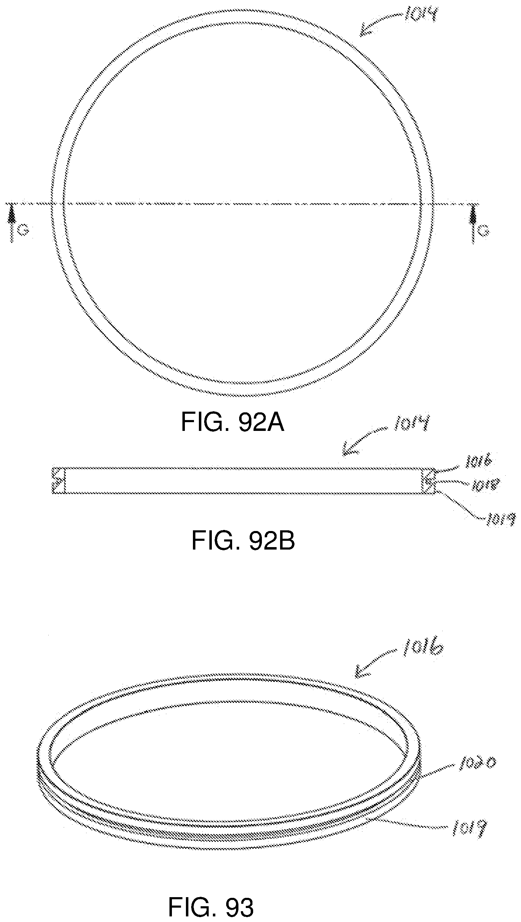

Retractor ring 910 may have a tendency to straighten (see, e.g., Figs. 92, 20A, and 21A). For example, retractor ring 910 may be self-biased to return to its expanded configuration in the absence of a constraining force holding retractor ring 910 in its annular or toroidal configuration. For example, retractor ring 910 may expand when joining arrangement 920 is undone. It may be easier, in some instances, for a user to connect retractor ring 910 to bag 912 when retractor ring 910 is in the expanded state than when retractor ring 910 is in the constrained state.

Rotating retractor ring 910 may roll a proximal portion of bag 912 onto retractor ring 910, and around retractor ring 910 in one or more layers, imparting tension in a distal portion of bag 912 that holds a tissue specimen. This may cause the distal portion to compress the tissue specimen. The user also may pull bag 912 proximally, by pulling retractor ring 910 proximally, to compress the distal portion of bag 912 on the tissue specimen, before, while, and/or after rolling bag 912 about retractor ring 910. Additionally or alternatively, the user may roll retractor ring 910 without pulling retractor ring 910 proximally.

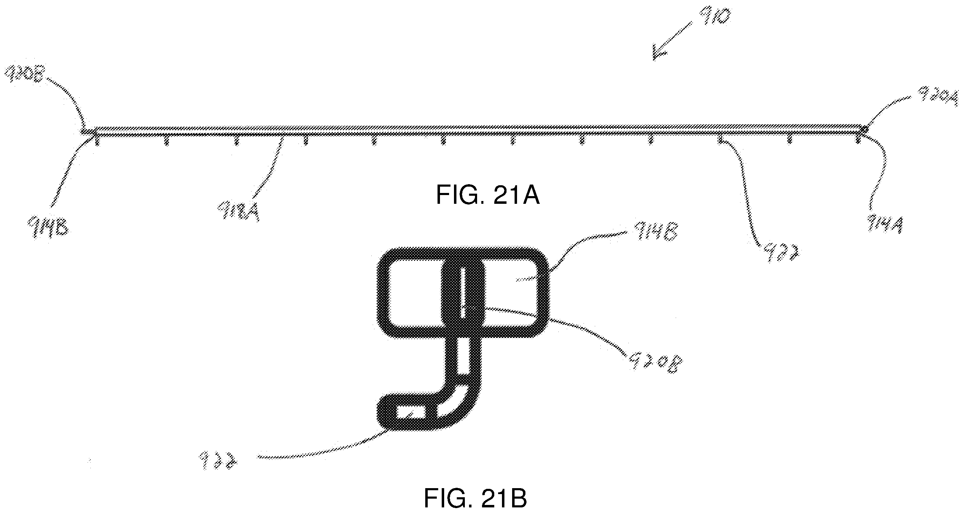

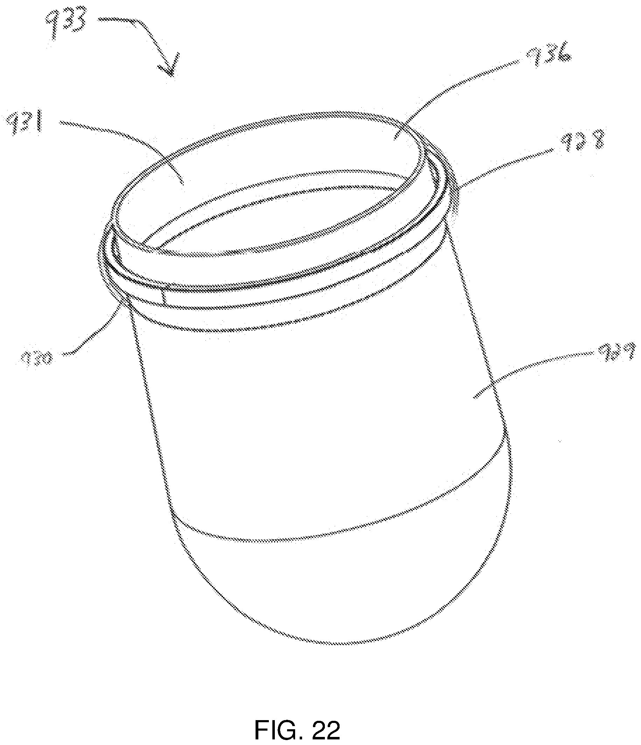

Figs. 22-27B show exemplary aspects of a bag assembly 933. Bag assembly 933 may include a bag 929 and a retractor ring 928. Bag 929 may include a channel 930 for receiving retractor ring 928, to couple retractor ring 928 to bag 929. Channel 930 may run transverse to a central longitudinal axis of bag 929. Channel 930 may project radially-outwardly from an exterior surface 926 (see, e.g., Fig. 21) of bag 929. Alternatively, channel 930 may project radially-inwardly from an interior surface 931 of bag 912. Channel 930 may be made of the same type of material as bag 929. Channel 930 may, for example, include a strip of material whose proximal and distal edges are sewn on, adhered to, or otherwise secured on/to exterior surface 926.

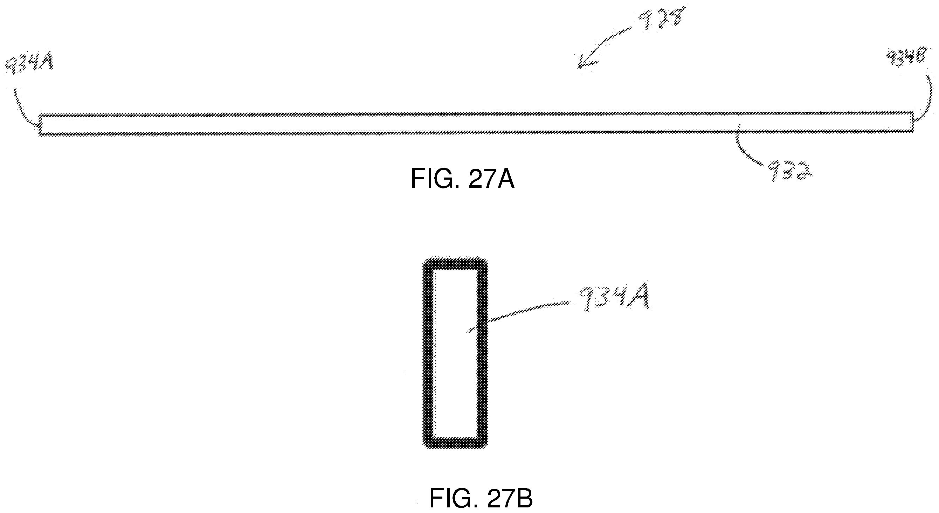

Channel 930 may have an opening (not shown), such as an aperture, slit, or other passage, for facilitating insertion of retractor ring 928 into channel 930. The user may slide retractor ring 928 into channel 930 while retractor ring 928 is in its expanded configuration (see, e.g., Fig. 27A). This insertion step may take place, for example, while the proximal portion of bag 929 is outside of the subject's body, and the distal portion of bag 929 is within the subject's body. Retractor ring 928 may bend into an annular or toroidal configuration to conform to channel 930. Alternatively, channel 930 may not have an opening, and retractor ring 928 may be pre-mounted within channel 930.

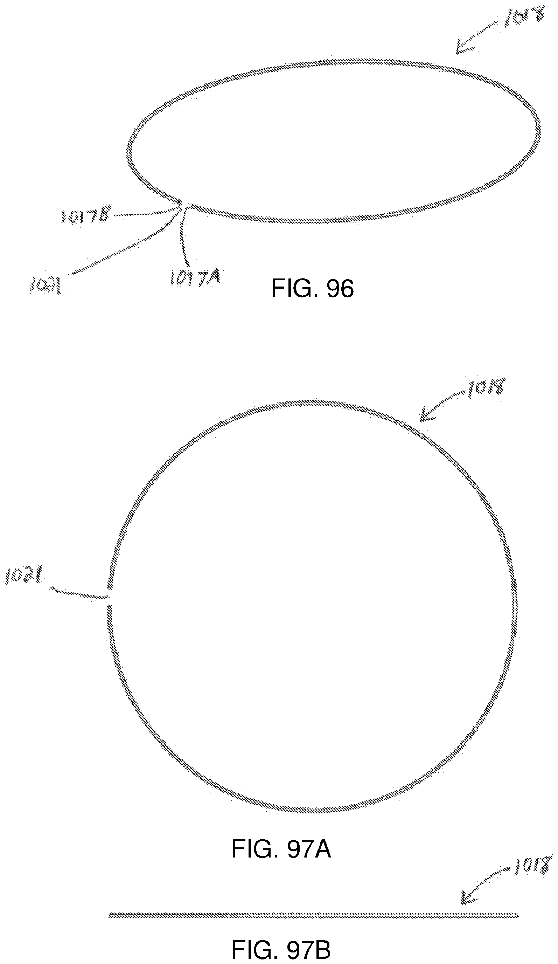

Channel 930 may be proximate to a bag opening 936 (see, e.g., Fig. 96) to assist the user with rolling a proximal portion of bag 929 and retractor ring 928. In use, the proximal portion of bag 929 may be wrapped around retractor ring 928, in one or more layers, by rotating (e.g., everting and/or inverting) retractor ring 928. Additionally or alternatively, the proximal portion of bag 929 may be wrapped around retractor ring 928 by rolling the proximal portion poloidally about retractor ring 928, in one or more layers, without rotating retractor ring 928.

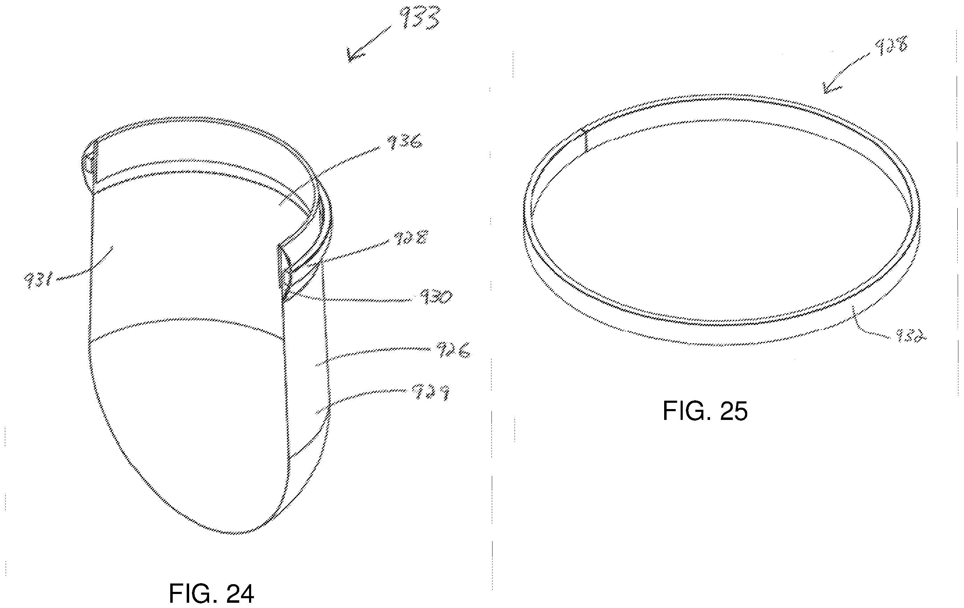

Figs. 25-27 show aspects of retractor ring 928 in greater detail. In one example, retractor ring 928 may be similar to retractor ring 910 (Fig. 16), except retractor ring 928 may be free of hooks 922 and/or joining arrangement 920 to facilitate inserting retractor ring 928 into channel 930. End faces 934A and 934B (see, e.g., Fig. 27A) of retractor ring 928 may be coupled to give retractor ring 928 an annular or toroidal shape. End faces 934A and 934B may be coupled by glue or other adhesive. Additionally or alternatively, retractor ring 928 may be constrained in its annular or toroidal configuration by channel 930, in which case glue or adhesive may be omitted.

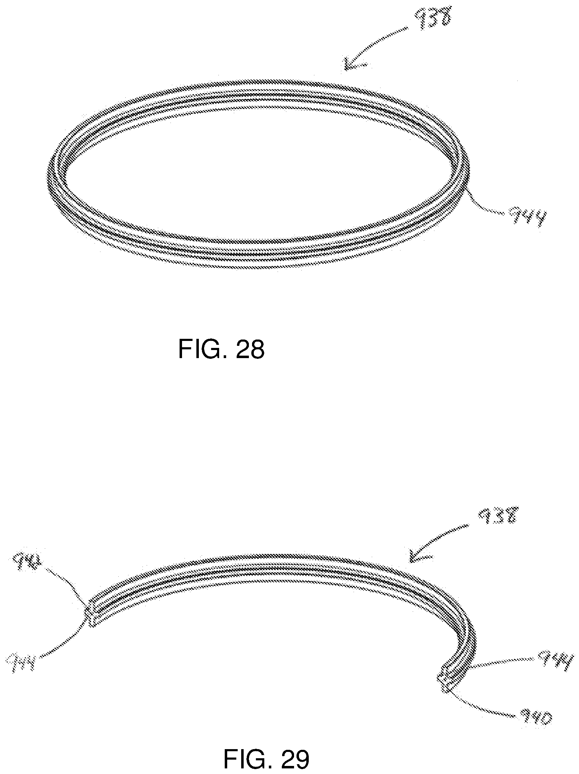

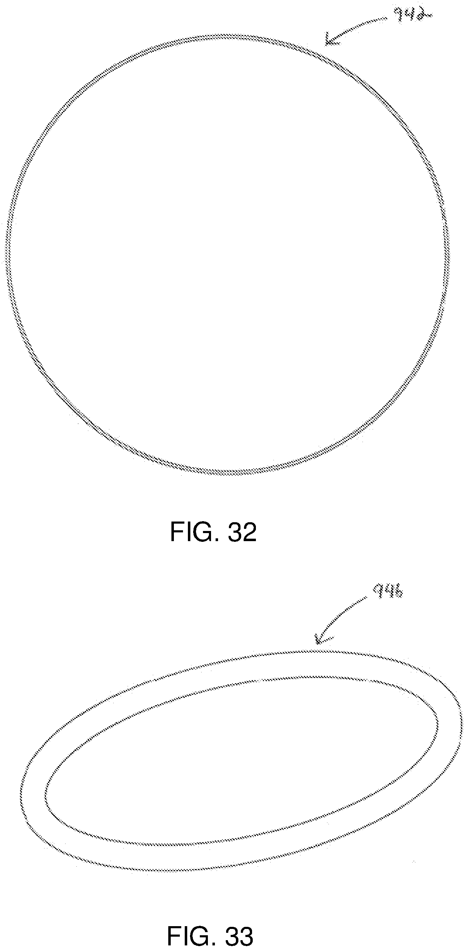

Figs. 28-31B show aspects of an exemplary retractor ring 938. Retractor ring 938 may, at least initially, be separate from any of the bags (not shown) disclosed herein, but may be coupled to a proximal portion of the bag when the proximal portion of the bag is to be rolled. Once coupled to the bag, retractor ring 938 may be rotated (e.g., inverted and/or everted) to roll the proximal portion of the bag onto retractor ring 938 in one or more layers, thereby tightening a distal portion of the bag around a tissue specimen (not shown). In one example, retractor ring 938 may include an inner reinforcement ring 942 (see, e.g., Fig. 32) in its center or core that may increase the strength and/or stiffness of retractor ring 938. Inner reinforcement ring 942 may help retractor ring 938 maintain its annular or toroidal shape even when being subjected to forces exerted on retractor ring 938 by the user and/or the bag. Inner reinforcement ring 942 may slidably engage the rest of retractor ring 938, such that inner reinforcement ring 942 may not evert or invert as the rest of retractor ring 938 rotates poloidally about inner reinforcement ring 942.

Retractor ring 938 may have a cross-shaped cross-section 940 (see, e.g., Fig. 29). More specifically, retractor ring 938 may have four splines 944 directed radially outwardly from its core. A first pair 944A of splines 944 may extend in opposite directions. A second pair 944B of splines 944 may also extend in opposite directions. The first pair 944A may extend transverse to the second pair 944B. For example, the first pair 944A may extend substantially perpendicular to the second pair 944B. Some of the splines 944 may be longer than the other splines 944. For example, the first pair 944A may be longer than the second pair 944B. Splines 944 may be rectangular in shape. Splines 944 may assist the user with rolling the ring by providing grips or holds by which to manipulate retractor ring 938, and/or also may assist with gripping the bag.

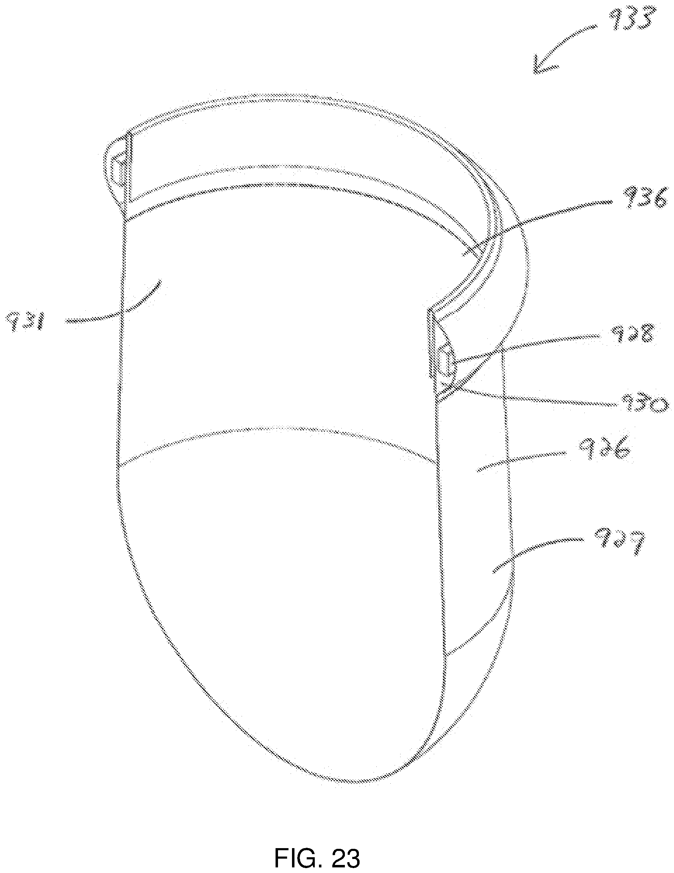

The retractor ring 938 may be coupled to the bag with hooks (similar to hooks 922 of Fig. 15), glue, or any other suitable means of attachment. Alternatively, retractor ring 938 may be coupled to the bag without a separate attachment means. For example, the user may press the proximal portion of the bag against retractor ring 938, start to roll the proximal portion of the bag about retractor ring 938 by inverting and/or everting retractor ring 938, then tuck a proximal end of the bag 912 under an oncoming rolled layer of the bag as the user continues to roll additional layers of the bag about retractor ring 938. Additionally or alternatively, retractor ring 938 may be inserted into or otherwise positioned in a channel (not shown, but similar to channel 930 of Fig. 23) on the bag.

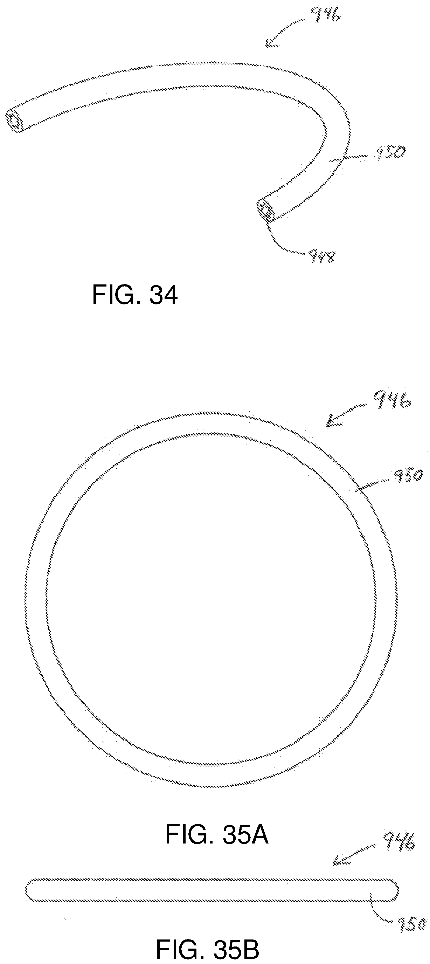

Figs. 33-43 show aspects of an exemplary retractor ring 946 that has an inner ring 948 (see, e.g., Fig. 34) and an outer ring 950. The act of rotating (e.g., inverting and/or everting) outer ring 946 around inner ring 948, may roll a bag (not shown) about retractor ring 946 in one or more layers. As with the other retractor rings and bags disclosed herein, retractor ring 946 may, at least initially, be separate from the bag. Retractor ring 946 may be coupled to a proximal portion of the bag in a similar manner to the other retractor rings, at the stage of a procedure where the proximal portion of the bag is to be rolled.

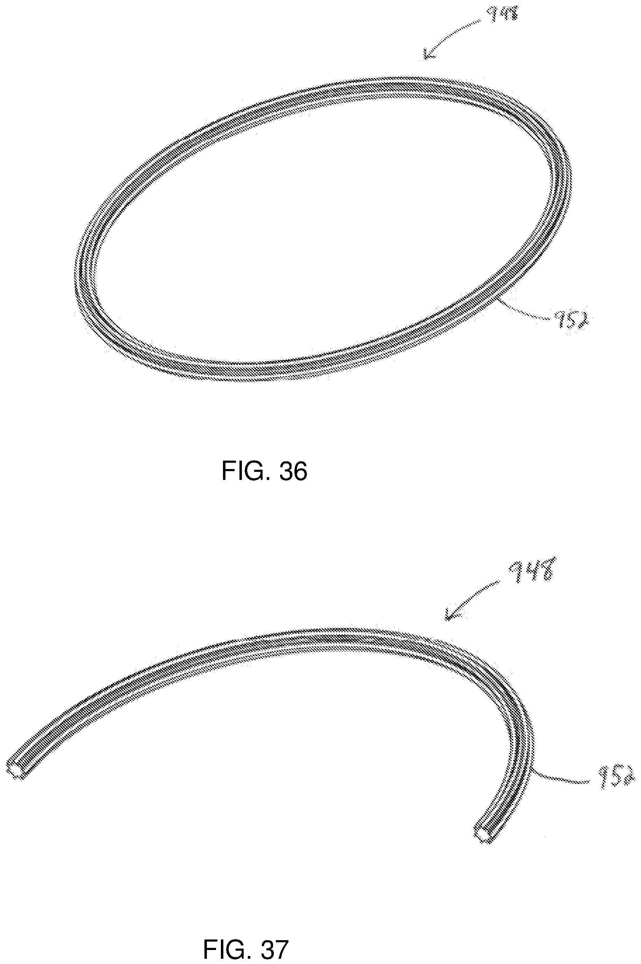

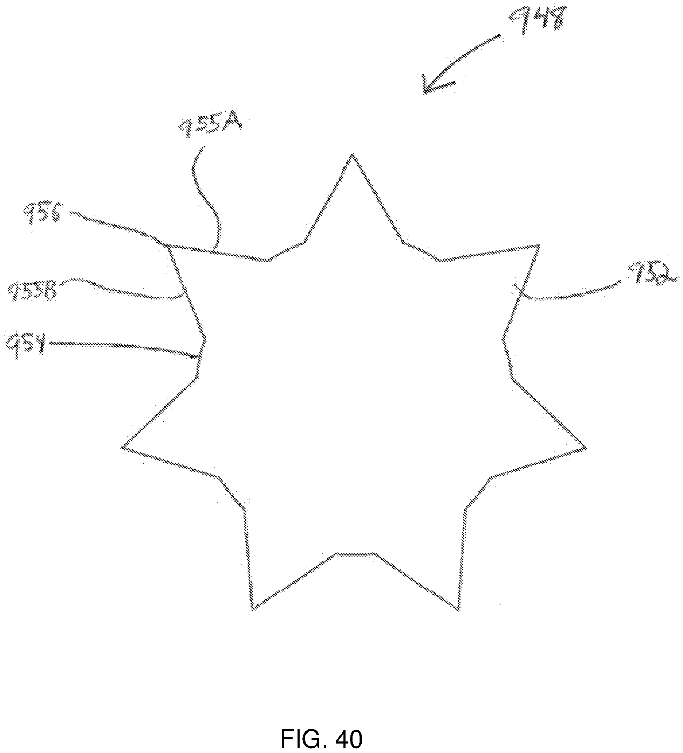

Inner ring 948 may have a plurality of radially-outwardly extending toroidal splines 952 (see, e.g., Figs. 37 and 40). Splines 952 may form toroidal peaks 956 and troughs 954 that alternate about inner ring 948 in the poloidal direction. Splines 952 may have angled faces 955A and 955B, and/or may be triangular, convex, helical, crowned, parallel, involute, or ball splines. Inner ring 948 may be more rigid than outer ring 950 such that outer ring 950 may deform and rotate (e.g., invert and/or evert) around inner ring 948. The rigidity of inner ring 948 also may help retractor ring 946 keep its annular or toroidal shape.

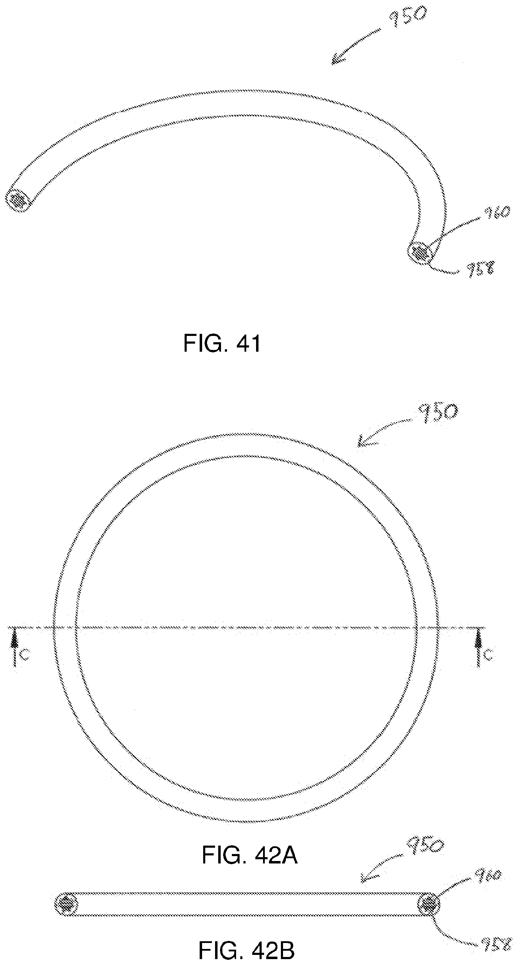

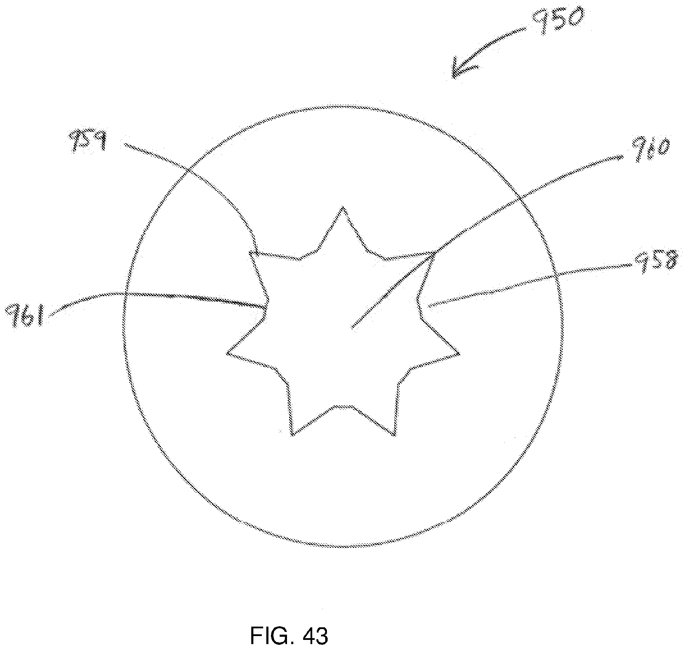

Outer ring 950 may have a plurality of radially-inwardly extending toroidal splines 958, forming toroidal peaks 961 and troughs 959 that alternate about outer ring 950 in the poloidal direction (see, e.g., Figs. 42B and 43). Splines 958 may circumscribe a channel 960 running through the center of outer ring 950, channel 960 being configured to receive inner ring 948. Channel 960 and inner ring 948 may be complementary in shape. Outer ring 950 may be more flexible than inner ring 948, and splines 958 may be capable of sliding over and across splines 952. Outer ring 950 may be coupled to the bag in any of the ways described in this disclosure (e.g., with hooks, glue, Velcro, adhesive, insertion into a channel, and/or other suitable form of coupling).

In use, retractor ring 946 may be coupled to a proximal portion of a bag in any of the ways described herein with respect to the other embodiments. Once coupled, outer ring 950 may rotate poloidally around inner ring 948 to roll the bag about outer ring 950. The flexibility of outer ring 950 may facilitate deformation and disengagement of splines 958 of outer ring 950 relative to splines 952 of inner ring 948. Outer ring 950 may lock onto inner ring 948 at certain orientations where peaks 961 of outer ring 950 are seated in troughs 954 of inner ring 948, and peaks 956 of inner ring 948 are seated in troughs 959 of outer ring 950. When in between the locking orientations, outer ring 950 may be inherently-biased to rotate into a locking orientation.

Figs. 44-57 show aspects of another exemplary retractor ring 962 having an inner ring 964 (see, e.g., Fig. 45) and an outer ring 966. Retractor ring 962 may operate in a manner similar to retractor ring 946 (see, e.g., Fig. 33). As with the other retractor rings and bags disclosed herein, retractor ring 962 may, at least initially, be separate from the bag. Retractor ring 962 may be coupled to a proximal portion of the bag in a similar manner to the other retractor rings, at the stage of a procedure where the proximal portion of the bag is to be rolled.

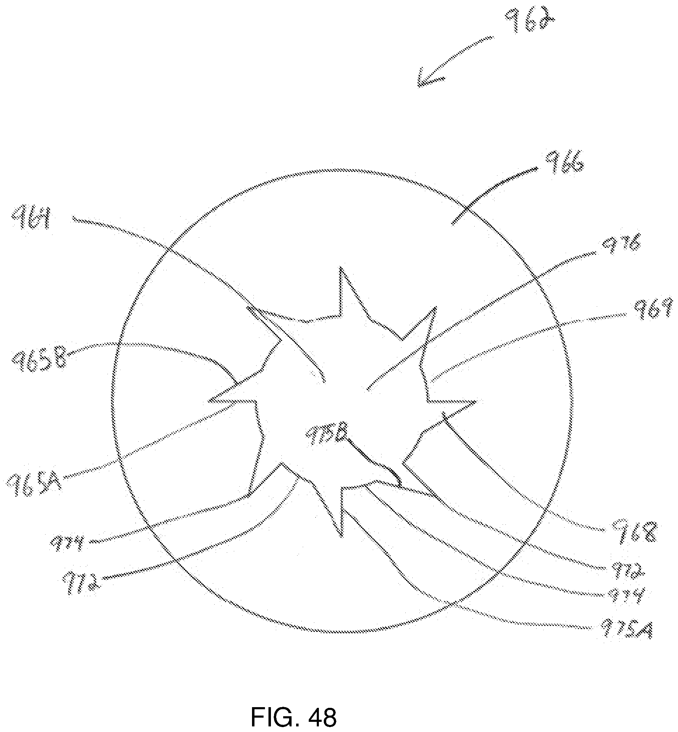

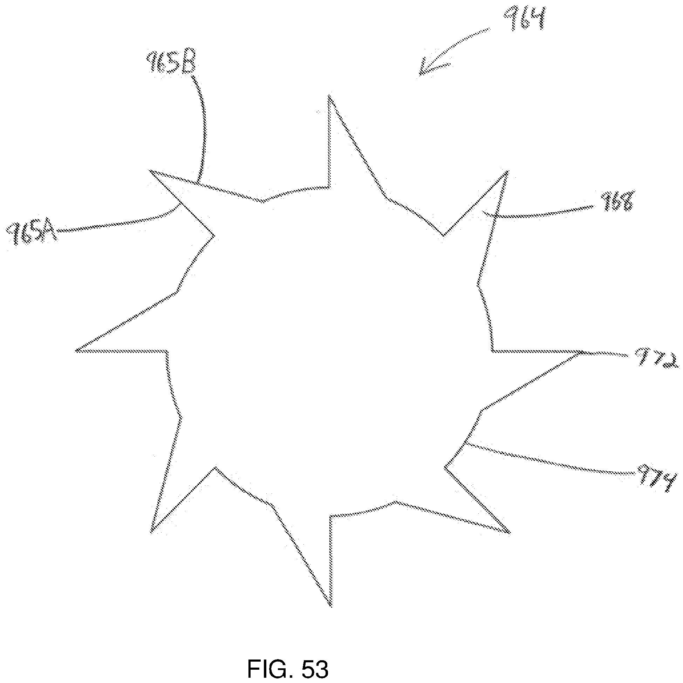

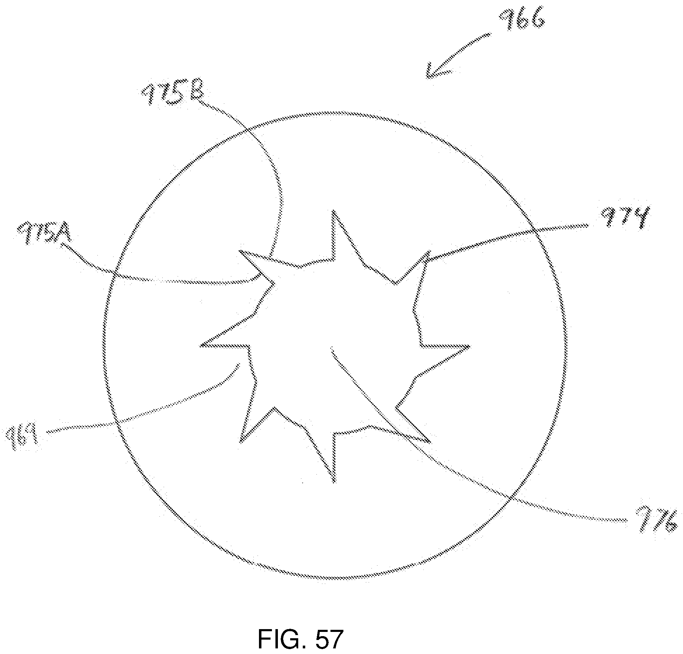

Splines 968 (see, e.g., Fig. 53) of inner ring 964 (like splines 952 of inner ring 948) may have two faces. A difference between inner ring 948 and inner ring 964 is that one of the faces, a face 965A, of spline 968, may be steeper than a face 965B. Outer ring 966 of retractor ring 962 may be similar to outer ring 950 of retractor ring 946. However, splines 969 of outer ring 966 (see, e.g., Fig. 57) may be shaped such that they may be complementary to splines 968 of inner ring 964. For example, peaks 972 and troughs 974 of outer ring 966, surrounding an outer ring channel 976 of outer ring 966, may interlock in a complementary manner with troughs 974 and peaks 972, respectively, of inner ring 964 (see, e.g., Fig. 48). Like splines 968 of inner ring 964, splines 969 of outer ring 966 may have two faces 975A and 975B, with face 975A being steeper than face 975B.

Engagement between steeper faces 965A and 975A may prevent or otherwise limit rotation of outer ring 966 in one direction because steeper faces 965A and 975A may not be slidably forced past each other. Rotation may be carried out in the opposite direction because less steep faces 965B and 975B may be slidably forced past each other more easily. This unidirectional rotation may facilitate rolling of the bag about retractor ring 962, while preventing or otherwise limiting unrolling of the bag off of retractor ring 962.

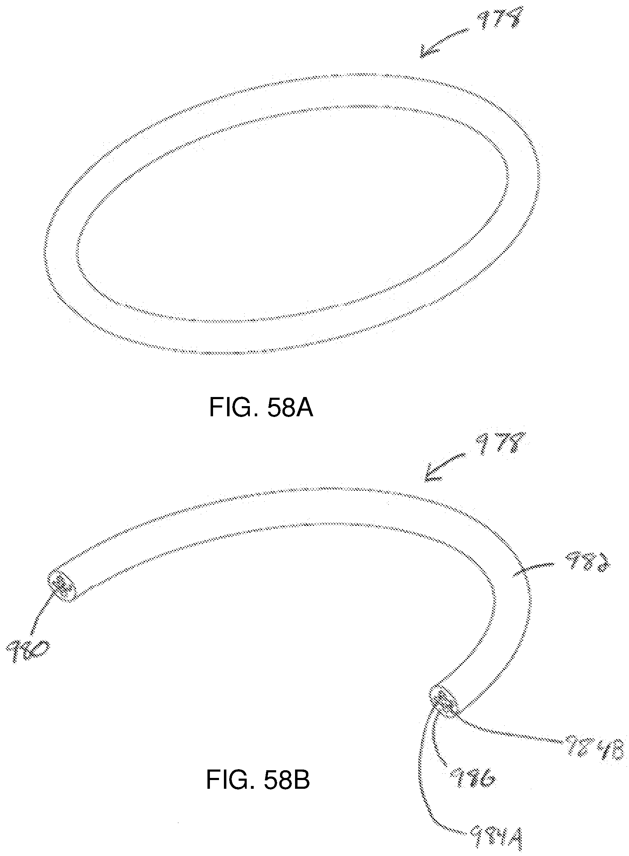

Figs. 58A-66 show aspects of an exemplary retractor ring 978 having an inner ring 980 (Fig. 58B) and an outer ring 982. As with the other retractor rings and bags disclosed herein, retractor ring 978 may, at least initially, be separate from the bag. Retractor ring 978 may be coupled to a proximal portion of the bag in a similar manner to the other retractor rings, at the stage of a procedure where the proximal portion of the bag is to be rolled.

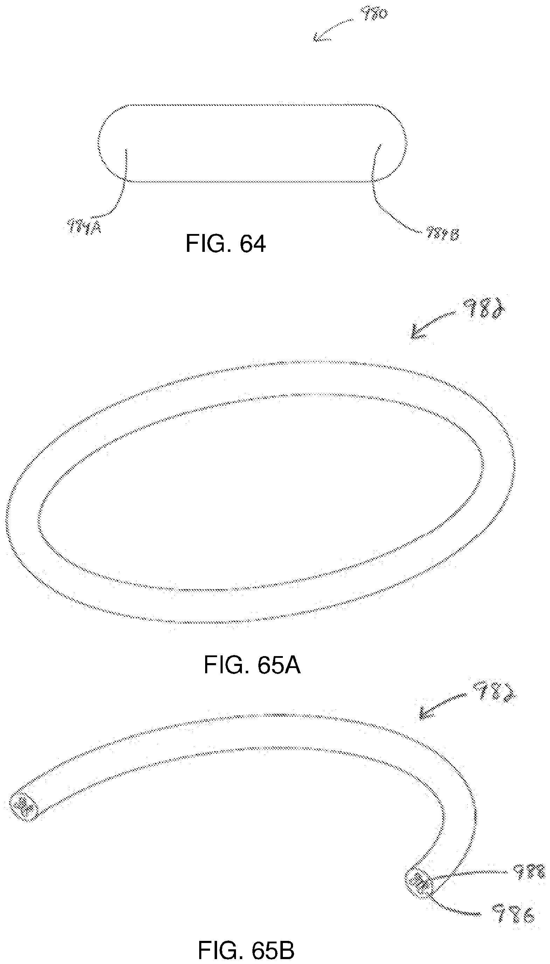

Outer ring 982 may be rotated (e.g., inverted and/or everted) about inner ring 980. Inner ring 980 may have a flat rectangular cross-sectional shape with rounded corners (see, e.g., Fig. 64). Radially-inner and radially-outer ends of inner ring 980 may form splines 984A and 984B that may extend substantially perpendicular to a central longitudinal axis of inner ring 980. Inner ring 980 may be stiffer than outer ring 982.

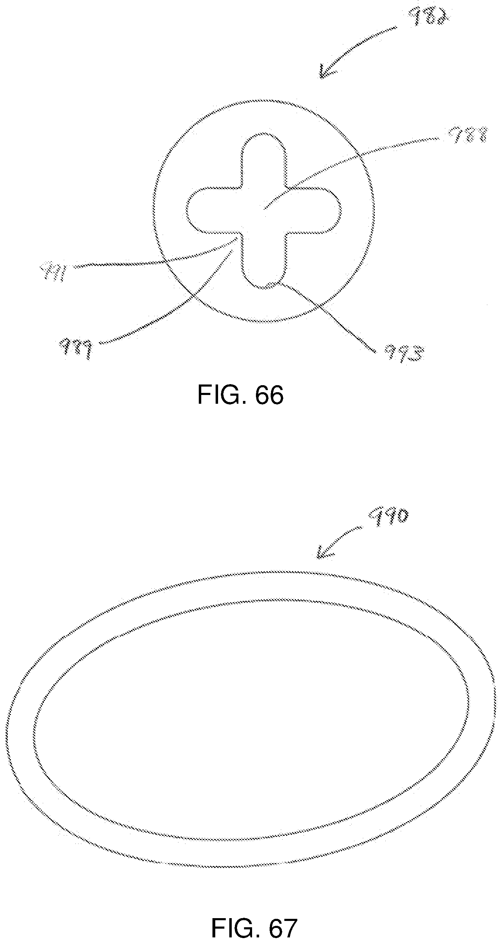

Outer ring 982 may have a channel 988 (see, e.g., Fig. 66) running through its center. Channel 988 may be circumscribed by radially-inwardly extending toroidal splines 989 of outer ring 982. Splines 989 may form alternating peaks 991 and troughs 993 extending in a poloidal direction about channel 988. In one example, peaks 991 and troughs 993 may give channel 988 a cross shape. Each of troughs 993 may be approximately ninety-degrees offset from an adjacent trough to provide similarly offset locking positions between inner ring 980 and outer ring 982. Opposing troughs 993 may be at least partially complementary to splines 984A and 984B of inner ring 980. Outer ring 982 may be in a stable state when splines 984 of inner ring 980 are received in troughs 993, and may be in an unstable state when splines 984 are in engagement with peaks 991. When outer ring 982 is in an unstable state with respect to inner ring 980, outer ring 982 may be inherently biased to rotate toward a stable state. It should be understood that outer ring 982 may be coupled to the bag at, for example, a proximal portion of the bag, in any manner described herein.

Figs. 67-77 show aspects of an exemplary retractor ring 990. As with the other retractor rings and bags disclosed herein, retractor ring 990 may, at least initially, be separate from the bag. Retractor ring 946 may be coupled to a proximal portion of the bag in a similar manner to the other retractor rings, at the stage of a procedure where the proximal portion of the bag is to be rolled.

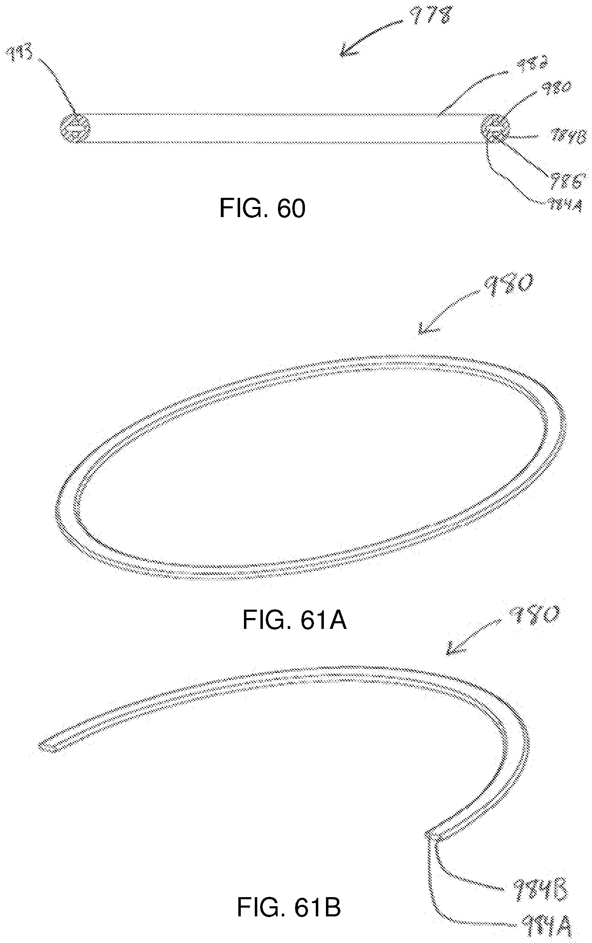

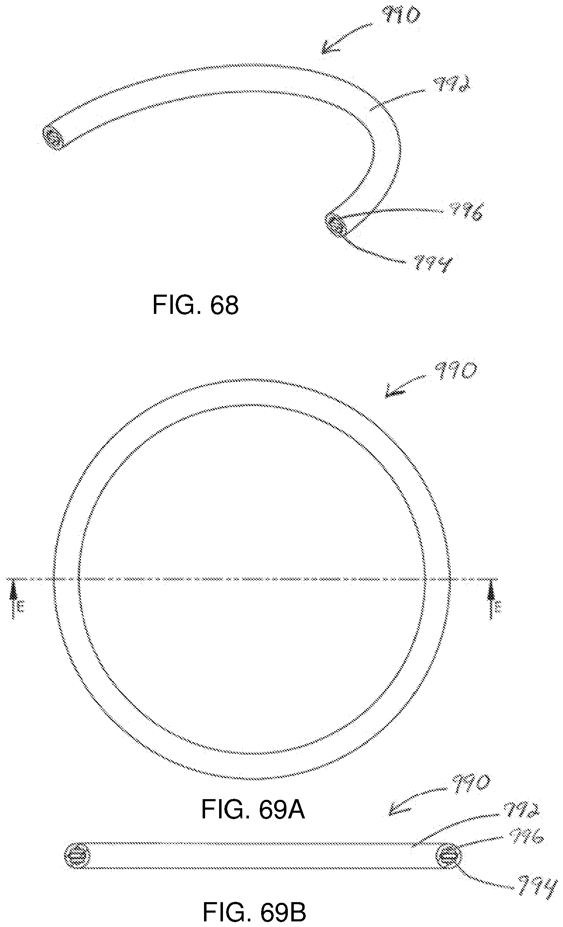

Retractor ring 990 may have an outer ring 992 that receives an inner ring 994 (see, e.g., Fig. 68). Inner ring 994 may be similar to, or identical to, inner ring 980 (Fig. 61A). Outer ring 992 may be similar to outer ring 982 (Fig. 65B), except outer ring 992 may include a central channel 996 surrounded by more splines 991 (and thus, more peaks 995 and troughs 993) than outer ring 982. The additional splines 991 may provide smaller increments between locking/stable orientations of inner ring 994 and outer ring 992, allowing for greater precision when rolling a bag around outer ring 982.

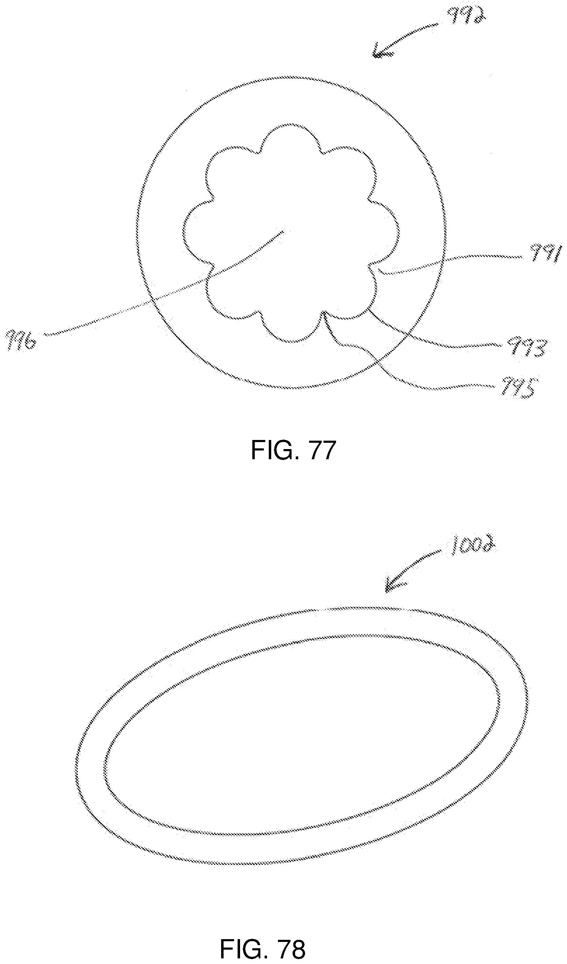

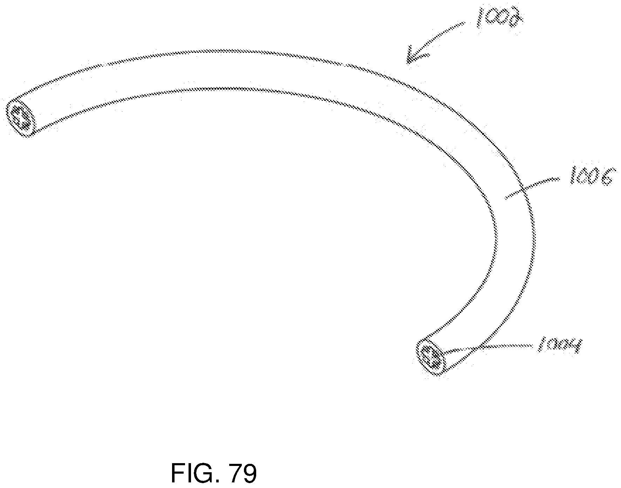

Figs. 78-84 show aspects of an exemplary retractor ring 1002 having an inner ring 1004 (see, e.g., Fig. 79) and an outer ring 1006. As with the other retractor rings and bags disclosed herein, retractor ring 1002 may, at least initially, be separate from the bag. Retractor ring 1002 may be coupled to a proximal portion of the bag in a similar manner to the other retractor rings, at the stage of a procedure where the proximal portion of the bag is to be rolled.

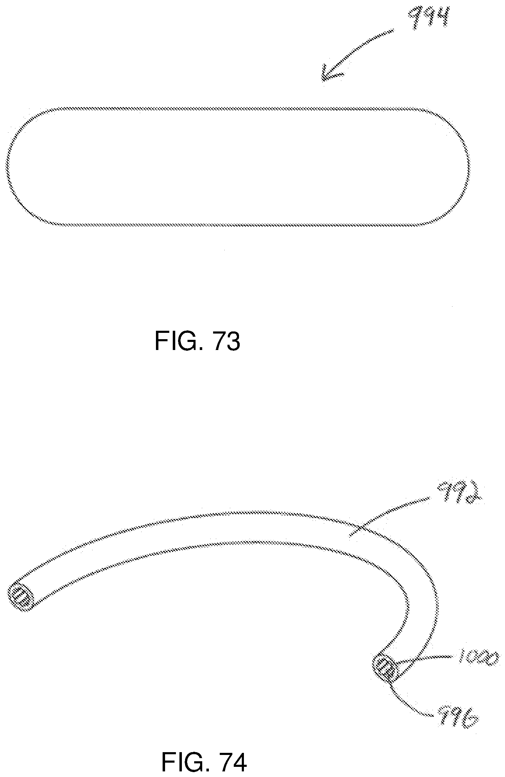

Outer ring 1006 may be similar to, or identical to, outer ring 992 (Fig. 77). Inner ring 1004 may be similar to inner ring 994 (Fig. 73). A difference between retractor ring 1002 and retractor ring 990 may be that inner ring 1004 may have an additional pair of splines 1012 (see, e.g., Fig. 81B) to provide additional contact points between outer ring 1006 and inner ring 1004. The addition of splines 1012 may result in inner ring 1004 having a cross-shaped cross-sectional shape.

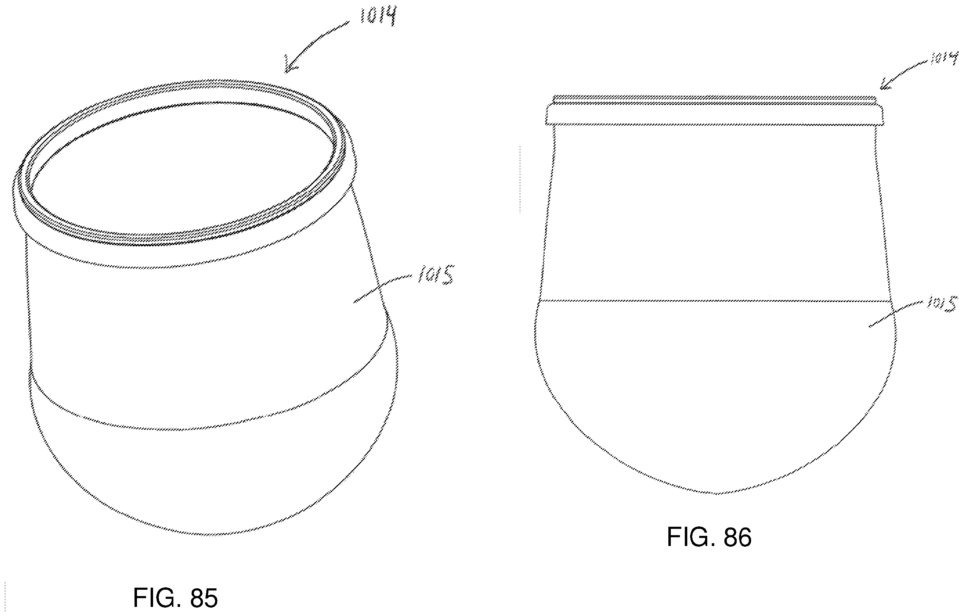

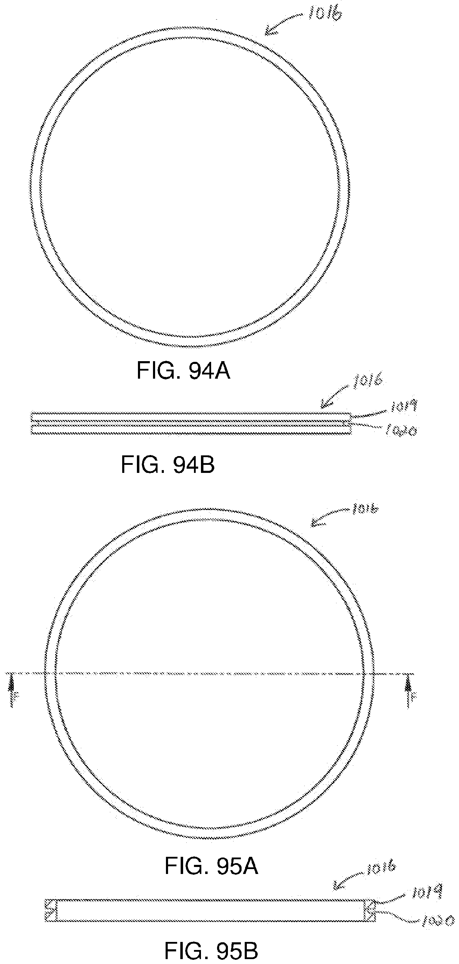

Figs. 85-98B show aspects of an exemplary bag assembly having a retractor ring 1014 coupled to a bag 1015. As with the other retractor rings and bags disclosed herein, retractor ring 1014 may, at least initially, be separate from bag 1015. Retractor ring 1014 may be coupled to a proximal portion of bag 1015 in a similar manner to the other retractor rings, at the stage of a procedure where the proximal portion of bag 1015 is to be rolled.

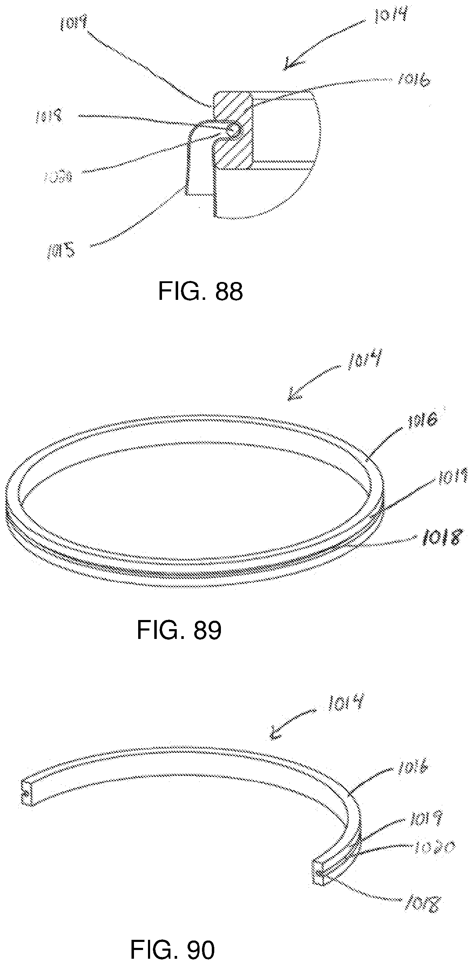

Retractor ring 1014 may have an inner ring 1016 (see, e.g., Fig. 88) and an outer ring 1018. A proximal portion of bag 1015 may be clamped between inner ring 1016 and outer ring 1018, and inner ring 1016 may rotate (e.g., evert and/or invert) around outer ring 1018 to roll the proximal portion of bag 1015 about retractor ring 1014 in one or more layers.

Inner ring 1016 may have a groove 1020 on a radially-outward facing surface 1019. Groove 1020 may receive a proximal portion of bag 1015 and outer ring 1018. Inner ring 1016 may be more flexible than outer ring 1018. Outer ring 1018 may have a discontinuity 1021 (see, e.g., Fig. 97) to facilitate mounting of outer ring 1018 onto inner ring 1016. For example, the user may be able to move ends 1017A and 1017B of outer ring 1018 apart, to help position outer ring 1018 around inner ring 1016 and within groove 1020 of inner ring 1016. Outer ring 1018 may be self-biased such that ends 1017A and 1017B may move back toward each other in the absence of a deforming force, thereby securing outer ring 1018 (and bag 1015) within groove 1020.

Figs. 85-88 show bag 1015 clamped between outer ring 1018 and inner ring 1016. In use, bag 1015 may be positioned around radially-outward facing surface 1019 of inner ring 1016, and then bag 1015 may be pressed into groove 1020 by outer ring 1018. Inner ring 1016 may rotate (e.g., invert and/or evert) about outer ring 1018. Since a proximal portion of bag 1015 may be clamped between inner ring 1016 and outer ring 1018, the proximal portion of bag 1015 may be rolled about outer ring 1018 to tighten bag 1015 around a tissue specimen in a distal portion of bag 1015. Additionally or alternatively, bag 1015 may be tightened by pulling bag 1015 proximally as far as possible relative to inner ring 1016 before clamping bag 1015 between inner ring 1016 and outer ring 1018. In another example, groove 1020 may be on a radially-inward facing surface of inner ring 1016, and outer ring 1018 may be inserted therein to clamp bag 1015 therein.

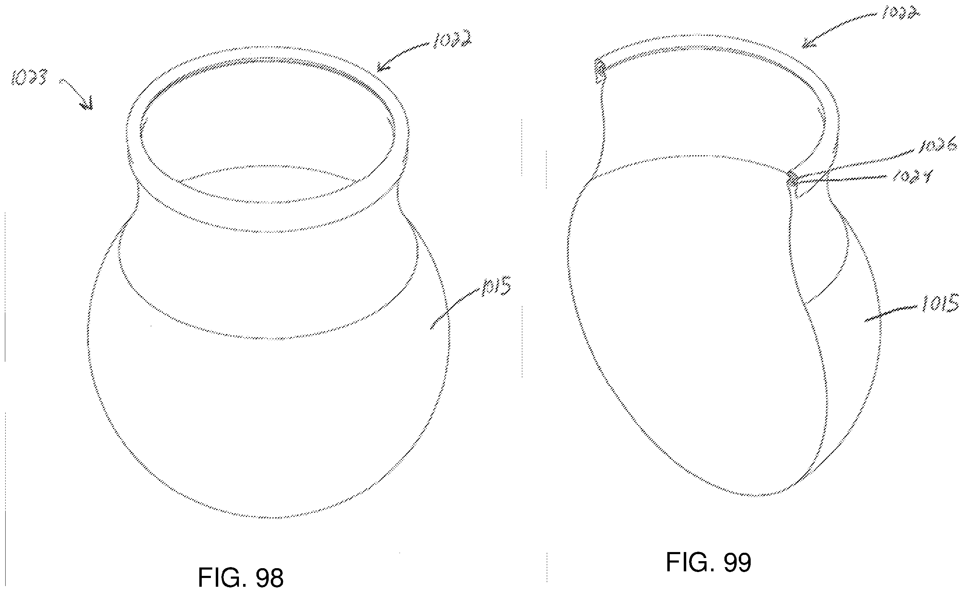

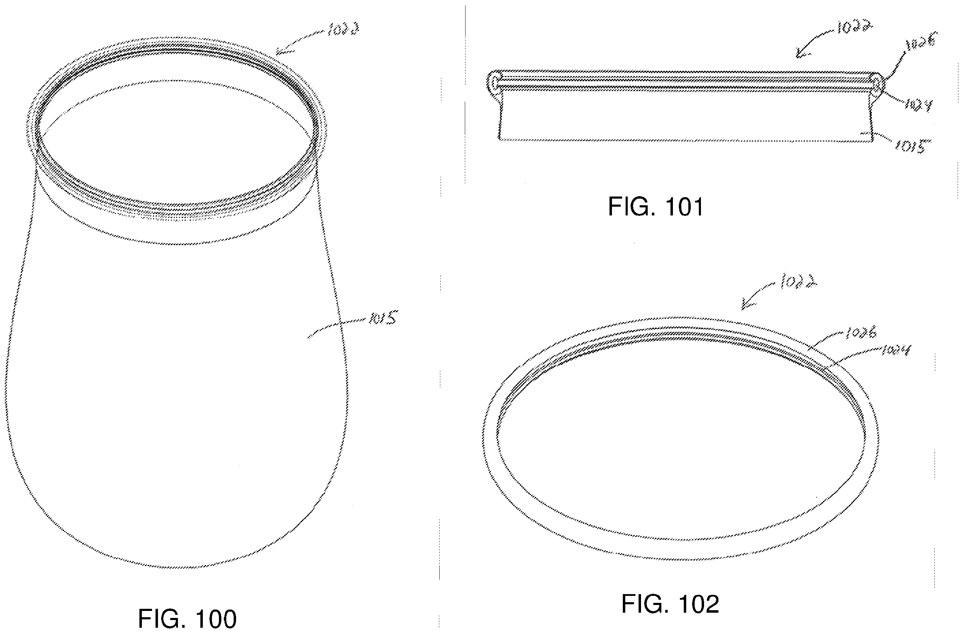

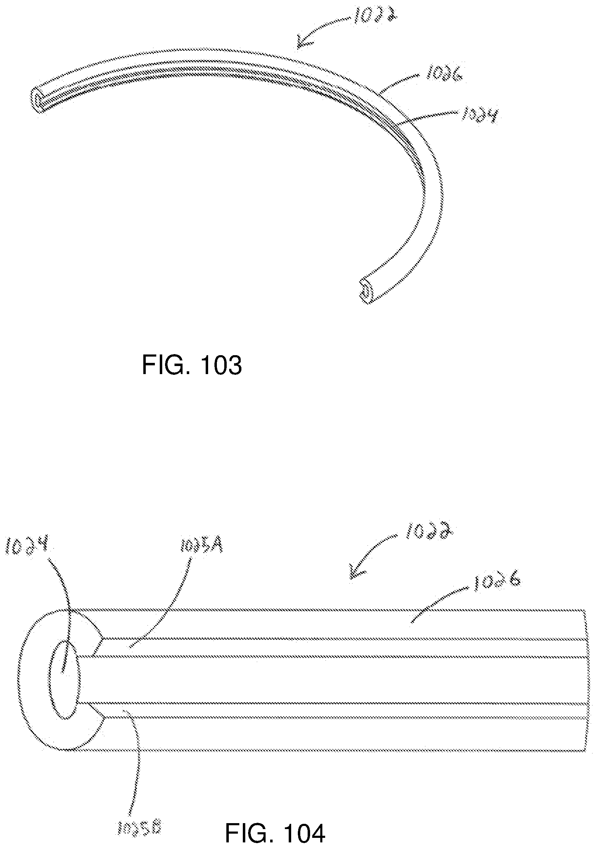

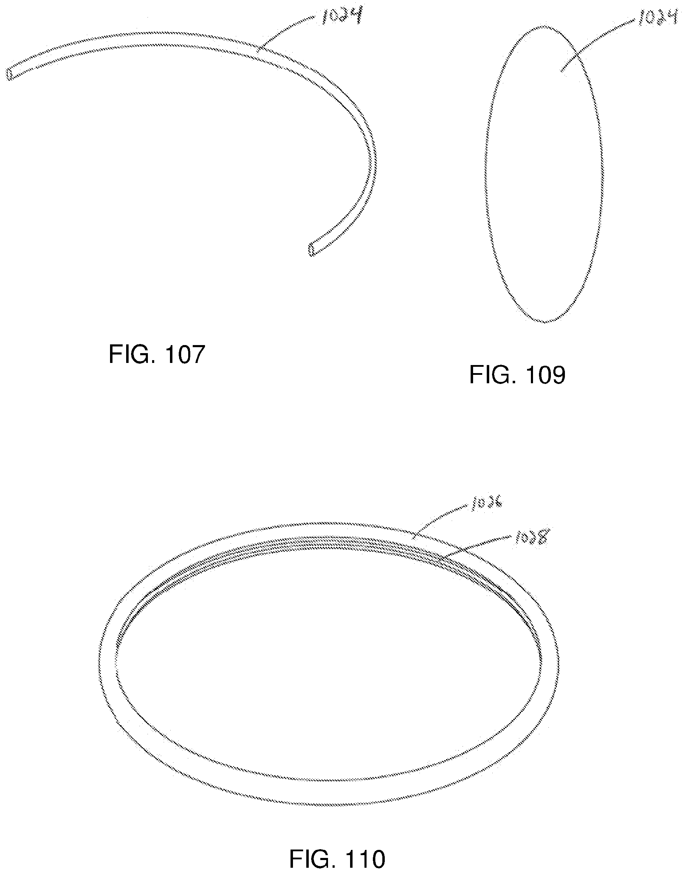

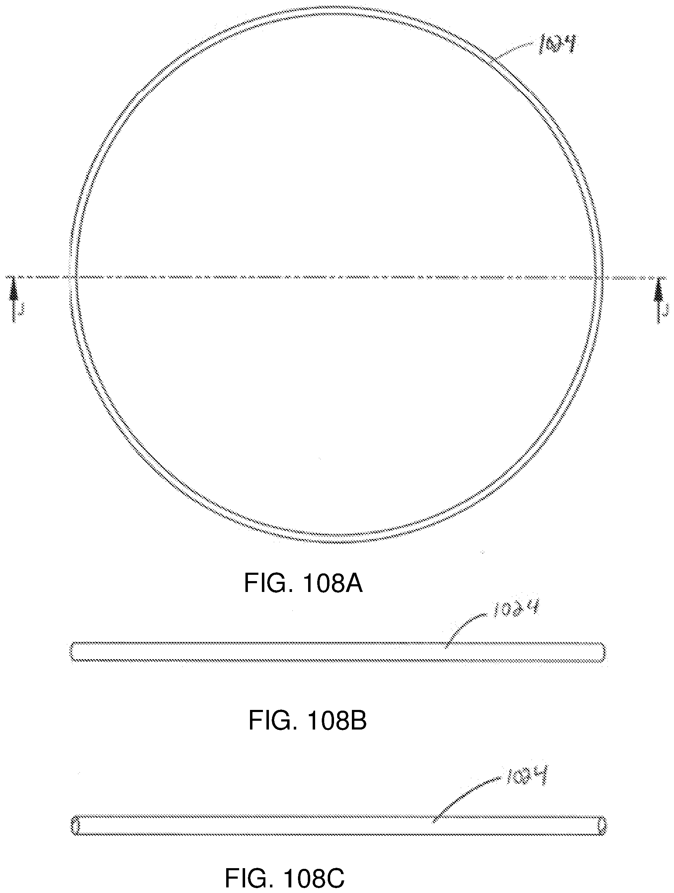

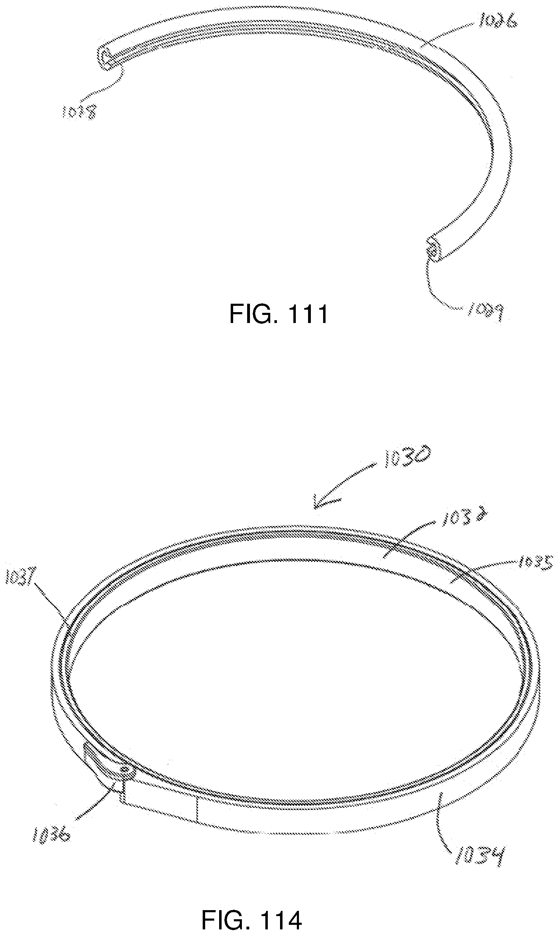

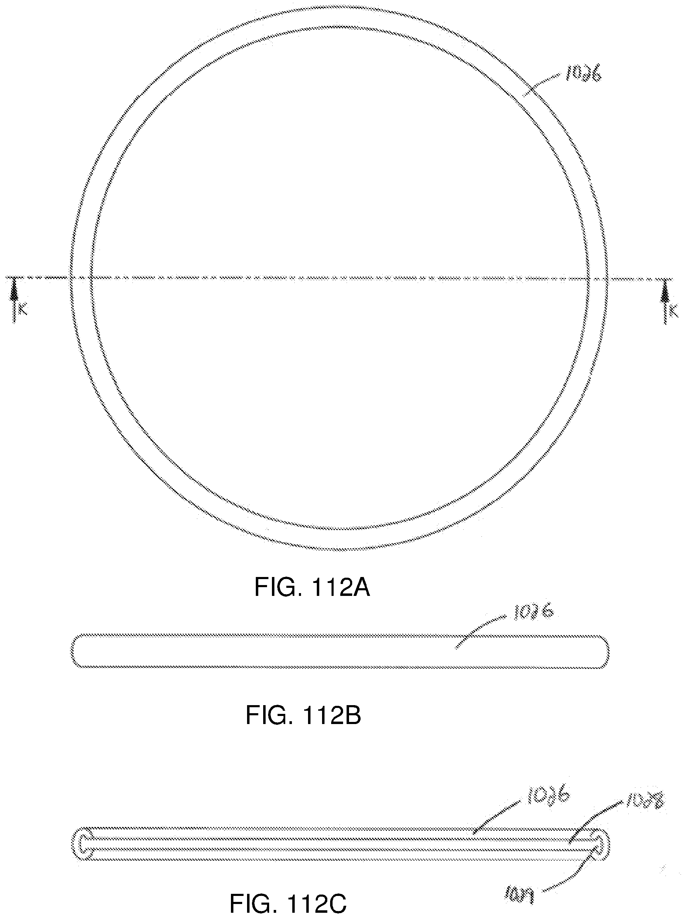

Figs. 98-112C show aspects of an exemplary bag assembly 1023 including a retractor ring 1022 and bag 1015. As with the other retractor rings and bags disclosed herein, retractor ring 1022 may, at least initially, be separate from bag 1015. Retractor ring 1022 may be coupled to a proximal portion of bag 1015 in a similar manner to the other retractor rings, at the stage of a procedure where the proximal portion of the bag is to be rolled.

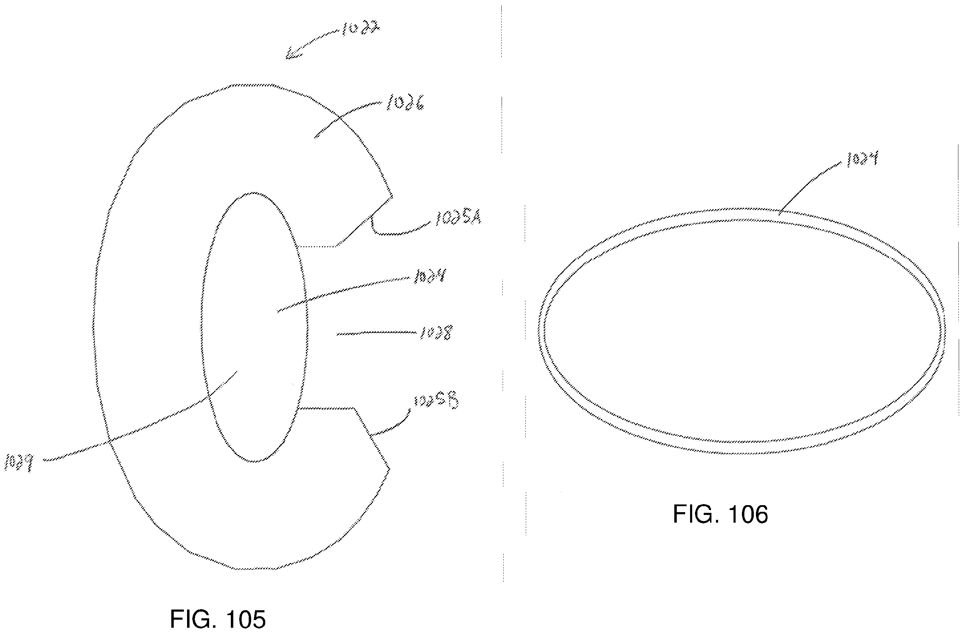

Retractor ring 1022 may include an inner ring 1024 and an outer ring 1026 (see, e.g., Fig. 99). Outer ring 1024 may snap onto inner ring 1024, or inner ring 1024 may snap into outer ring 1026, to clamp bag 1015 between outer ring 1026 and inner ring 1024. Outer ring 1026 may have an elliptical cross-sectional shape (see, e.g., Fig. 105). Outer ring 1026 may be in a stable or equilibrium state whenever a major axis of its elliptical cross-sectional shape extends substantially parallel to a central longitudinal axis of outer ring 1026. Outer ring 1026 may have a slot 1028 extending along its radially-inner surface. Slot 1028 may open into a passage 1029 extending through a center of outer ring 1026. Slot 1028 may include sloped edges 1025A and 1025B to facilitate entry of inner ring 1024 through slot 1028 and into passage 1029. Engagement between inner ring 1024 and sloped edges 1025A and 1025B may expand a width of slot 1028. Passage 1029 may be at least partially complementary to inner ring 1024. Inner ring 1024 also may have an elliptical cross-sectional shape, smaller than, but similar to, outer ring 1026.

In use, bag 1015 may be placed around inner ring 1024, and then outer ring 1026 may be snapped onto inner ring 1025 and bag 1015, to clamp bag 1015 between inner ring 1025 and outer ring 1026. Alternatively, bag 1015 may be positioned across slot 1028 of outer ring 1026, and then inner ring 1024 may be snapped into slot 1028 and passage 1029 of outer ring 1026, to clamp bag 1015 between inner and outer rings 1024 and 1026. Once retractor ring 1022 is coupled to bag 1015, bag 1015 may be rolled around retractor ring 1022 by rotating (e.g., everting and/or inverting) retractor ring 1022 as a whole, or alternatively, by rotating (e.g., everting and/or inverting) outer ring 1026 about inner ring 1024. The elliptical cross-sectional shape of inner ring 1024 and outer ring 1026 may facilitate rolling of bag 1015 about retractor ring 1022 in discrete amounts as retractor ring 1022 seeks stable or equilibrium orientations where the major axis of inner ring 1024 and/or of outer ring 1026 may be substantially parallel to the central longitudinal axis retractor ring 1022. When in an unstable or non-equilibrium state, retractor ring 1022 may be inherently biased toward the stable or equilibrium state. Both of inner and outer rings 1024 and 1026 may be flexible enough to rotate as a unit to roll bag 1015. Alternatively, inner ring 1024 may be more rigid than outer ring 1026, and outer ring 1026 may rotate about inner ring 1024.

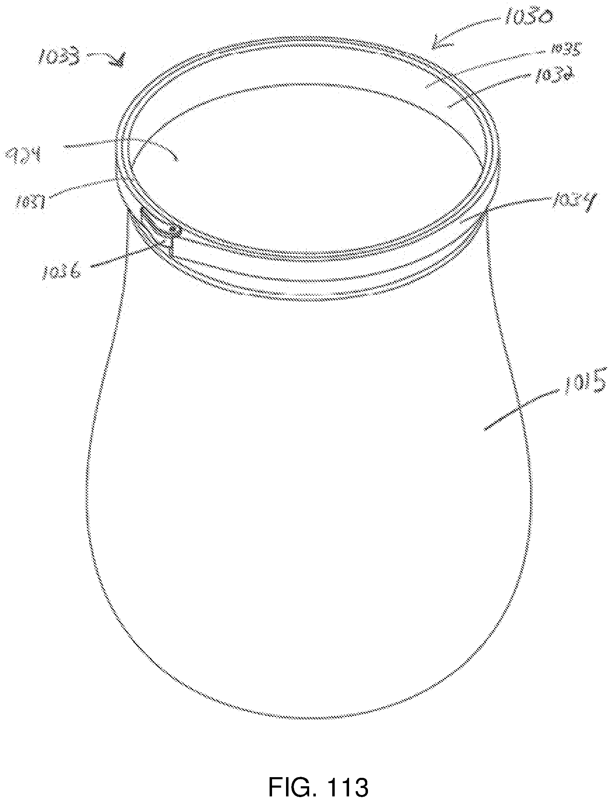

Figs. 113-115 show aspects of an exemplary bag assembly 1033 having a retractor ring 1030 and bag 1015. As with the other retractor rings and bags disclosed herein, retractor ring 1030 may, at least initially, be separate from bag 1015. Retractor ring 1030 may be coupled to a proximal portion of bag 1015 in a similar manner to the other retractor rings, at the stage of a procedure where the proximal portion of bag 1015 is to be rolled.

Retractor ring 1030 may include an inner ring 1032 and an outer ring 1034. Outer ring 1034 may engage inner ring 1032 to clamp bag 1015 between outer ring 1034 and inner ring 1032. Inner ring 1032 may have a rectangular cross-sectional shape. In a stable or equilibrium state of inner ring 1032, side faces 1035 of inner ring 1032 may extend substantially parallel to a central longitudinal axis of inner ring 1032, and end faces 1037 of inner ring 1032 may extend transverse to (e.g., substantially perpendicular to) the central longitudinal axis. A radially-outwardly facing side face (hidden behind outer ring 1034 in Figs. 113-115) of inner ring 1032 may engage bag 1015, clamping bag 1015 against a radially-inwardly facing side face (also hidden from view in Figs. 113-115) of outer ring 1034.

Outer ring 1034 may have a shape similar to inner ring 1032, and a similar stable or equilibrium state. Outer ring 1034 may include a buckle 1036 on one side that may open to expand outer ring 1034 (to facilitate insertion of bag 1015 and inner ring 1032 into outer ring 1034) and close to constrict outer ring 1034 (to force outer ring 1034 against bag 1015 and inner ring 1032). Buckle 1036 may include a hinged actuation lever for selectively expanding and contracting outer ring 1034. Buckle 1036 may include a clasp, toggle clip, toggle buckle, lever, snap, button, spring loaded clasp, ratchet action clasp, a power clamp, a screw mechanism, a push-pull clamp, C-clamp, and/or any other suitable fastening means to selectively tighten and loosen outer ring 1034 relative to inner ring 1032.

Once outer ring 1034 is clamped onto bag 1015 and inner ring 1032, retractor ring 1030 may be rotated (e.g., everted and/or inverted) about its toroidal axis to wrap bag 1015 around ring retractor 1030 in one or more layers. It is contemplated that, in one example, inner ring 1032 and outer ring 1034 may be made of the same material, and thus, may have the same degree of flexibility. Additionally or alternatively, bag 1015 may be tightened another way. For example, a proximal portion of bag 1015 may be pulled proximally through a gap between an expanded outer ring 1034 and inner ring 1032. This pulling may create tension in a distal portion of bag 1015 that houses a tissue specimen. When a desired degree of tension has been imparted, the user may constrict outer ring 1034, by closing buckle 1036, to clamp bag 1015 against inner ring 1032. This process may be repeated one or more times during a procedure to pull more of bag 1015 out of the subject's body, and then secure bag 1015, thereby increasing tension in the distal portion of bag 1015. In such a scenario, inner ring 1032 and outer ring 1034 may have different degrees of stiffness. For example, outer ring 1034 may be more flexible than inner ring 1032. It is also contemplated that this pulling/sliding approach to tightening bag 1015 may be used in combination with the aforementioned rolling approach, or in place of it.

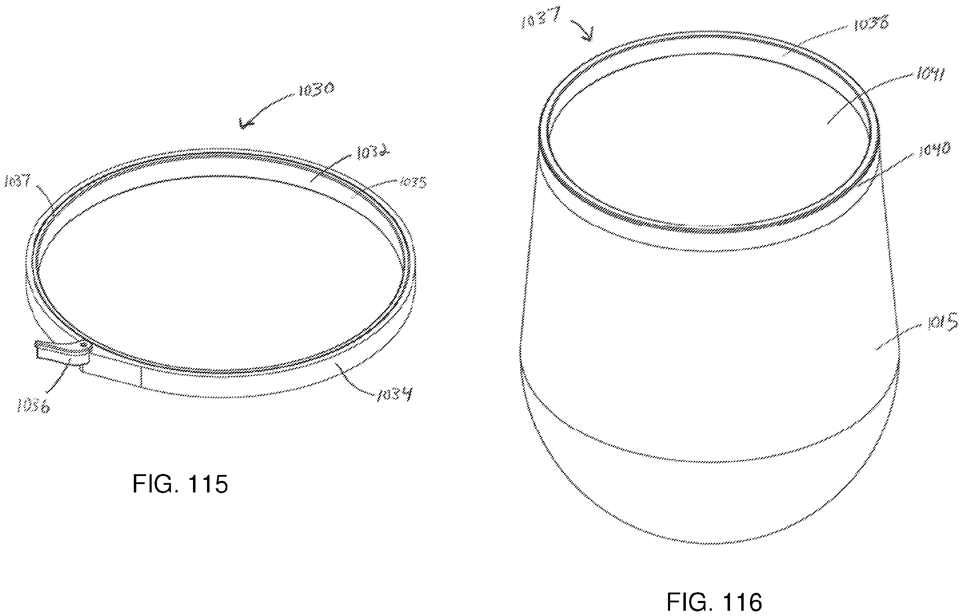

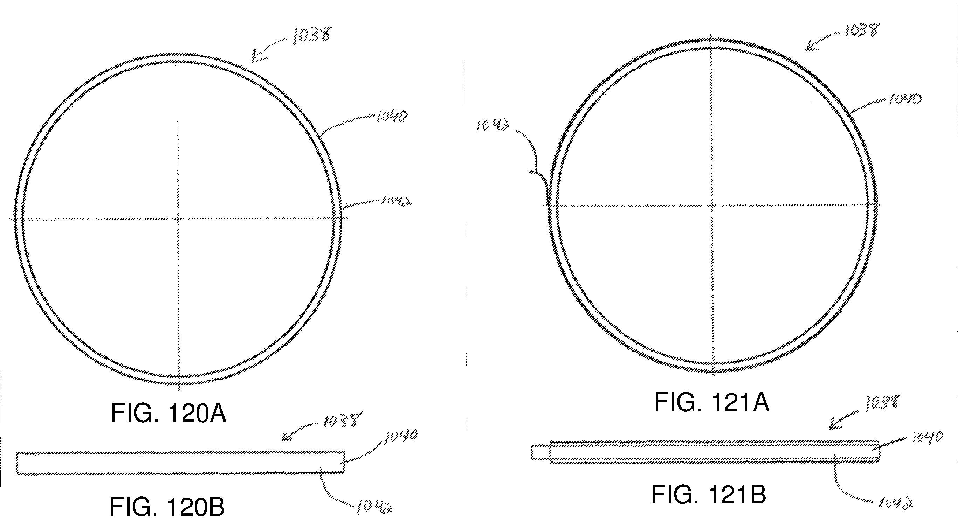

Figs. 116-121B show aspects of an exemplary bag assembly 1037 having a retractor ring 1038 and bag 1015. As with the other retractor rings and bags disclosed herein, retractor ring 1038 may, at least initially, be separate from bag 1015. Retractor ring 1038 may be coupled to a proximal portion of bag 1015 in a similar manner to the other retractor rings, at the stage of a procedure where the proximal portion of bag 1015 is to be rolled.

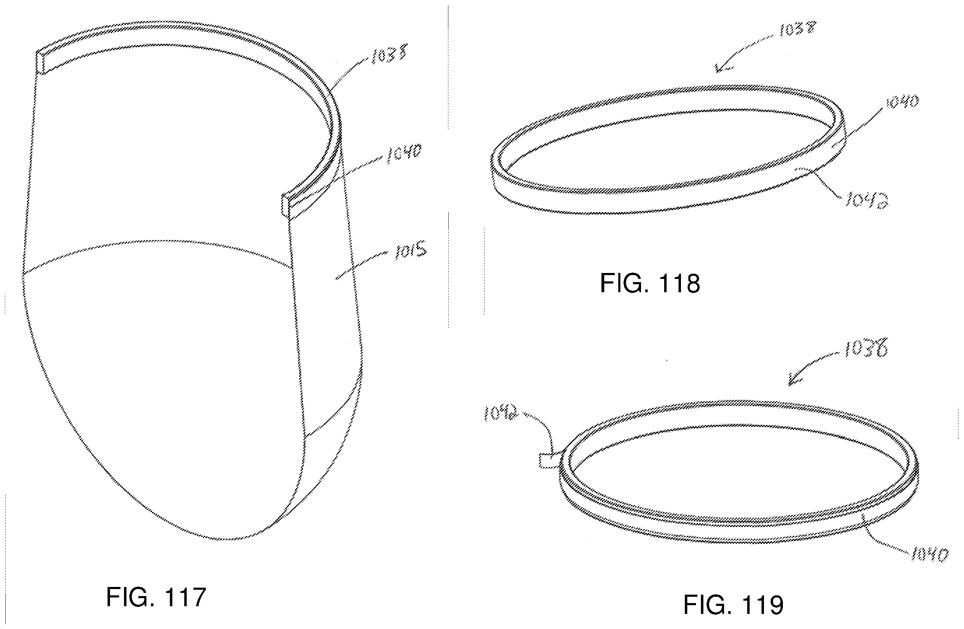

Retractor ring 1038 may be similar to inner ring 1032 (Figs. 113-115). Retractor ring 1038 may include an adhesive surface 1040 that may adhere to an interior surface 1041 of bag 1015 to couple retractor ring 1038 to bag 1015. Adhesive surface 1040 may help the user to easily and quickly couple retractor ring 1038 to bag 1015. Prior to application of retractor ring 1038 onto bag 1015, a removable strip 1042 may cover adhesive surface 1040. Removable strip 1042 may be peeled off by the user (see, e.g., Fig. 119) to expose adhesive surface 1040, so adhesive surface 1040 can be applied to any portion of interior surface 1041 of bag 1015 including, for example, a proximal portion of interior surface 1041. The adhesive may be an epoxy, polyurethane, polyimide, or other type of glue or adhesive. The adhesive may be pressure-sensitive, heat-sensitive, or may be specifically formulated in some other way to facilitate adherence to bag 1015.

Once retractor ring 1038 is coupled to bag 1015, the user may rotate (e.g., evert and/or invert) retractor ring 1038 to wrap the proximal portion of bag 1015 around retractor ring 1038 in one or more layers, thereby tightening a distal portion of bag 1015 around the tissue specimen contained therein. Retractor ring 1038 is shown in its stable or equilibrium state in Figs. 116-121B. Retractor ring 1038 may be inherently biased toward a stable or equilibrium state when in an unstable or non-equilibrium state.

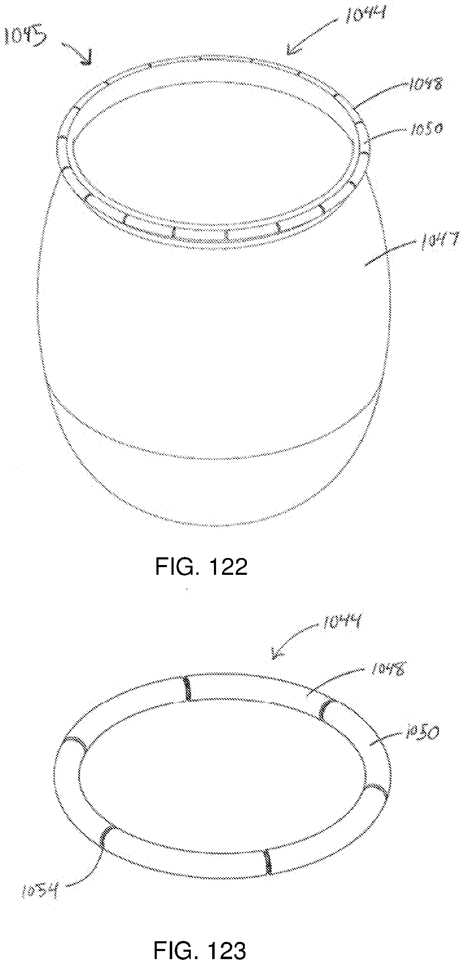

Figs. 122-127 show aspects of an exemplary bag assembly 1045 having a retractor ring 1044 and a bag 1047. As with the other retractor rings and bags disclosed herein, retractor ring 1044 may, at least initially, be separate from bag 1047. Retractor ring 1044 may be coupled to a proximal portion of bag 1047 in a similar manner to the other retractor rings, at the stage of a procedure where the proximal portion of bag 1047 is to be rolled.

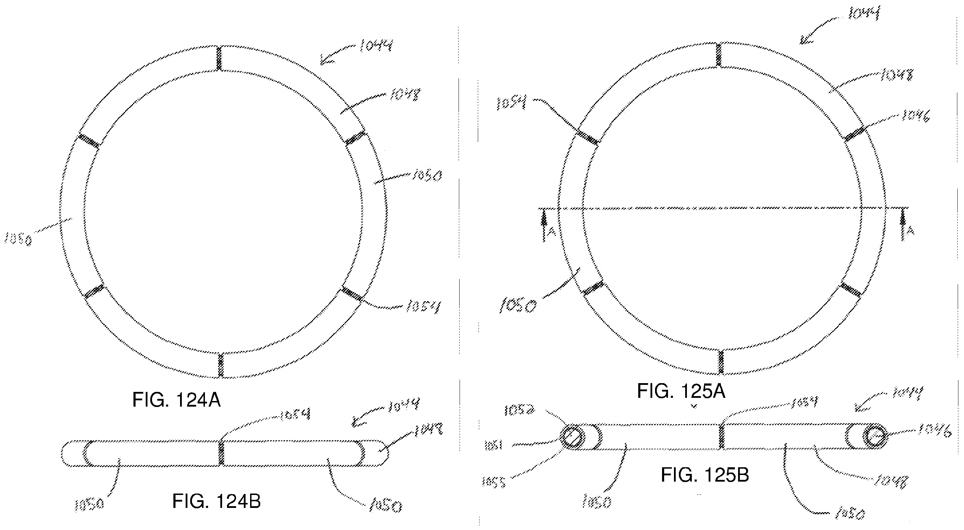

Retractor ring 1044 may include an inner ring 1046 and one or more outer ring segments 1048. Inner ring 1046 may be similar to any of the inner rings described herein. For example, inner ring 1046 may have an annular or toroidal shape, and may be more rigid than outer ring segments 1048, to facilitate rotation of outer ring segments 1048 poloidally around inner ring 1046. Inner ring 1046 may have a coating, such as a lubricant, that may facilitate the rotation of outer ring segments 1048 around inner ring 1046. Additionally or alternatively, inner ring 1046 may have a rough surface to enhance frictional engagement between inner ring 1046 and outer ring segments 1048. In one example, inner ring 1046 and outer ring segments 1048 may have splines (see, e.g., Fig. 125B) that are engaged, such that outer ring segments 1048 may lock onto inner ring 1046 in certain orientations, and/or may only rotate about inner ring 1046 in one direction.

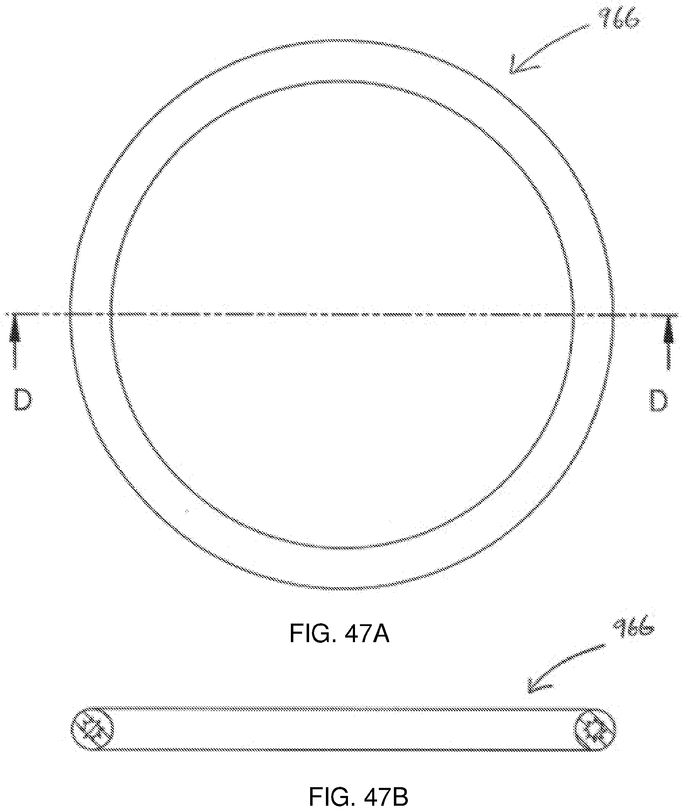

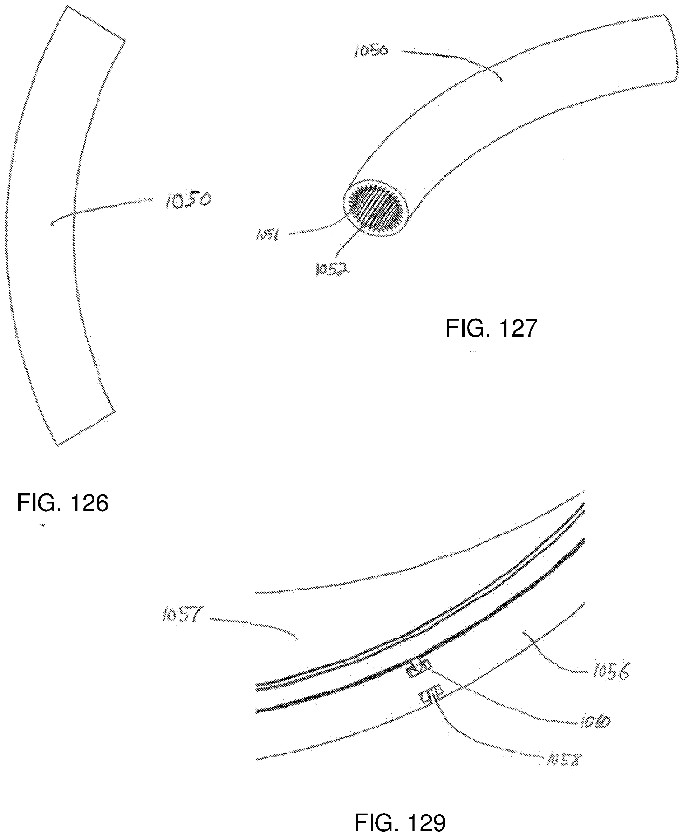

Outer ring segments 1048 may rotate independently of each other around inner ring 1046. Outer ring segments 1048 may resemble an annulus or toroid when coupled to inner ring 1046, albeit one with gaps to create discrete segments. Each of outer ring segments 1048 may have a channel 1052 (see, e.g., Fig. 127) running therethrough. The cross-sectional shape of channel 1052 may be complimentary to a cross-sectional shape of inner ring 1046. In one example, outer ring channel 1052 may be defined by splines 1051 that engage with splines 1055 (see, e.g., Fig. 125B) of inner ring 1046, similar to retractor ring 962 (see, e.g., Fig. 47, showing outer ring splines 969 engaged with inner ring splines 968).

Retractor ring 1044 may be coupled to bag 1047 in any manner discussed in this disclosure. After retractor ring 1044 is coupled to bag 1047, outer ring segments 1048 may be rotated together, or individually, to wrap bag 1047 about outer ring segments 1048. Depending, for example, on the size, shape, and/or type of tissue the user seeks to remove from the subject's body, the user may want to tighten one region of bag 1047 independently of another region of bag 1047. If more tension is desired in one region/zone/sector of bag 1048 than another, the outer ring segment that rolls that region/zone/sector may be rotated more than other outer ring segments. If less tension is desired in one region/zone/sector than another, the outer ring segment for that region/zone/sector may be rotated less than other outer ring segments. This fine adjustment of tension allows the user to, for example, exert consistent compressive force on an irregularly-shaped tissue specimen.

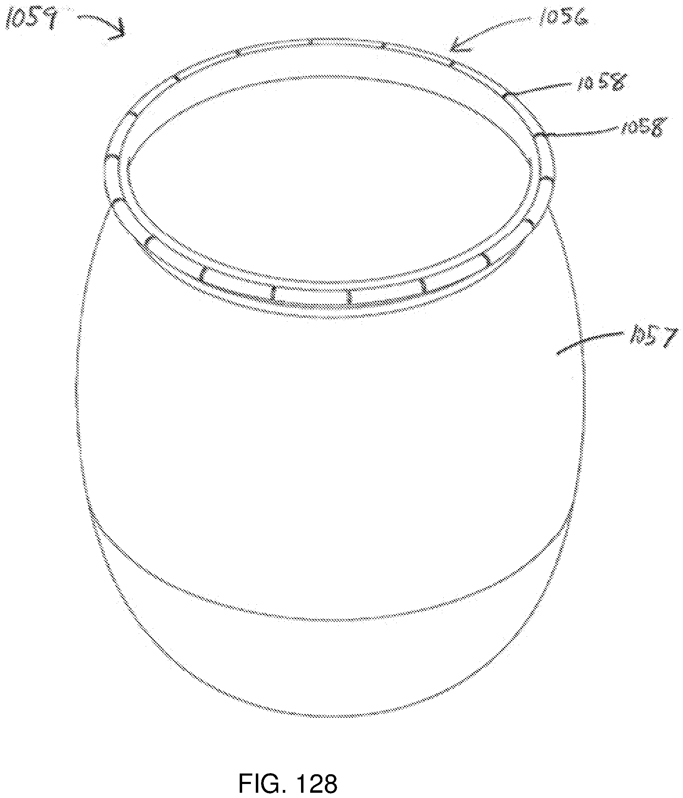

Figs. 128 and 129 show aspects of an exemplary bag assembly 1059. Bag assembly 1059 may include a bag 1057 and a retractor ring 1056. As with the other retractor rings and bags disclosed herein, retractor ring 1056 may, at least initially, be separate from bag 1057. Retractor ring 1056 may be coupled to a proximal portion of bag 1057 in a similar manner to the other retractor rings, at the stage of a procedure where the proximal portion of bag 1057 is to be rolled.

Retractor ring 1056 may have one or more annular slots or grooves 1058 along its outer surface. Grooves 1058 may run in the poloidal direction. The grooves 1058 may be evenly-spaced around the circumference of ring 1056, but other spacing arrangements also are contemplated. Grooves 1058 may be "T-track" grooves having a T-shaped cross-section (see, e.g., Fig. 129).

Grooves 1058 may receive anchors 1060 on a proximal end of bag 1057. Anchors 1060 may extend radially-outwardly from the proximal end of bag 1057. Anchors 1060 may be T-shaped, and may be complementary to portions of grooves 1058. Anchors 1060 may snap into grooves 1058, allowing retractor ring 1056 and bag 1057 to be coupled by the user during a procedure. Once in grooves 1058, anchors 1060 may slide through grooves 1058 in a poloidal direction to roll the proximal end of bag 1057 about retractor ring 1056 in one or more layers. Each anchor 1060 may slide independently of the other anchors 1060 so that different levels of tension may be imparted to different regions/zones/sectors of bag 1057.

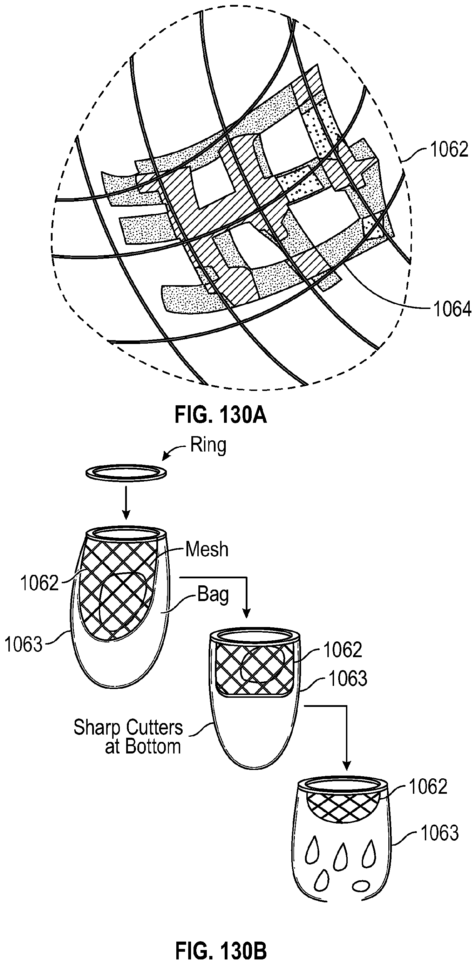

Figs. 130A and 130B show a mesh bag 1062 with cutters 1064 coupled to segments of the mesh. Mesh bag 1062 may, for example, be similar to the mesh bag(s) disclosed in U.S. Provisional Application No. 62/438,916. Cutters 1064 may be metal, plastic, or other material, and may have sharpened edges to facilitate cutting tissue. Cutters 1064 may be coupled to mesh bag 1062 by glue or other adhesive, may be fastened to mesh bag 1062 with clips, may be sewn onto mesh bag 1062, may be welded to mesh bag 1062, or may be fastened to mesh bag 1062 by other suitable means. Additionally or alternatively, cutters 1064 may include sharpened portions of segments forming the mesh of bag 1062. In one example, cutters 1064 may include blades similar to those disclosed in the '916 application. Cutters 1064 may line a bottom or distal portion of mesh bag 1062 to ensure contact with a tissue specimen inserted into mesh bag 1062. The user may actuate (e.g., expose and/or drive) cutters 1064 by compressing mesh bag 1062 against the tissue specimen. The compression may be imparted by use of any of the retractor rings described herein (Fig. 130B) to roll mesh bag 1062. Mesh bag 1062 may be contained in an outer bag 1063, such that pieces of the tissue specimen that have been cut by cutters 1064 may be contained in outer bag 1063.

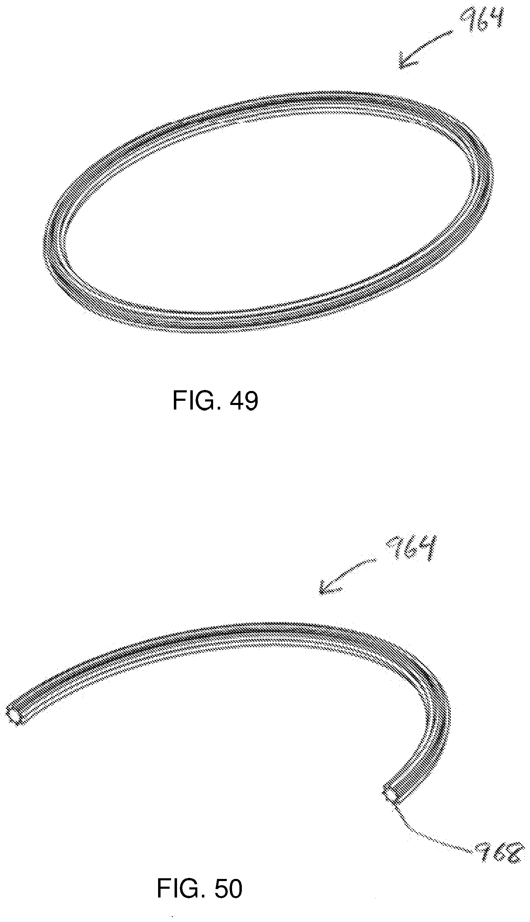

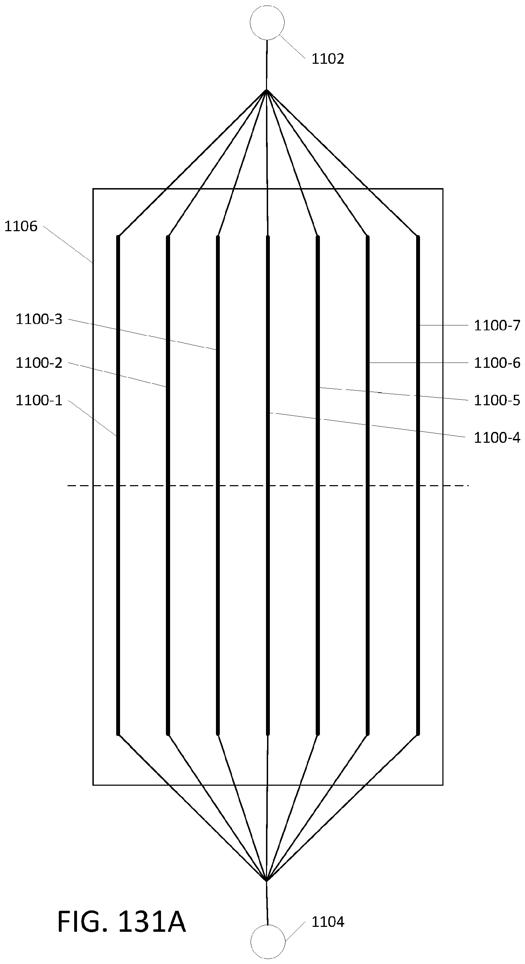

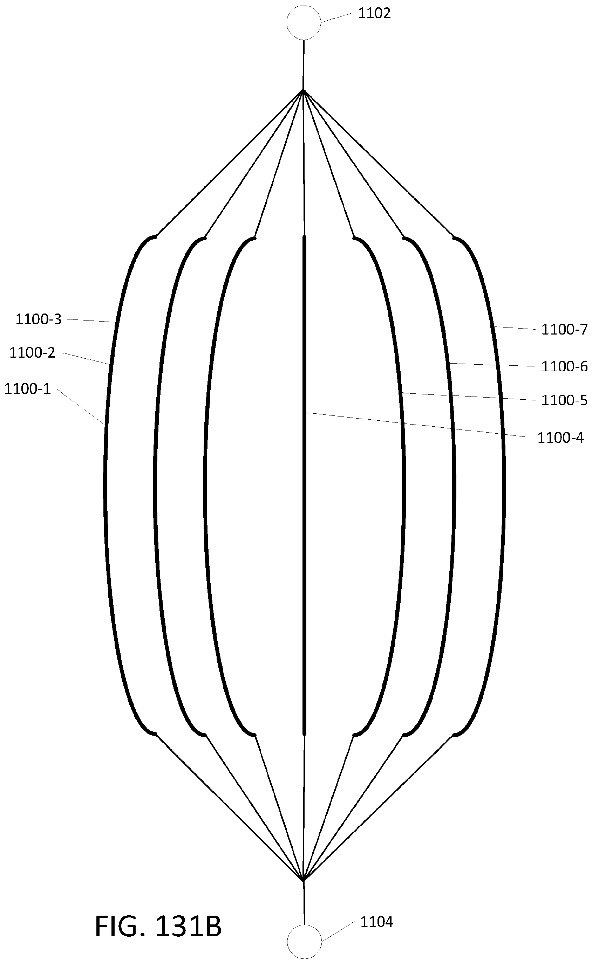

Figs. 131A and 131B show a plurality of cutting elements 1100-1 through 1100-7 (collectively referred to as 1100) having common ends 1102 and 1104. Cutting elements are disposed in an interior of bag 1106. Fig. 131A illustrates bag 1106 in an unfolded stated as indicated by the dashed line. Heat seals can be used to separate and/or keep the cutting elements substantially parallel in the bag. Cutting elements 1100 can have any of the structures described above in association with Figs. 50A-73. A handle (not shown), such as handle 306, 310, 311, or 313 shown in Figs. 47A-49, can be coupled to each of common ends 1102 and 1104. Common ends 1102 and 1104 can be pulled in a sawing motion such that multiple cutting elements (e.g., cutting elements 1100-3, 1100-4, and 1100-5) cut through a tissue specimen simultaneously. Although, Fig. 131A shows seven cutting elements and a rectangular bag 110, embodiments are not so limited. The size of the pieces into which the tissue specimen is cut can be dependent on the arrangement and/or spacing of cutting elements 1100, and/or the quantity of cutting elements 1100.

As illustrated in Fig. 131A, cutting elements 1100 can be substantially parallel to each other when coupled to bag 1106. However, when cutting elements 1100 are released from bag 1106, cutting elements 1100 expand (flare outwardly) as illustrated in Fig. 131B. Cutting elements 1100 can have a preformed shape to which cutting elements 1100 transition upon being released from bag 1106. One or more of cutting elements 1100 can be made of a shape memory alloy, such as Nitinol, and can expand outward when subjected to particular temperatures (e.g., body temperature).

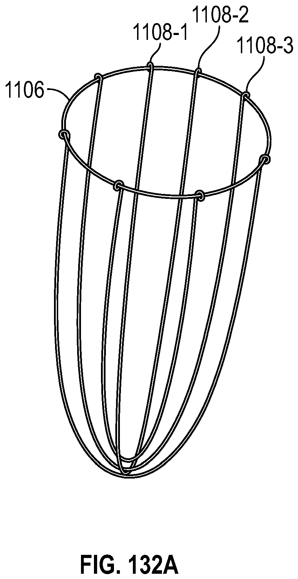

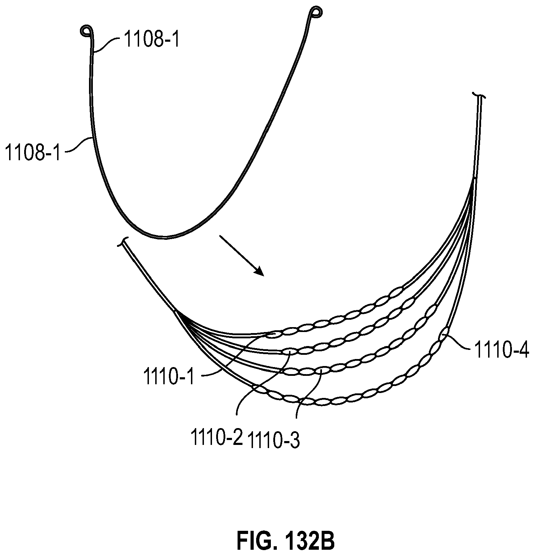

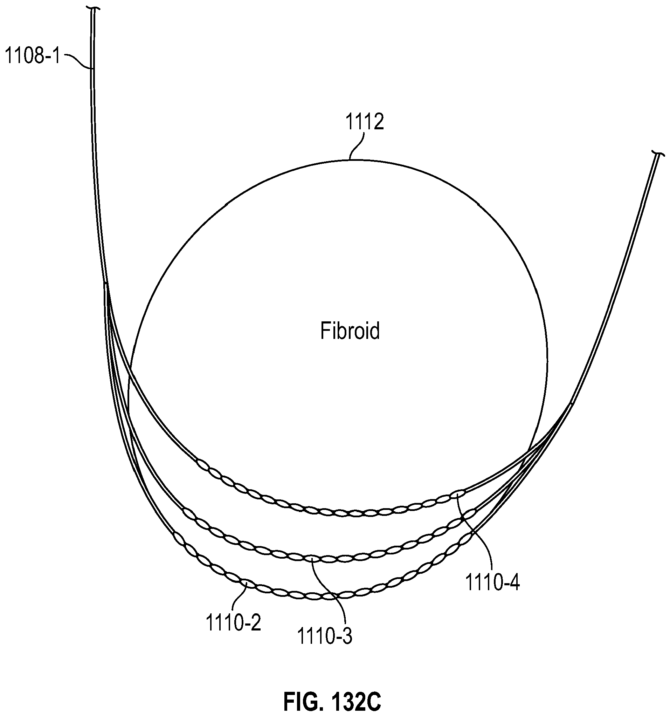

Fig. 132A shows a perspective view of a bag including three strands 1108-1, 1108-2, and 1108-3 (collectively referred to as 1108). In the example of Fig. 132A, each of strands 1108 includes a plurality of cutting elements. Fig. 132B shows a plurality of cutting elements 1110-1 through 1110-4 (collectively referred to as 1110) of strand 1108-1 in an expanded arrangement. When a strand (e.g., 1108-1) is released from bag 1106, the cutting elements 1110 of strand 1108-1 expand (flare outwardly). Cutting elements 1110 can be analogous to cutting elements 1100 illustrated in Figs. 131A and 131B. Fig. 132C illustrates cutting elements 1110-2, 1110-3, and 1110-4 of an expanded strand (e.g., strand 1108-1) in contact with tissue specimen 1112 (e.g., a fibroid). Cutting element 1110-1 is hidden by tissue specimen 1112.

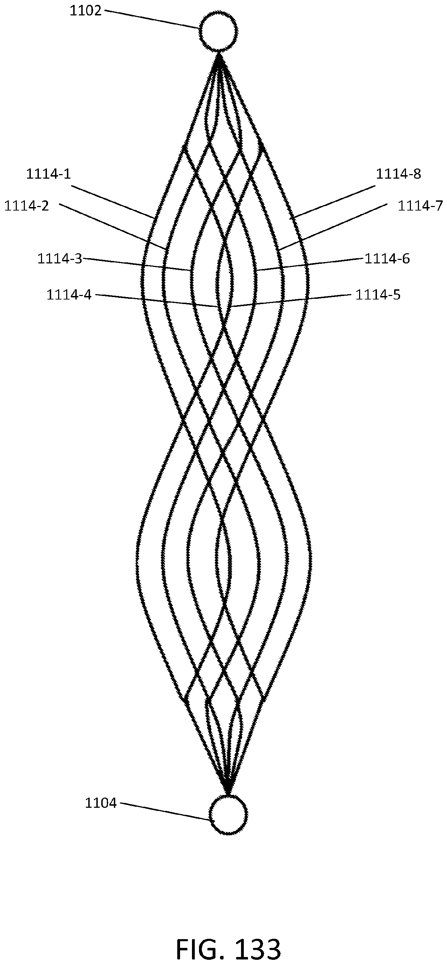

Fig. 133 shows cutting elements 1114-1 through 1114-8 (collectively referred to as 1114) having a weaved (crisscrossed) pattern. Beneficially, the weaved pattern illustrated in Fig. 133 can cut a tissue specimen into smaller pieces than, for example, the substantially parallel pattern illustrated in Fig. 131A. However, the weaved pattern may require additional force to cut through a tissue specimen because of increased resistance from the additional contact of one or more cutting elements 1114 against the tissue specimen.

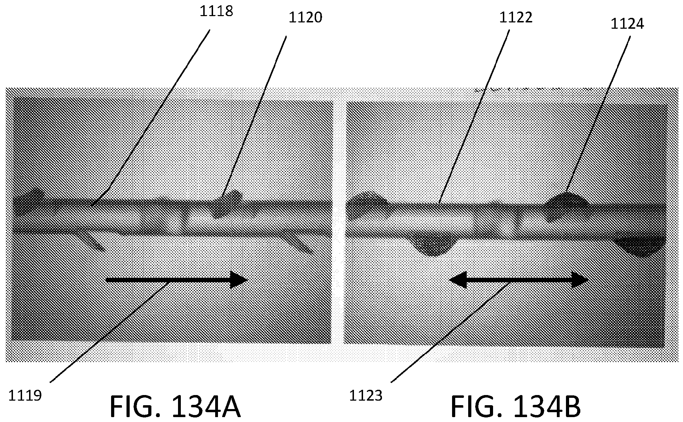

Fig. 134A shows barbed suture 1118, including barb 1120, that can serve as a cutting element (e.g., cutting elements 1100 illustrated in Figs. 131A and 131B). Fig. 134B shows bladed suture 1122, including blade 1124, that can serve as a cutting element. Barb 1120 differs from blade 1124 in that barb 1122 provides resistance in one direction along the axis of barbed suture 1118. Barbed suture 1118 resists motion in the direction indicated by the arrow 1119. In contrast, bladed suture 1122 permits motion in both directions along the axis of bladed suture 1122 as indicated by the arrow 1123.

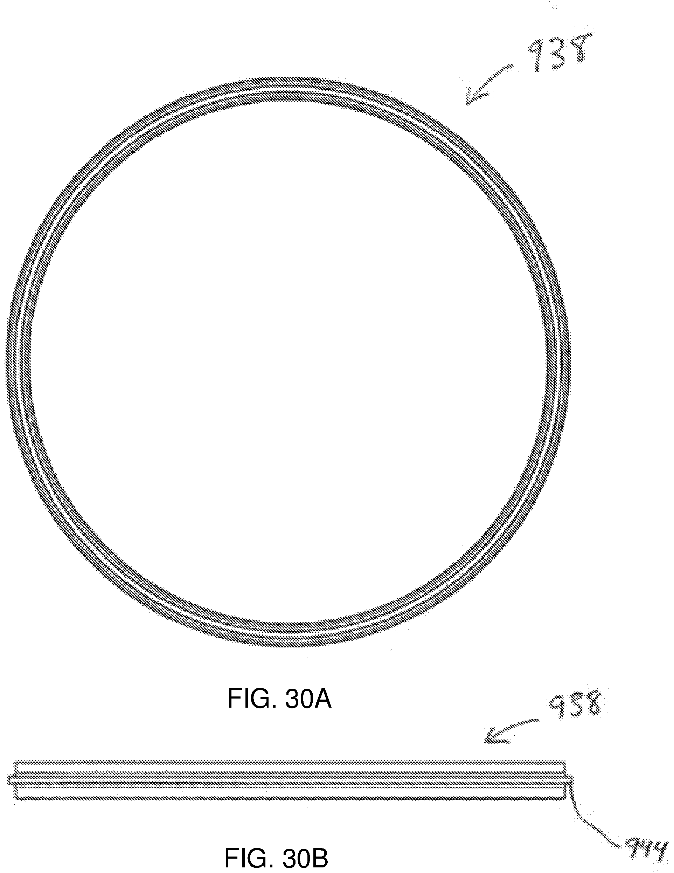

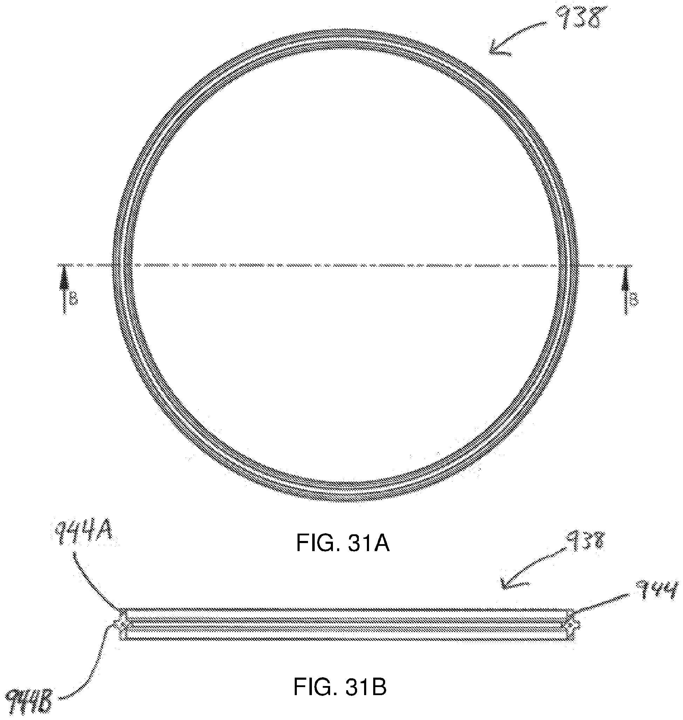

A concern with using a barbed or bladed suture as a cutting element is damaging the bag. However, at least one embodiment includes a bag including an inner layer that can be separated from an outer layer of the bag, such as double-layered bag 342 illustrated in Figs. 30A-30D. As discussed above in association with Fig. 31, gap 348 between inner layer 346 and outer layer 344 of bag 302 can be inflated to separate outer layer 344 from inner layer 346. As a result, outer layer 344 is protected from the cutting elements.

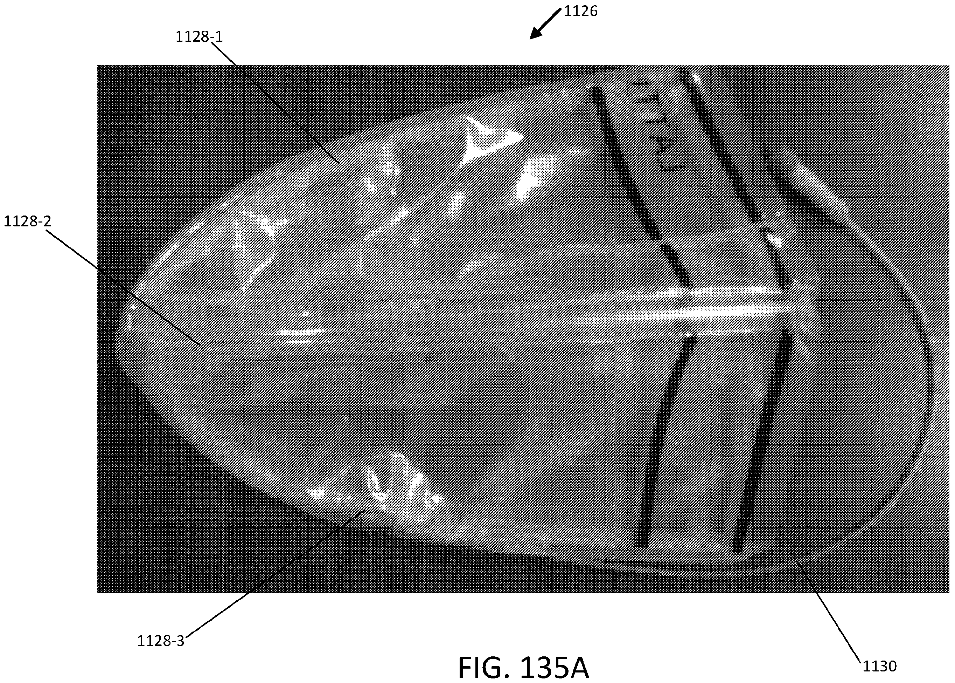

Fig. 135A shows a side view of bag 1126 including inflatable ribs 1128-1, 1128-2, and 1128-3 (collectively referred to as 1128). Bag 1126 includes a fourth inflatable rib that is hidden by inflatable rib 1128-2. Inflatable ribs 1128 prevent outer layer of bag 1126 from being damaged by cutting elements (not shown) by separating the inner layer from the outer layer of bag 1126. Inflatable ribs 1128 can support a tissue specimen (not shown) in bag 1126 while the tissue specimen is being cut. Inflatable ribs 1128 can be inflated by injecting fluid (e.g., air, saline, carbon dioxide (CO₂) into inflatable ribs 1128 via inflating connection 1130.

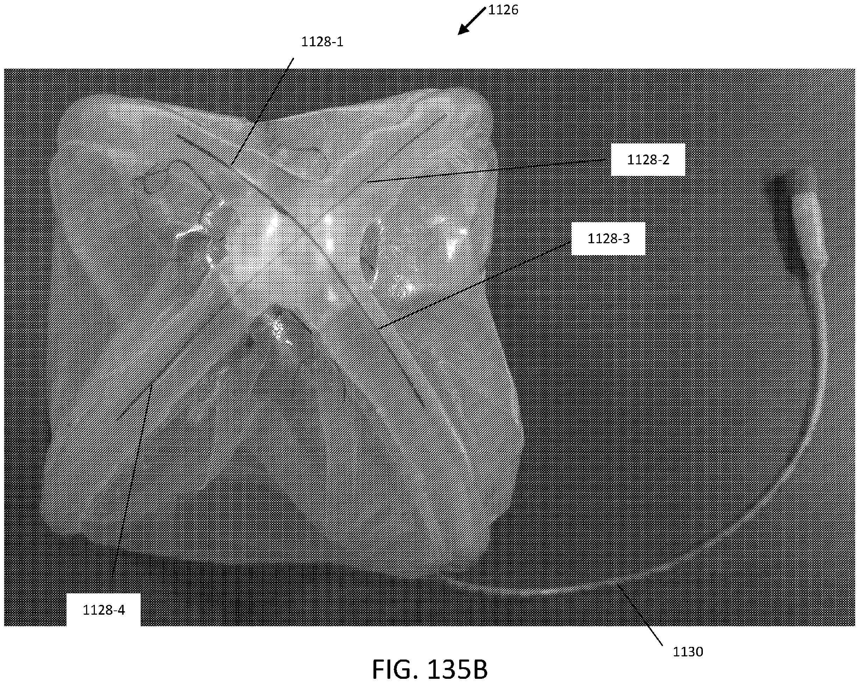

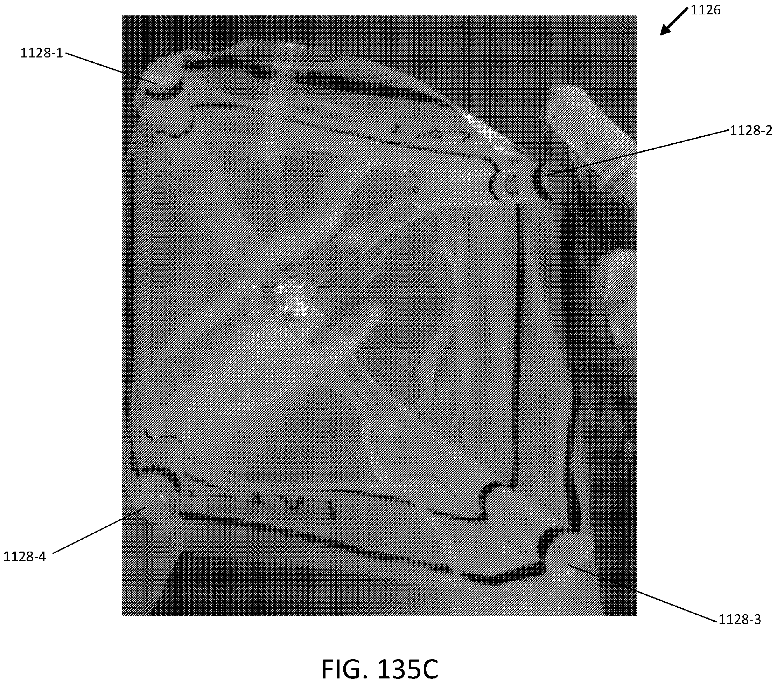

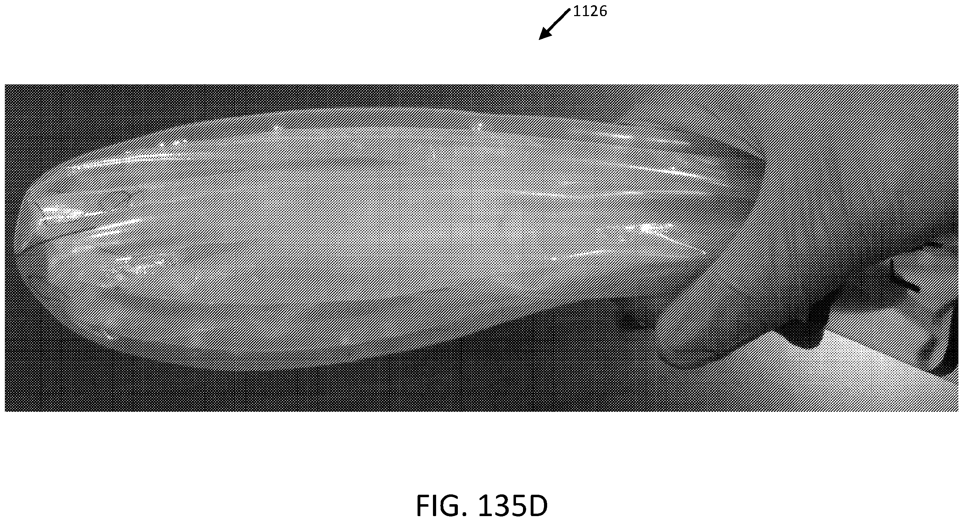

Fig. 135B shows a bottom view of bag 1126 including inflated inflatable ribs 1128-1 through 1128-4. Inflatable ribs 1128 are inflated with fluid injected to inflating connection 1130. The lines on inflatable ribs 1128 that form an "X" are for illustration purposes only. Inflatable ribs 1128 can be coupled together as shown in Fig. 135B such that inflatable ribs 1128 are inflated simultaneously. In at least one embodiment, each of inflatable ribs 1128 can be a separate chamber, each rib having its own inflating connection. Fig. 135C shows a top view of bag 1126 including inflated inflatable ribs 1128-1 through 1128-4. As can be seen in Figs. 135B and 135C, when inflatable ribs 1128 are inflated bag 1126 opens up and forms a bowl shape. Fig. 135D shows a side view of bag 1126 in a compressed state. In Fig. 135D, the inflatable ribs (not labeled) are deflated such that bag 1126 can be compressed.

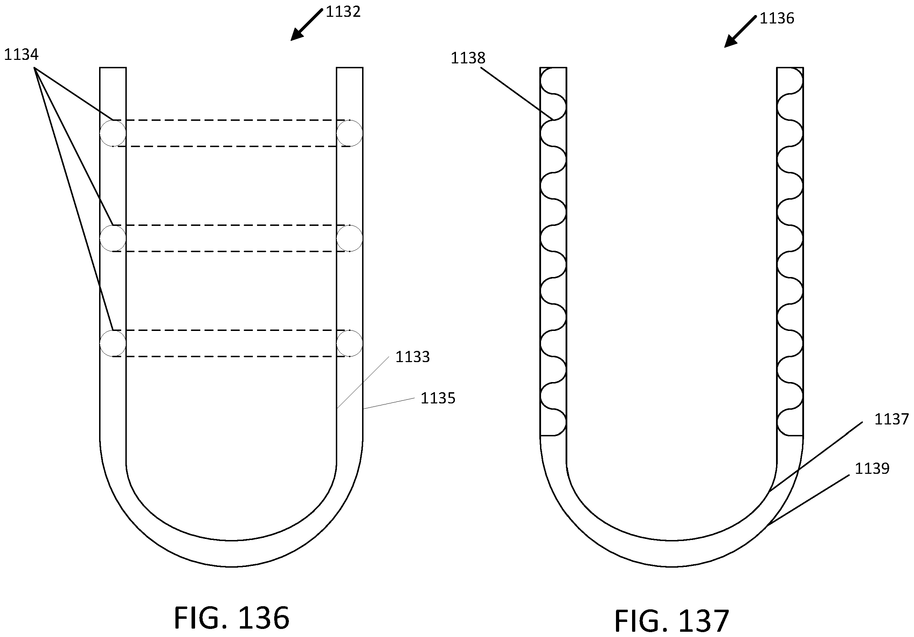

Fig. 136 shows a cross-sectional view of bag 1132 including inflatable toroids (e.g., tori) 1134. Fig. 136 illustrates inflatable toroids in an inflated state such that inner layer 1133 is separated from outer layer 1135. Although Fig. 136 shows three toroids, embodiments are not so limited. Each of toroids 1134 can be a separate chamber such that each toroid is inflated individually. Alternatively, toroids 1134 can be coupled together such that toroids 1134 are inflated simultaneously.

Fig. 137 shows a cross-sectional view of bag 1136 including inflatable corrugation 1138. Fig. 137 illustrates inflatable corrugation 1138 in an inflated state such that inner layer 1137 is separated from outer layer 1139. Although Fig. 137 shows inflatable corrugation 1138 having a semi-circular profile, embodiments are not so limited. At least a portion of inflatable corrugation 1138 can be inflated. For example, the concave portion facing outer layer 1139 can be inflated, the concave portion facing inner layer 1137 can be inflated, or both portions can be inflated. A portion of inflatable corrugation can be coupled together such that the portion can be inflated simultaneously.

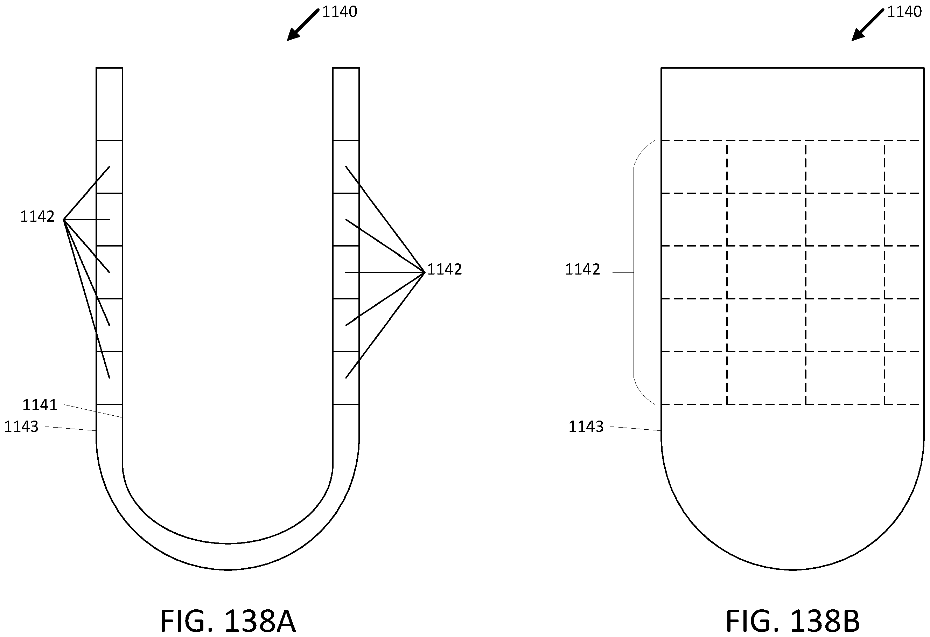

Fig. 138A shows a cross-sectional view of bag 1140 including inflatable cells 1142. Fig. 138 illustrates inflatable cells 1142 in an inflated state such that inner layer 1141 is separated from outer layer 1143. Inflatable cells 1142 can travel the circumference of bag 1140. Fig. 138B shows a side view of bag 1140 of Fig. 138B. Each of inflatable cells 1142 can be a separate chamber such that each cell is inflated individually. Alternatively, inflatable cells 1142 can be coupled together such that multiple ones of inflatable cells 1142 are inflated simultaneously. Not all of the inflatable cells 1142 have to be inflated; a subset of inflatable cells 1142 can be inflated.

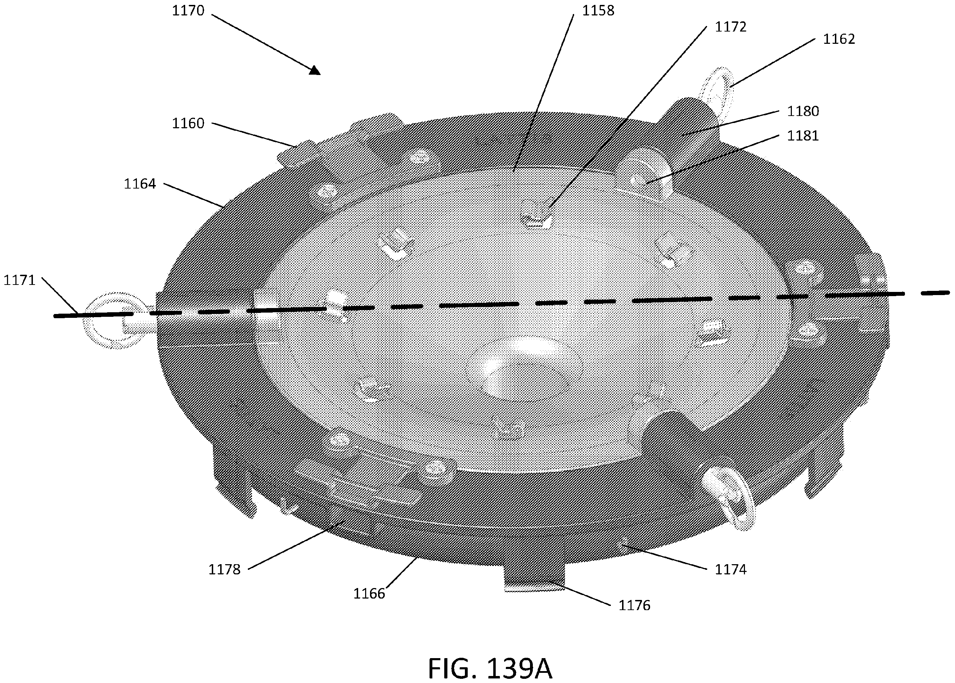

Fig. 139A shows a perspective view of wound retractor 1170. Wound retractor 1170 includes inner ring 1158 and outer ring 1164. Inner ring 1158 fits within the annulus of outer ring 1164. Outer ring 1164 and inner ring 1158 each include at least one aperture, apertures 1180 and 1181, respectively, through which coupler 1162 can pass to couple inner ring 1158 to outer ring 1164. Inner ring 1158 can be removed and/or separated from outer ring 1164 by removing coupler 1162 from at least aperture 1181. In at least one embodiment coupler 1162 can be a spring plunger. As illustrated in Fig. 139A, inner ring 1158 has a funnel shape. Inner ring 1158 includes at least one retainer 1172 that can secure a strand or a cutting element. Retainers 1172 can each be a compliant mechanism made from the same material as inner ring 1158. A method of use of wound retractor 1170 is discussed below.

Wound retractor 1170 includes bag rolling ring 1166. Bag rolling ring 1166 is removably coupled to outer ring 1164 via at least one fastener 1176. Bag rolling ring 1166 includes at least one hook 1174 to which any of the bags disclosed herein can be coupled. Although Fig. 139A shows bag rolling ring 1166 including hooks 1174, embodiments are not so limited. For example, bag rolling ring 1166 can include pins, posts, and/or clips to couple a bag to bag rolling ring 1166. Bag rolling ring 1166 is used to roll the bag around bag rolling ring 1166 as described below. Bag rolling ring 1166 is made from a material with a firmness sufficient to prevent buckling during rolling the bag and to maintain optimal tension on a tissue specimen inside the bag.

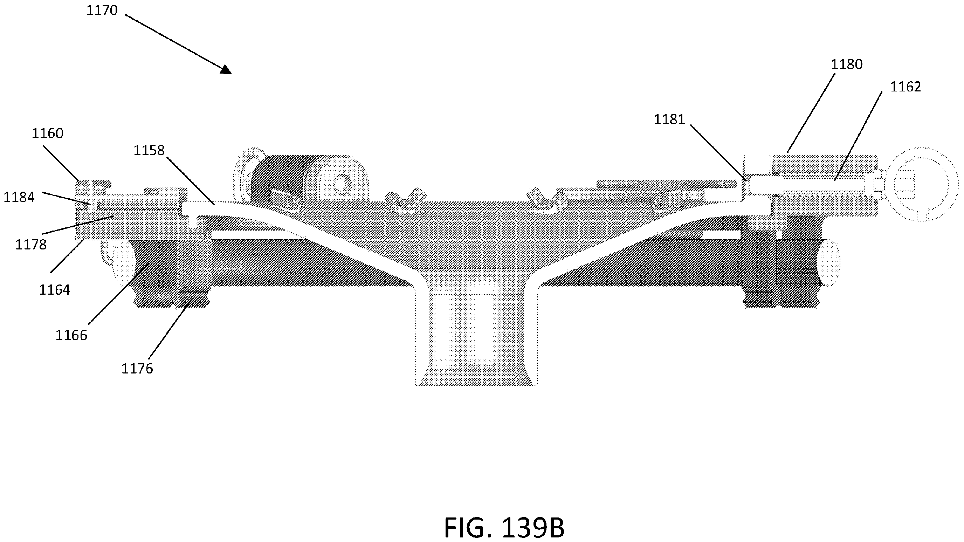

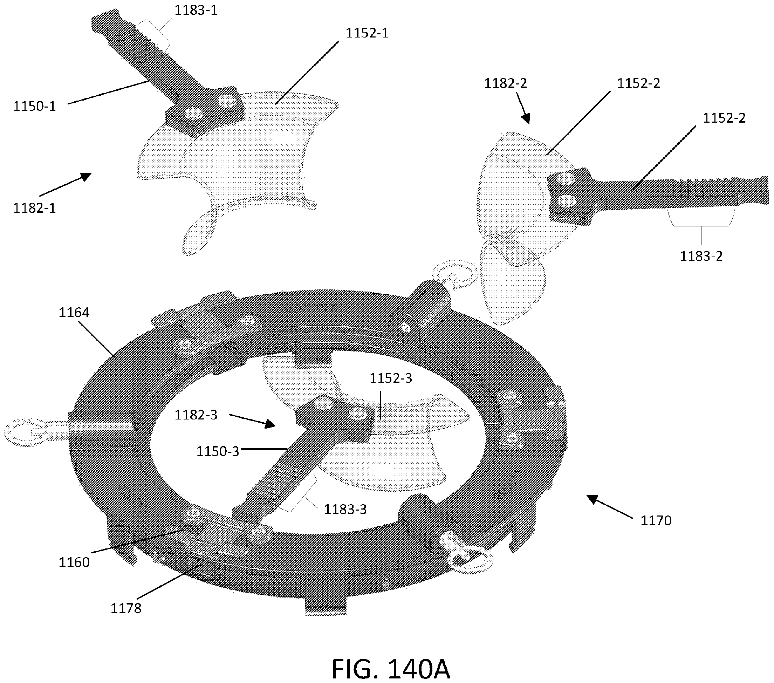

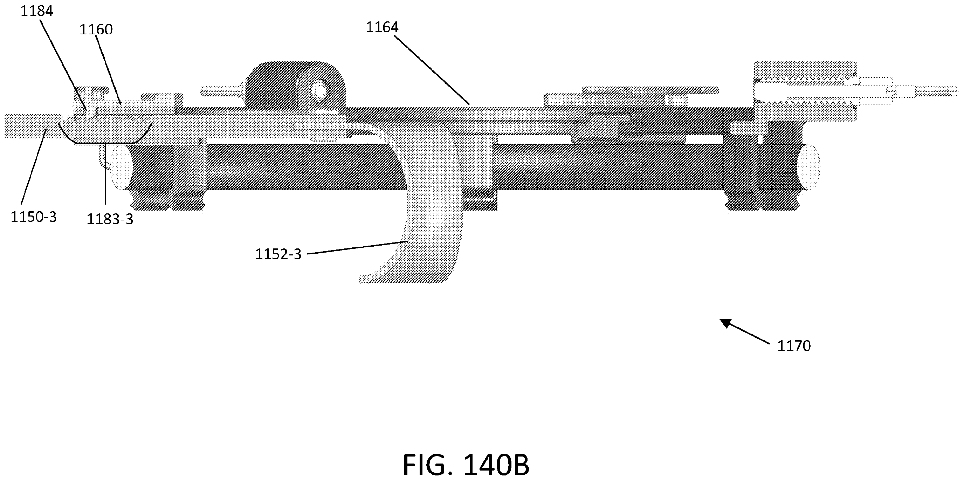

Fig. 139B shows a sectional view of wound retractor 1170 of Fig. 139A. The section is taken at line 1171 illustrated in Fig. 139A. As shown in Fig. 139B, outer ring 1164 includes at least one retractor detent 1160. Retractor detent 1160 includes pawl (click) 1184 of a ratchet. The rack of the ratchet is on retractor arm 1150 discussed in association with Figs. 140A-140D below. Retractor detent 1160 is coupled to outer ring 1164 such that pawl 1184 is positioned in channel 1178 of outer ring 1164. When retractor arm 1150 is position within channel 1178 (see Fig. 140B), pawl 1184 interfaces with rack 1183 on the retractor arm 1150.

Inner ring 1158 is coupled to outer ring 1164 via coupler 1162 positioned in apertures 1180 and 1181. Bag rolling ring 1166 is coupled to outer ring 1164 via fastener 1176. Fasteners 1176 can each be a compliant mechanism made from the same material as outer ring 1164. As discussed below, bag rolling ring 1166 should be able to rotate within fastener 1176.

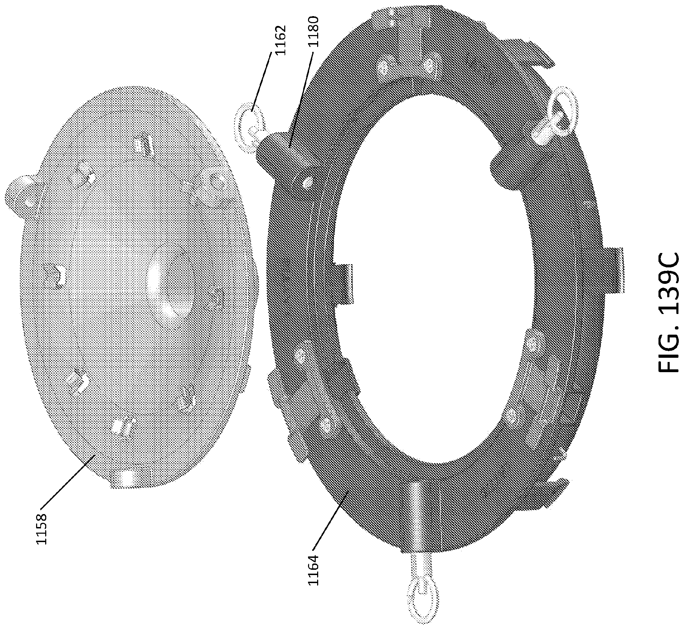

Fig. 139C shows a perspective view of wound retractor 1170 with inner ring 1158 removed from outer ring 1164. Couplers 1162 are positioned in only aperture 1180 such that inner ring 1158 can be removed from outer ring 1164.

Fig. 140A shows a perspective view of outer ring 1164 of wound retractor 1170 and a plurality of retractors 1182-1, 1182-2, and 1182-3 (collectively referred to as 1182). Each of the retractors 1182 include retractor arm 1150 and retractor blade 1152. Retractor blade 1152 can be analogous or similar to petal 672 illustrated in Fig. 34R. Fig. 140A illustrates retractor 1182-3 positioned within the annulus of outer ring 1164 to be inserted in channel 1178.

Fig. 140B shows a sectional view of outer ring 1164 of wound retractor 1170 with retractor arm 1150 positioned within channel 1178. Pawl 1184 of retractor detent 1160 interfaces with rack 1183-3, permitting retractor 1182-3 to be pulled such that retractor blade 1152-3 is moved incrementally closer to outer ring 1164 while providing resistance to motion in the opposite direction.

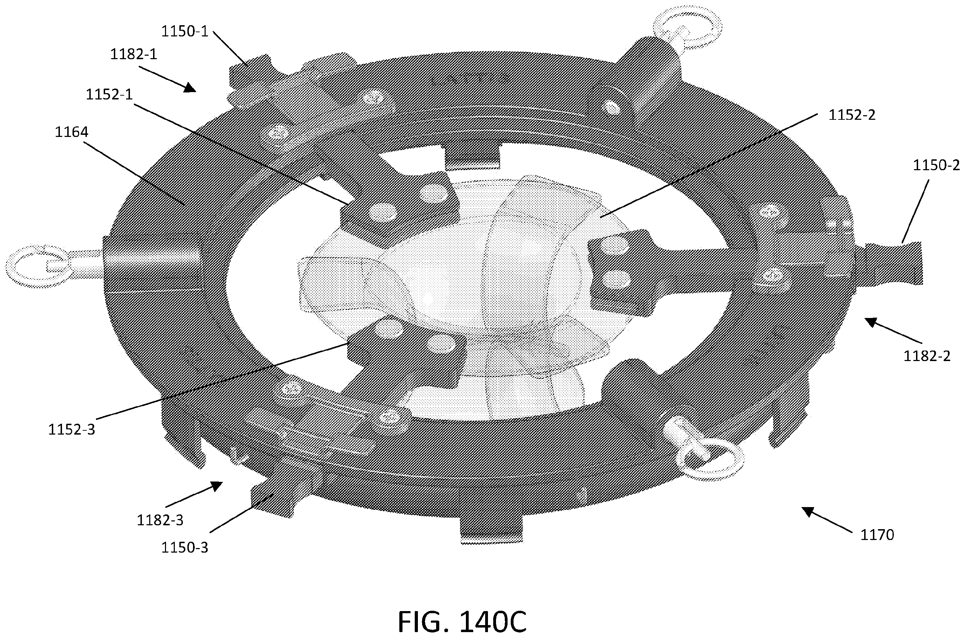

Fig. 140C shows a perspective view of outer ring 1164 of wound retractor 1170 with retractors 1182 positioned within channels 1178 of outer ring 1164. As shown in Fig. 140C, edges of retractor blades 1152-1, 1152-2, and 1152-3 of retractors 1182-1, 1182-2, and 1182-3, respectively, overlap one another to cooperatively form an annulus.

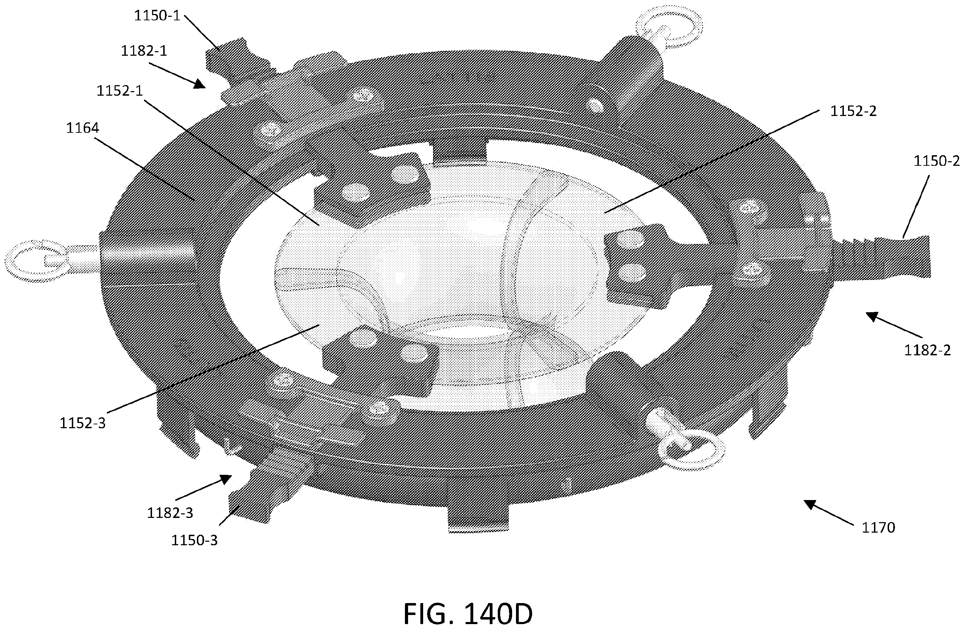

Fig. 140D shows a perspective view of outer ring 1164 of wound retractor 1170 with retractors 1182 positioned within channels 1178 of outer ring 1164. As shown in Fig. 140D, retractor blades 1152 have been positioned closer to outer ring 1164 than shown in Fig. 140C. Edges of retractor blades 1152 still overlap one another to cooperatively form the annulus; however, the diameter of the annulus is greater than that shown in Fig. 140C.

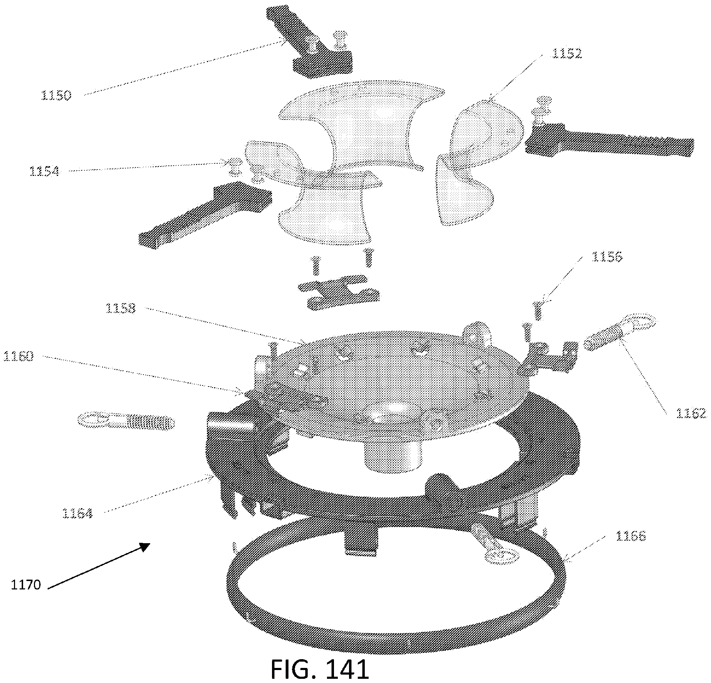

Fig. 141 shows an exploded view of wound retractor 1170. Retractor detents 1160 can be coupled to outer ring 1164 via screws 1156. Each of the retractor blades 1152 can be coupled to each of the retractor arms 1150 via rivets 1154.

The following is an example method of use of wound retractor 1170 shown in Figs. 139A-141. An incision (e.g., 2.5 centimeters (cm) long) is created in a patient's skin with a knife (e.g., scalpel). A cannula, for example, one having a 2.8 cm diameter, is inserted through the incision using a metal and/or plastic trocar (e.g., a taper point or cutting/trocar point). The inner diameter of the cannula should be at least the length of the incision (e.g., 2.5 cm). The trocar is then removed, but the cannula is left in place.

A bag, such as bag 1126, 1132, or 1136 shown in Figs. 135A-137, contains a plurality of molded, flexible tracks/guides (e.g., four tracks) for a plurality of cutting elements, such as cutting elements 1100 illustrated in Figs. 131A and 131B, and/or a plurality of strands, such as strands 1108 illustrated in Figs. 132A-132C. In at least one embodiment, each of the cutting elements include a length of chainsaw having five links. Each cutting element or strand has a color-coded and/or numbered handle coupled to each end of the cutting elements or strands. For example, if there are four cutting elements then there are eight color-coded and numbered handles.

The upper portions of the cutting elements or strands are held in place against the sides of the bag (e.g., with plastic tabs) so that they do not become tangled when the bag is rolled or compressed. The plastic tabs can be perforated so as to facilitate freeing the cutting elements or strands from the bag. The handles can be staggered in height so as to facilitate passing the bag through the cannula.

The bag is inserted (pushed) into the patient's body (e.g., the patient's abdomen) through the cannula using an inserter with the bag rolled up (like an umbrella) inside the inserter. The cannula can include a seal that can be used as visual port, for example, for a camera. A camera can facilitate inserting a tissue specimen into the bag. The insertion of the bag can be analogous to the process described in association with Figs. 46H-46L. Once the bag is inserted into the patient, the bag lies free. The tissue specimen is then inserted into the bag.

After inserting the tissue specimen in the bag, the neck of the bag is grasped (e.g., with a grasper) and pulled up through the incision. The cannula is then removed. Small tags can be fastened to the edge of the bag to facilitate this process. The handles and cutting elements and/or strands are pulled away/detached from the sides of the bag. The bag is attached to bag rolling ring 1166 via hooks 1174 and rolled taut. Rolling the bag with bag rolling ring 1166 may require four hands. To facilitate rolling the bag can be brought up taut against the underside of the incision (e.g., against the abdominal wall). Once the bag is rolled taut such that the tissue specimen is held against the underside of the incision, the cutting elements and/or strands are brought up through the annulus of bag rolling ring 1166. Outer ring 1164, including inner ring 1158, is coupled to (snapped onto) bag rolling ring 1166. The cutting elements and/or strands are attached to inner ring 1158 via retainers 1172 located at even intervals around the periphery of the outer portion of inner ring 1158.

If the bag includes an outer layer and an inner layer, such as bag 1126, inflatable ribs 1128 are inflated. For example, inflatable ribs 1128 can be inflated with CO₂ by connecting CO₂ tubing to inflating connection 1130, thereby expanding the outer layer and moving it away from the inner layer and the cutting elements and/or strands.

The cutting elements and/or strands are individually removed from fasteners 1172, one at a time, and pulled back and forth in a sawing motion. The cutting elements and/or strands quickly reduce the tissue specimen to smaller, manageable sized pieces. When one of the cutting elements and/or strands cuts all the way through the tissue specimen, that cutting element or strand is removed. The cutting elements and/or strands are utilized and removed sequentially according the numbers assigned to each of the handles. After all of the cutting elements and/or strands have cut through the tissue specimen and have been removed from inner ring 1158, inner ring 1158 is separated and/or removed from outer ring 1164.

Outer ring 1164 includes three retractor detents 1160, each located at the ten o'clock, two o'clock, and six o'clock positions. Retractor arms 1150 of three retractors 1182 are then positioned into channels 1178 of outer ring 1164. Retractors 1182 include retractor blades 1152 coupled to retractor arm 1150. Retractor arm 1150 is a knurled shaft including rack 1183 that interfaces with pawl 1184 of retractor detent 1160 to form a ratchet. The ratchet allows retractor arm 1150 to be tightened (pulled away from the center of the incision) and held in place. Retractor blades 1152 can each be a curved piece of semi-rigid, semi-flexible plastic. Retractor blades 1152 are inserted into the incision. As illustrated in Figs. 140C and 140D, each one of the three retractor blades 1152 overlaps with the adjacent two retractor blades 1152, so as to form a collar that completely lines and protects the inside of the incision.

The cut pieces of the tissue specimen are extracted through the collar of retractor blades 1152 that protect the neck of the bag. If a cut piece of the tissue specimen is too large to fit through the collar, the cut piece can be cut again using a knife (e.g., scalpel). After the cut pieces have been extracted from the bag, the bag is removed along with outer ring 1164 and retractor arms 1182. If the bag is a dual layer bag, the space between the inner and outer layers and/or inflatable ribs 1128 are deflated and removed along with the inner layer.

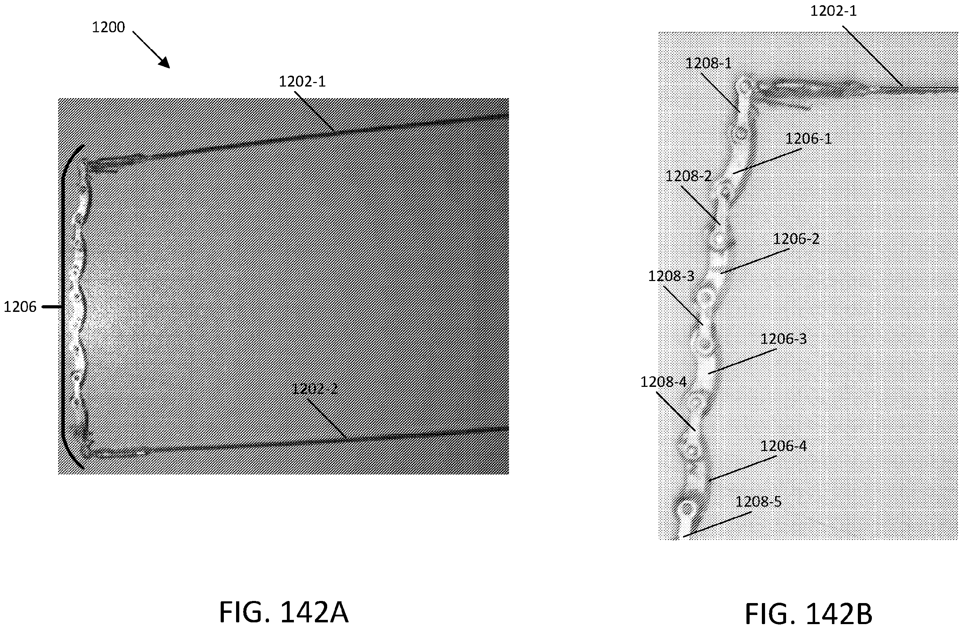

Fig. 142A shows an exemplary cutter 1200 in accordance with the present disclosure, similar to previous embodiments illustrated in Figs 131A-133. Cutter 1200 includes strands 1202-1 and 1202-2 coupled to a plurality of cutting elements 1206. In the example of Figs. 142A-142F, cutting elements 1206 includes a chain of cutting elements, or knives 1206, pivotally attached to, and interspersed with, links 1208. Although Fig. 142A shows five cutting elements, any suitable number of cutting elements 1206 can be used. While the term "cutting elements" is recited generally herein, those of skill in the art will recognize that they can be referred to as, and considered to be, blades or knives.

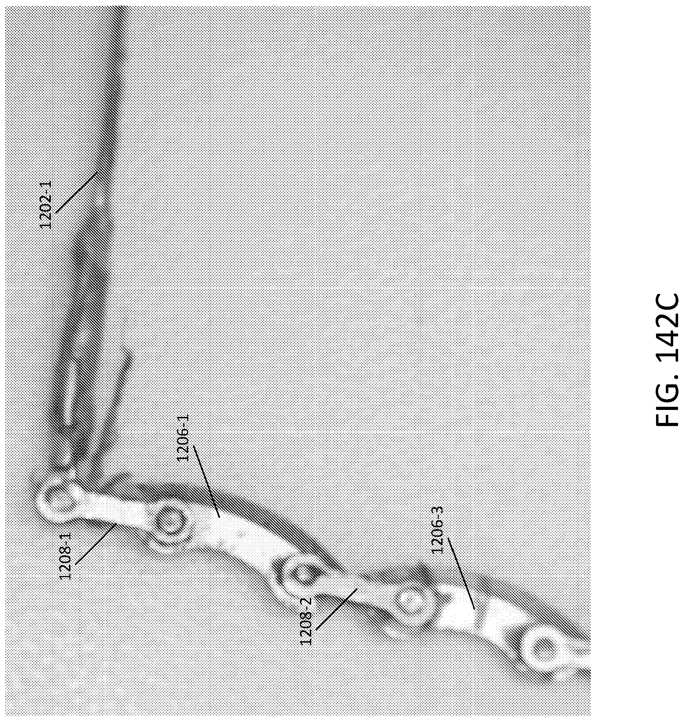

Figs. 142B and 142C show a portion of cutter 1200 of Fig. 142A. Strand 1202-1 is coupled to link 1208-1 such as by routing strand 1202-1 around one of the pins disposed in link 1208-1. Link 1208-1 is pivotally coupled to cutting element 1206-1 by a pin proximate a first end thereof. Cutting element/knife/blade 1206-1 is in turn pivotally coupled to link 1208-2 proximate a second end of blade 1206-1 by a second pin. Cutting element 1206-2 is in turn coupled to links 1208-2 and 1208-3, respectively. Similarly, cutting element 1206-3 is coupled to links 1208-3 and 1208-4 and cutting element 1206-4 is coupled to links 1208-4 and 1208-4.

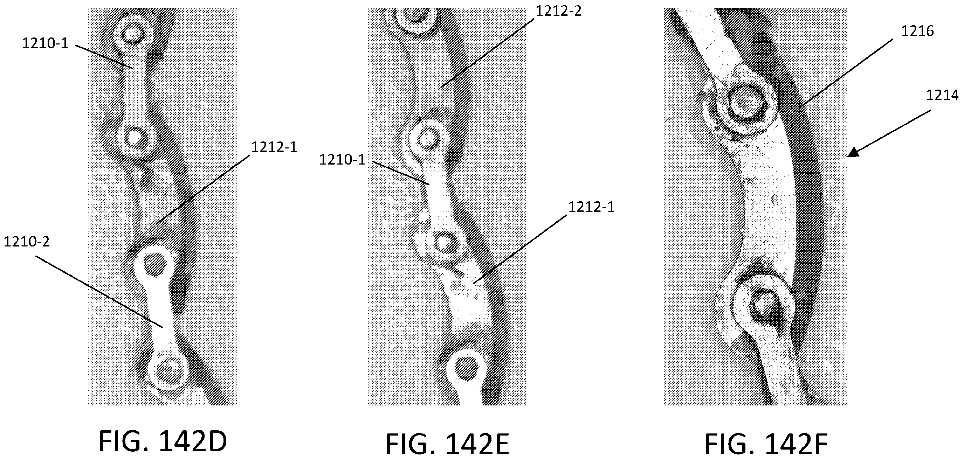

Figs. 142D and 142E show a portion of a chain of cutting elements 1212-1 and 1212-2. Cutting elements 1212-1 and 1212-2 and links 1210-1 and 1210-2 can be analogous to cutting elements 1206 and links 1208 illustrated in Figs. 142A-142C, respectively.

Fig. 142F shows a cutting element 1214 in accordance with the present disclosure. Cutting element 1214 can be analogous to cutting elements 1206 and 1212 illustrated in Figs. 142A-142E. As shown in Fig. 142F, cutting element 1214 has a curved (arcuate, if desired) cutting edge 1216 that is formed along one edge of element 1214, whereas the opposing edge of element 1214 is presented as being concave, but may be straight or convex, if desired.. By providing a cutting edge along one edge of elements 1214, sharp portions of the cutters can be oriented inwardly toward the center of the bag, thereby reducing the chance that the cutting edges 1216 will cut the bag. Moreover, by providing the cutters as a chain link with pins, similar in some manners to a bicycle chain, the links are constrained to move along a single plane, rather than being permitted to twist. Thus, in a chain of cutting elements, such as that shown in Fig. 142A, all of the cutting edges can be one side of the chain so to prevent a bag from being inadvertently cut. The cutting edges can face the interior space of a bag such that the cutting edges contact a tissue specimen (e.g., tissue specimen 1112 shown in Fig. 132C). Cutting element 1214 and cutting edge 1216 are curved with cutting edge 1216 located at the convex end of cutting element 1216. This location of cutting edge 1216 maximizes the surface area and/or length of cutting edge 1216. Cutter 1200 can be used in various arrangements as disclosed elsewhere herein. For example, cutter 1200 can be provided singularly and overlapped with other cutters and used individually in a predetermined order. In another embodiment, cutters 1200 can be used in place of any cutting elements described elsewhere in this disclosure, such as cutting elements 1114 in Figure 133, or cutting elements 1110, cutting elements 1100, strands 1108, for example.

One or more components of embodiments of the present disclosure can be single-use and/or disposable.

It is contemplated that any of the rings disclosed herein may be made of silicone, polymer, metal, or any other suitable material. Where a ring has parts with different flexibilities/rigidities, the more flexible part may be made of a more flexible material (e.g., silicone), and the more rigid part may be made of a more rigid material (e.g., plastic or metal).

It will be apparent to those skilled in the art that various modifications and variations can be made in the disclosed devices and methods without departing from the scope of the disclosure. Other aspects of the disclosure will be apparent to those skilled in the art from consideration of the specification and practice of the features disclosed herein. It is intended that the specification, examples and especially such examples named embodiments of the disclosure, which do not fall under the claimed subject-matter be considered as exemplary only.

## Claims

1. A tissue extraction device, comprising:
a bag having an interior;
a cutting element (1200) extending through the interior of the bag, **characterized in that** the cutting element includes:
a strand (1202), and
a plurality of cutting members (1206) coupled to the strand forming a chain of cutting members, wherein each cutting member includes an edge for cutting tissue as the cutting member is drawn across tissue.

2. The tissue extraction device of claim 1, wherein the cutting members are pivotally attached to and interspersed with links (1208).

3. The tissue extraction device of claim 1, wherein each cutting member (1206) is curved.

4. The tissue extraction device of claim 1, wherein the edge of each cutting member (1206) is linear.

5. The tissue extraction device of claim 1, wherein the edge of each cutting member (1206) is curved.

6. The tissue extraction device of claim 1, wherein the edge of each cutting member (1206) is discrete and is separated from all other edges by a gap.

7. The tissue extraction device of claim 1, further including a member coupled to an interior surface of the bag, such that a channel is defined between the member and the interior surface, wherein the cutting element (1200) extends through the channel.

8. The tissue extraction device of claim 1, wherein the cutting element (1200) includes eyelets.

9. The tissue extraction device of claim 8, further including handles, wherein the handles are removably coupled to the eyelets.

10. The tissue extraction device of claim 1, further including a wound retractor surrounding the cutting element.

11. The tissue extraction device of claim 10, wherein the bag includes an inner layer and an outer layer.

12. The tissue extraction device of claim 11, wherein a rim of the inner layer is removably coupled to a rim of the outer layer.

## Patentansprüche

1. Gewebeextraktionsvorrichtung, umfassend:
einen Beutel mit einem Inneren;
ein Schneidelement (1200), das sich durch das Innere des Beutels erstreckt, **dadurch gekennzeichnet, dass** das Schneidelement Folgendes beinhaltet:
einen Strang (1202), und
eine Vielzahl von Schneidelementen (1206), die an den Strang gekoppelt ist und eine Kette von Schneidelementen bildet, wobei jedes Schneidelement eine Kante zum Schneiden von Gewebe beinhaltet, wenn das Schneidelement über Gewebe gezogen wird.

2. Gewebeextraktionsvorrichtung nach Anspruch 1, wobei die Schneidelemente schwenkbar an Verbindungsstücken (1208) angebracht und damit durchsetzt sind.

3. Gewebeextraktionsvorrichtung nach Anspruch 1, wobei jedes Schneidelement (1206) gekrümmt ist.

4. Gewebeextraktionsvorrichtung nach Anspruch 1, wobei die Kante jedes Schneidelements (1206) linear ist.

5. Gewebeextraktionsvorrichtung nach Anspruch 1, wobei die Kante jedes Schneidelements (1206) gekrümmt ist.

6. Gewebeextraktionsvorrichtung nach Anspruch 1, wobei die Kante jedes Schneidelements (1206) einzeln ist und von allen anderen Kanten durch einen Spalt getrennt ist.

7. Gewebeextraktionsvorrichtung nach Anspruch 1, ferner beinhaltend ein Glied, das an eine Innenfläche des Beutels gekoppelt ist, sodass ein Kanal zwischen dem Glied und der Innenfläche definiert ist, wobei sich das Schneidelement (1200) durch den Kanal erstreckt.

8. Gewebeextraktionsvorrichtung nach Anspruch 1, wobei das Schneidelement (1200) Ösen beinhaltet.

9. Gewebeextraktionsvorrichtung nach Anspruch 8, ferner beinhaltend Griffe, wobei die Griffe entfernbar an die Ösen gekoppelt sind.

10. Gewebeextraktionsvorrichtung nach Anspruch 1, ferner beinhaltend einen Wundretraktor, der das Schneidelement umgibt.

11. Gewebeextraktionsvorrichtung nach Anspruch 10, wobei der Beutel eine innere Schicht und eine äußere Schicht beinhaltet.

12. Gewebeextraktionsvorrichtung nach Anspruch 11, wobei ein Rand der inneren Schicht entfernbar an einen Rand der äußeren Schicht gekoppelt ist.

## Revendications

1. Dispositif d'extraction de tissus, comprenant :
une poche comportant un intérieur ;
un élément coupant (1200) s'étendant à travers l'intérieur de la poche, **caractérisé en ce que** l'élément coupant comprend :
un fil (1202), et
une pluralité d'éléments coupants (1206) couplés au fil formant une chaîne d'éléments coupants, dans lequel chaque élément coupant comprend un bord pour couper le tissu lorsque l'élément coupant est tiré à travers le tissu.

2. Dispositif d'extraction de tissus selon la revendication 1, dans lequel les éléments coupants sont fixés de manière pivotante et intercalés avec des maillons (1208).

3. Dispositif d'extraction de tissus selon la revendication 1, dans lequel chaque élément coupant (1206) est courbé.

4. Dispositif d'extraction de tissus selon la revendication 1, dans lequel le bord de chaque élément coupant (1206) est linéaire.

5. Dispositif d'extraction de tissus selon la revendication 1, dans lequel le bord de chaque élément coupant (1206) est courbé.

6. Dispositif d'extraction de tissus selon la revendication 1, dans lequel le bord de chaque élément coupant (1206) est discret et est séparé de tous les autres bords par un espace.

7. Dispositif d'extraction de tissus selon la revendication 1, comprenant en outre un élément couplé à une surface intérieure de la poche, de sorte qu'un canal soit défini entre l'élément et la surface intérieure, dans lequel l'élément coupant (1200) s'étend à travers le canal.

8. Dispositif d'extraction de tissus selon la revendication 1, dans lequel l'élément coupant (1200) comprend des oeillets.

9. Dispositif d'extraction de tissus selon la revendication 8, comprenant en outre des poignées, dans lequel les poignées sont couplées de manière amovible aux oeillets.

10. Dispositif d'extraction de tissus selon la revendication 1, comprenant en outre un écarteur de plaie entourant l'élément coupant.

11. Dispositif d'extraction de tissus selon la revendication 10, dans lequel la poche comprend une couche interne et une couche externe.

12. Dispositif d'extraction de tissus selon la revendication 11, dans lequel un pourtour de la couche interne est couplé de manière amovible à un pourtour de la couche externe.
